(19) Europäisches Patentamt
European Patent Office
Office européen des brevets

(11) **EP 4 017 287 B1**

(12) **EUROPEAN PATENT SPECIFICATION**

(45) Date of publication and mention
of the grant of the patent:
**11.12.2024   Bulletin 2024/50**

(21) Application number: **20855636.5**

(22) Date of filing: **19.08.2020**

(51) International Patent Classification (IPC):
**A23L 15/00** (2016.01)   **C07K 14/77** (2006.01)
**C12N 15/81** (2006.01)   **A23J 1/18** (2006.01)
**A23J 1/08** (2006.01)   **A23J 3/20** (2006.01)
**A23J 3/04** (2006.01)   **A21D 2/26** (2006.01)
**A21D 13/80** (2017.01)   **A23L 27/60** (2016.01)
**C12N 1/16** (2006.01)   **A21D 2/18** (2006.01)
**A21D 13/44** (2017.01)   **A21D 13/50** (2017.01)
**A23C 11/10** (2021.01)   **A23F 5/00** (2006.01)
**A23J 1/00** (2006.01)   **A23J 3/22** (2006.01)
**A23L 2/66** (2006.01)   **A23L 11/70** (2021.01)

(52) Cooperative Patent Classification (CPC):
**C12N 15/815; A21D 2/18; A21D 2/185;
A21D 2/186; A21D 2/264; A21D 2/267;
A21D 13/44; A21D 13/50; A23C 11/103;
A23F 5/00; A23J 1/008; A23J 1/08; A23J 1/18;
A23J 3/04; A23J 3/20;**                   (Cont.)

(86) International application number:
**PCT/US2020/047076**

(87) International publication number:
**WO 2021/034980 (25.02.2021 Gazette 2021/08)**

(54) **NON-ANIMAL BASED PROTEIN SOURCES WITH FUNCTIONAL PROPERTIES**

NICHTTIERISCHE PROTEINQUELLEN MIT FUNKTIONELLEN EIGENSCHAFTEN

SOURCES DE PROTÉINES D'ORIGINE NON ANIMALE PRÉSENTANT DES PROPRIÉTÉS FONCTIONNELLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **19.08.2019   US 201962888674 P**

(43) Date of publication of application:
**29.06.2022   Bulletin 2022/26**

(73) Proprietor: **Clara Foods Co.
Daly City, CA 94014 (US)**

(72) Inventors:
• **MAHADEVAN, Kritika**
  **San Francisco, California 94080 (US)**
• **AYOUGHI, Farnoosh**
  **San Francisco, California 94080 (US)**
• **JOSHI, Isha**
  **San Francisco, California 94080 (US)**
• **KREPS, Joel Andrew**
  **San Francisco, California 94080 (US)**
• **KSHIRSAGAR, Harshal**
  **San Francisco, California 94080 (US)**
• **IVEY, Frank Douglas**
  **San Francisco, California 94080 (US)**
• **ZHONG, Weixi**
  **San Francisco, California 94080 (US)**
• **LIN, Eric**
  **San Francisco, California 94080 (US)**
• **CHAPEAUX, Alexandre**
  **San Francisco, California 94080 (US)**
• **GOVIND, Sridharan**
  **San Francisco, California 94080 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Carpmaels & Ransford LLP**
**One Southampton Row**
**London WC1B 5HA (GB)**

(56) References cited:
**WO-A2-2018/162557    US-A1- 2018 084 814**
**US-A1- 2018 355 020    US-A1- 2018 355 020**

- **ANONYMOUS: "SERPINB14 - Ovalbumin - Gallus gallus (Chicken) | UniProtKB | UniProt", 23 January 2007 (2007-01-23), pages 1 - 8, XP093056038, Retrieved from the Internet <URL:https://www.uniprot.org/uniprotkb/P01012/entry> [retrieved on 20230620]**
- **RAEKER M.O. ET AL: "Cake-Baking (High-Ratio White Layer) Properties of Egg White, Bovine Blood Plasma, and Their Protein Fractions", CEREAL CHEM 72:299-303, 1 January 1995 (1995-01-01), pages 1 - 5, XP093056078, Retrieved from the Internet <URL:https://www.cerealsgrains.org/publications/cc/backissues/1995/Documents/72_299.pdf> [retrieved on 20230620]**
- **DATABASE UniprotKB 3 May 2011 (2011-05-03), "RecName: Full=Methionine aminopeptidase 2 {ECO:0000255|HAMAP-Rule:MF_03175};", XP055794969, Database accession no. C4R2P3**

- **GODA SHUICHIRO, TAKANO KAZUFUMI, YAMAGATA YURIKO, KATAKURA YOSHIO, YUTANI KATSUHIDE: "Effect of extra N-terminal residues on the stability and folding of human lysozyme expressed in Pichia pastoris", PROTEIN ENGINEERING, vol. 13, no. 4, 2000, pages 299 - 307, XP055794971**
- **ITO, K . ET AL.: "Structural characteristics of hen egg ovalbumin expressed in yeast Pichia pastoris", BIOSCIENCE, BIOTECHNOLOGY AND BIOCHEMISTRY, vol. 69, no. 4, 2005, pages 755 - 761, XP055175533, DOI: 10.1271/bbb.69.755**
- **ANONYMOUS: "FT-R5851B Ovalbumin Product Information", INTERCHIM, 8 April 2009 (2009-04-08), XP055794978, Retrieved from the Internet <URL:http://www.interchim.fr/ft/R/R5851B.pdf>**

(52) Cooperative Patent Classification (CPC): (Cont.)
**A23J 3/227; A23L 2/66; A23L 11/70; A23L 15/35; A23L 27/60; C07K 14/77**

**Description**

**BACKGROUND OF THE INVENTION**

Proteins are important dietary nutrients and food ingredients. They can serve as a fuel source or as sources of amino acids, including the essential amino acids that cannot be synthesized by the body. The daily recommended intake of protein for healthy adults is 10% to 35% of a person's total calorie needs, and currently the majority of protein intake for most humans is from animal-based sources. In addition, proteins are used in a wide variety of foods and food ingredients. In many cases, these proteins are sourced from animals. With the world population growth and the coinciding growth in global food demand, there is a need to provide alternative sustainable, non-animal-based sources of proteins as useful source of protein for daily diet, food ingredients and food products.

US 2018/355020 describes methods and compositions for egg white protein production.

Raeker M.O. ET AL: "Cake-Baking (High-Ratio White Layer) Properties of Egg White, Bovine Blood Plasma, and Their Protein Fractions", 1995, describes cake-baking (high-ratio white layer) properties of egg white, bovine blood plasma, and their protein fractions.

**SUMMARY OF THE INVENTION**

The invention provides a food item comprising an ingredient composition, the ingredient composition comprising a recombinant ovalbumin (rOVA), wherein the rOVA is present in the food item in an amount between 2% and 15% (w/w), and wherein the food item does not comprise any egg-white proteins except rOVA.

In some embodiments, provided herein are ingredients for producing egg-less food items as defined in the claims. The ingredient composition for producing an egg-less food item comprises a recombinant ovalbumin (rOVA), wherein the pH of the rOVA may be between about 3.5 and about 7.0; wherein the rOVA when present in the egg-less food item in an amount between about 2% and about 15% (w/w); and wherein the rOVA provides to the egg-less food item at least one egg white characteristic selected from gelling, foaming, whipping, fluffing, binding, springiness, aeration, coating, film forming, emulsification, browning, thickening, texturizing, humectant, clarification, and cohesiveness.

In some cases, the composition may be dried or may be a powder. In some cases, the composition may comprise at least 75% rOVA (w/w of total protein or w/w of total composition). In some cases, the powder composition may be a concentrate. In some cases, the powder composition may be an isolate. In some cases, the powder composition may be at least about 75%, at least about 80%, at least about 85%, or at least about 90% rOVA (w/w). In some cases, the powder composition is at least about 80%, at least about 85%, or at least about 90% rOVA (w/w). In some cases, the powder is a concentrate. In some cases, the powder composition is an isolate.

In some cases, the composition may be a liquid. In some cases, the liquid composition may comprise at least 50% rOVA (w/w of total protein or w/w of composition). In some cases, the liquid the composition comprises at least about 60%, at least about 65%, at least about 75%, at least about 80%, at least about 85%, or at least about 90% rOVA (w/w). The term w/w of total protein in the context of a % rOVA means that the rOVA comprises a defined percentage of the total protein in the composition. In one example, a composition comprising at least 50% rOVA w/w of total protein would have at least half of the total protein being rOVA and the other half or so being another protein. Thus, the total composition does not necessarily need to be at least 50% rOVA by weight, only the composition's protein content must be at least 50% rOVA.

In some cases, the rOVA provides an equivalent or an improvement in the characteristic compared to native egg white in a similar food item. In some cases, the rOVA provides a foam capacity of at least 20%, 30%, 40%, or 50% greater than native egg white. In some cases, the rOVA provides a time to foaming that may be at least 20%, 30%, 40%, or 50% faster than native egg white. In some cases, the pH of the rOVA when solubilized is between about 3.5 and about 4.5. In some cases, the rOVA provides a hardness to the egg-less food composition that may be greater than native egg white. In some cases, the rOVA provides a chewiness to the egg-less food composition that may be greater than native egg white. In some cases, the rOVA provides a springiness comparable to native egg white.

In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA. In some cases, the amino acid sequence of the rOVA lacks an N-terminal methionine. In some cases, the rOVA further includes an EAEA amino acid sequence (SEQ ID NO: 75) at its N-terminus.

In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized.

In some cases, the pH when solubilized may be between about 6 and about 6.8. In some cases, the pH of the rOVA when solubilized may be less than about 6.1. In some cases, the rOVA may be present in the egg-less food item in an amount of less than about 8%. In some cases, the rOVA may be present in the egg-less food item in an amount of about 7% or less than 7%.

In some embodiments, provided herein are baked goods. A baked food product, may comprise: (i) a recombinant ovalbumin (rOVA), wherein the pH of the rOVA when solubilized may be between about 3.5 and about 7.0; (ii) at least one fat or oil; (iii) at least one grain starch; and (iv) at least one sweetener; wherein the rOVA provides the baked food product at least one egg white characteristic selected from binding, springiness, aeration, browning, texturizing, humectant, and cohesiveness, and the baked food product does not comprise any natural egg white proteins or a natural egg white.

The rOVA is present at 2% to 15% in the product (w/w of total protein or w/w of total food product prior to baking). In some cases, the rOVA is present at about 2% to about 5% in the product (w/w). In some cases, the baked good may comprise a dairy component or a leavening agent, or a combination thereof. In some cases, the product may be a cake, a bread, a roll, a pastry, a cracker, a muffin, a scone, a biscuit, or a cookie. In some cases, the baked product may have a crumb structure equivalent to or better than a similar baked product made with a natural egg white or a natural whole egg. In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA. In some cases, the percentage weight loss is lower in a baked product made with rOVA when compared to an equivalent baked product made with whole egg.

In some embodiments, provided herein are emulsified products. An emulsified product may comprise: (i) a recombinant ovalbumin (rOVA); (ii) at least one fat or oil; (iii) water; wherein the rOVA is present in the product at 2% to 15% (w/w). In some cases, the emulsified product may comprise an acidifying agent. In some cases, the product may be a salad dressing, a sauce, mayonnaise, sandwich spread or a gravy.

In some embodiments, described herein are food products comprising (i) a recombinant ovalbumin (rOVA), wherein the pH of the rOVA when solubilized may be between about 3.5 and about 7.0; (ii) at least one sweetener; and (iii) optionally, a consumable liquid; wherein the rOVA is present in the food product at 2% to 15% (w/w) and wherein the rOVA provides foaming, whipping, fluffing or aeration to the food product.

In some cases, the rOVA may further provide gelation to the food product. In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized. In some cases, the food product may be a meringue, a whipped dessert, a whipped topping or a soufflé. In some cases, the rOVA may provide a foam capacity to the food product of at least 20%, 30%, 40%, or 50% greater than native egg white. In some cases, the rOVA may provide a time to foaming to the food product that may be at least 20%, 30%, 40%, or 50% faster than native egg white. In some cases, the pH of the rOVA when solubilized is between about 3.5 and about 4.5.

In some cases, the rOVA is present in the food product at about 5% to about 10% (w/w). In some cases, the rOVA is present in the food product at about 7% to about 8% (w/w). In some cases, the rOVA is present in the food product at about 4%, about 7%, or about 12% (w/w). In some cases, the pH of the rOVA when solubilized is about 6. In some cases, the rOVA is present in the food product at between about 9% and about 10% (w/w). In some cases, the pH of the rOVA when solubilized is about 7. In some cases, the product may be a beverage. In some cases, the beverage may be a consumable alcohol. In some cases, the rOVA provides foaming, whipping, fluffing or aeration to the consumable alcohol beverage. In some cases, the beverage is a coffee drink. In some cases, the rOVA provides foaming, whipping, fluffing or aeration to the coffee drink. In some cases, the coffee drink lacks a dairy component.

In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA. In some cases, the rOVA does not contaminate the food product with Salmonella. In some cases, the food product is a protein bar, an energy bar, a nutrition bar or a granola bar. In some cases, the food product comprises between about 4% and about 8% (w/w) rOVA. In some cases, the bar is baked or is unbaked.

In some embodiments, described herein is a meat-analog food product. A meat-analog food product may comprise: (i) a recombinant ovalbumin (rOVA); (ii) at least one fat or oil; and (iii) a plant-derived protein; wherein the rOVA is present in the food product between 2% and 15% (w/w); and wherein the rOVA acts as a binding agent or a gelling agent, or a combination thereof.

In some cases, the plant protein may be an extruded plant protein. In some cases, the plant protein may be a non-extruded plant protein. In some cases, the meat analog food product may be selected from a burger, patty, sausage, hot dog, sliced deli meat, jerky, bacon, nugget, a ground meat-like composition, and a formed meat-like composition. In some cases, the rOVA may provide a hardness to the food product that may be greater than native egg white. In some cases, the rOVA may provide a chewiness to the food product that may be greater than native egg white. In some cases, the rOVA may provide a springiness comparable to native egg white.

In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized. In some cases, the rOVA is present in the food product at about 4%, at about 5%, or at about 6% (w/w). In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA.

In some embodiments, provided herein are egg-white substitutes. An egg-white substitute may comprise: (i) a recombinant ovalbumin (rOVA); (ii) at least one fat or oil; and (iii) a polysaccharide or polysaccharide-containing ingredient; wherein the rOVA is present in the composition at 2% to 15% (ww); and wherein the composition may have one or more characteristics selected from hardness, adhesiveness, fracturability, cohesiveness, gumminess, and chewiness, and the one or more characteristics are equivalent to or improved as compared to natural egg white when the egg-white substitute may be cooked.

In some cases, the egg-white substitute may further comprise a flavoring agent or a coloring agent, or a combination thereof. In some cases, the polysaccharide or polysaccharide-containing ingredient may be a starch. In some cases, the polysaccharide or polysaccharide-containing ingredient may be selected from gellan gum, sodium alginate, and psyllium or any combination thereof. In some cases, the rOVA may provide a hardness to the food product that may be greater than native egg white.

In some cases, the rOVA may provide a chewiness to the food product that may be greater than native egg white. In some cases, the rOVA may provide a gumminess and/or springiness comparable to native egg white. In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized. In some cases, the rOVA is present in the food product between about 10% and about 12% (w/w).

In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA.

In some embodiments, described herein are powdered ingredient compositions. A powdered ingredient composition may comprise a recombinant ovalbumin (rOVA), wherein the pH of the rOVA when solubilized may be between about 3.5 and about 7.0, wherein the rOVA may be at least 75% w/w of the composition, and wherein the rOVA may comprise one or more N-linked glycosylation sites having mannose linked to an N-acetyl glucosamine, and wherein the N-linked glycosylation sites lack galactose. In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA. In some cases, the amino acid sequence of the rOVA lacks an N-terminal methionine. In some cases, the rOVA further includes an EAEA amino acid sequence (SEQ ID NO: 75) at its N-terminus. In some cases, the composition comprises at least at least about 80%, at least about 85%, or at least about 90% rOVA (w/w).

In some embodiments, a liquid composition may comprise a recombinant ovalbumin (rOVA) and the composition may comprise at least 50% rOVA (w/w of total protein or w/w of total composition). In some cases, the composition may comprise at least about 60%, at least about 65%, at least about 75%, at least about 80%, at least about 85%, or at least about 90% rOVA (w/w).

In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA.

In some cases, the amino acid sequence of the rOVA lacks an N-terminal methionine. In some cases, the rOVA further includes an EAEA amino acid sequence (SEQ ID NO: 75) at its N-terminus. In some cases, the pH of the solubilized rOVA may be between about 3.5 and about 7.0. In some cases, the pH of the solubilized rOVA may be between about 6 and about 6.8. In some cases, the pH of the solubilized rOVA may be less than about 6.1.

In some cases, the rOVA may provide to an egg-less food item at least one egg white characteristic selected from gelling, foaming, whipping, fluffing, binding, springiness, aeration, coating, film forming, emulsification, browning, thickening, texturizing, humectant, clarification, and cohesiveness. In some cases, the rOVA may provide an equivalent or an improvement in the characteristic compared to native egg white in a similar egg-less food item. In some cases, the rOVA may provide to the egg-less food item a foam capacity of at least 20%, 30%, 40%, or 50% greater than native egg white.

In some cases, the rOVA may provide to the egg-less food item a time to foaming that may be at least 20%, 30%, 40%, or 50% faster than native egg white. In some cases, the rOVA may provide to the egg-less food item a hardness that may be greater than native egg white. In some cases, the pH of the rOVA when solubilized is between about 3.5 and about 4.5. In some cases, the rOVA is present in the egg-less food item at about 5% to about 10% (w/w). In some cases, the rOVA is present in the egg-less food item at about 7% to about 8% (w/w). In some cases, the rOVA is present the egg-less food item at about 4%, about 7%, or about 12% (w/w). In some cases, the pH of the rOVA when solubilized is about 6. In some cases, the rOVA may provide to the egg-less food item a chewiness that may be greater than native egg white. In some cases, the rOVA may provide to the egg-less food item a springiness comparable to native egg white.

In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized. In some cases, the rOVA does not contaminate the egg-less food item with Salmonella.

In some embodiments, described herein are dry or powdered compositions comprising a recombinant ovalbumin (rOVA), wherein the composition may comprise at least 50% rOVA (w/w of total protein or w/w of total composition). In some cases, the composition may comprise at least about 60%, at least about 65%, at least about 75%, at least about 80%, at least about 85%, at least about 90%, or at least about 95% rOVA (w/w). In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1.

In some cases, the rOVA may comprise an amino acid sequence of a duck OVA, an ostrich OVA, or a chicken OVA. In some cases, the amino acid sequence of the rOVA lacks an N-terminal methionine. In some cases, the rOVA further includes an EAEA amino acid sequence (SEQ ID NO: 75) at its N-terminus. In some cases, the rOVA may provide to an egg-less food item at least one egg white characteristic selected from gelling, foaming, whipping, fluffing, binding, springiness, aeration, coating, film forming, emulsification, browning, thickening, texturizing, humectant, clarification, and cohesiveness. In some cases, the rOVA may provide an equivalent or an improvement in the characteristic compared to native egg white in a similar egg-less food item.

In some cases, the rOVA may provide to the egg-less food item a foam capacity of at least 20%, 30%, 40%, or 50% greater than native egg white. In some cases, the rOVA may provide to the egg-less food item a time to foaming that may be at least 20%, 30%, 40%, or 50% faster than native egg white. In some cases, the pH of the rOVA when solubilized is between about 3.5 and about 4.5. In some cases, the rOVA is present in the egg-less food at about 4%, about 7%, or about 12% (w/w). In some cases, the pH of the rOVA when solubilized is about 6.

In some cases, the rOVA may provide to the egg-less food item a hardness that may be greater than native egg white. In some cases, the rOVA may provide to the egg-less food item a chewiness that may be greater than native egg white. In some cases, the rOVA may provide to the egg-less food item a springiness comparable to native egg white. In some cases, the rOVA provides improved gelation when the rOVA comprises an amino acid sequence of a chicken OVA and the pH is between about 6.5 and 7.0 when solubilized. In some cases, the rOVA provides improved gelation when the rOVA comprises an amino acid sequence of an ostrich OVA and the pH is less than about 6.0 and above about 3.7 when solubilized.

Described herein are methods of making a food product. A method of making a food product may comprise: (i) providing a recombinant ovalbumin (rOVA) at a pH when solubilized of between about 3.5 and about 7.0; (ii) combining the rOVA in an amount between 2% and 15% (w/w) with one or more consumable ingredients to form a food product, wherein the rOVA may provide at least one egg white characteristic to the food product selected from gelling, foaming, whipping, fluffing, binding, springiness, aeration, coating, film forming, emulsification, browning, thickening, texturizing, humectant, clarification and cohesiveness.

Described herein are methods of making an ingredient. A method of producing an ingredient composition may comprise: (i) expressing a recombinant ovalbumin (rOVA) in a microbial cell, wherein the rOVA may be secreted by the microbial cell into a liquid media; (ii) harvesting the liquid media containing secreted rOVA; (iii) performing a separation step at a pH of about 3.5; (iv) solubilizing the rOVA at a pH of about 12; (v) adjusting the final pH of the rOVA to between about 3.5 and about 7.0 to generate the ingredient composition.

In some cases, the separation step may comprise ion exchange chromatography or ammonium sulfate precipitation. In some cases, the ion exchange chromatography may be cation exchange chromatography or anion exchange chromatography, or a combination thereof. In some cases, the method further may comprise a filtration step following the solubilizing step. In some cases, the microbial cell may be a fungal cell. In some cases, the fungal cell may be a Pichia sp. In some cases, the microbial cell expresses a recombinant helper factor; wherein the helper factor enhances the level of expression or accumulation of rOVA.

In some cases, the rOVA may comprise an amino acid sequence of SEQ ID NO: 2 or SEQ ID NO: 1 or an amino acid sequence with at least 70% identity to SEQ ID NO: 2 or SEQ ID NO: 1. In some cases, the rOVA may comprise an amino acid sequence of a duck OVA, an ostrich OVA, or a chicken OVA. In some cases, the amino acid sequence of the secreted rOVA lacks an N-terminal methionine. In some cases, the secreted rOVA further includes an EAEA amino acid sequence (SEQ ID NO: 75) at its N-terminus.

The recombinant ovalbumin (rOVA) may be used as an ingredient in making a baked good. rOVA may be used as an ingredient in making an egg-less food product. rOVA may be used as an ingredient in making a meat-analog food product. rOVA may be used as an ingredient in making an egg-white substitute. rOVA may be used as a substitute egg-wash for a baked product; wherein the substitute egg-wash may provide film formation equivalent to or better than an egg-wash may comprise a natural egg white or a natural whole egg.

rOVA may comprise an amino acid sequence of SEQ ID NO: 2 or SEQ ID NO: 1 or an amino acid sequence with at least 70% identity to SEQ ID NO: 2 or SEQ ID NO: 1. rOVA may comprise an amino acid sequence of a duck OVA, an ostrich OVA, or a chicken OVA. In some cases, the rOVA is present in the egg-wash in an amount between 8% and 9% (w/w).

Also described herein are large-scale production of recombinant ovalbumin (rOVA). A large-scale production of rOVA, may comprise an at least 1-liter liquid culture of microbial cells expressing the rOVA. In some cases, the large-scale production may comprise an at least 10-liter liquid culture of microbial cells expressing the rOVA. In some cases, the large-scale production may comprise an at least 100-liter liquid culture of microbial cells expressing the rOVA. In some cases, the large-scale production may comprise an at least 1000-liter liquid culture of microbial cells expressing the rOVA. In some cases, the large-scale production comprises an at least 10,000-liter liquid culture of microbial cells expressing the rOVA. In some cases, the large-scale production comprises an at least 100,000-liter liquid culture of microbial cells expressing the rOVA. In some cases, the large-scale production comprises about a 200,000-liter liquid culture of microbial cells expressing the rOVA.

Also disclosed herein may be an ingredient composition for producing an egg-less food item comprising a recombinant ovalbumin. The recombinant ovalbumin may provide at least one egg white characteristic selected from the group consisting of gelling, foaming, whipping, fluffing, binding, springiness, aeration, coating, film forming, emulsification, browning, thickening, texturizing, humectant, clarification and cohesiveness.

The egg white characteristic provided by the recombinant ovalbumin may be substantially the same or better than the same characteristic provided by a native egg white. The composition does not contain any native egg white protein. The composition may not contain any animal products.

The composition may not contain any protein extracted from an egg. The color of the composition may be improved in whiteness or colorlessness as compared to a native egg white. The recombinant ovalbumin may comprise a polypeptide sequence derived from the group consisting of chicken, goose, quail, ostrich, and duck.

The recombinant ovalbumin may be sensory neutral with regard to taste, smell, mouthfeel or any combination thereof. The recombinant ovalbumin may provide the features of foaming and coagulation to the composition.

In some embodiments, provided herein are baked products comprising the ingredient composition provided herein. The recombinant ovalbumin may provide structure, texture or both structure and texture to the baked product. The recombinant ovalbumin may provide a protein fortification to the baked product.

The recombinant ovalbumin may be compatible with gluten formation. The baked product may be selected from the group consisting of cake, cookie, bagel, biscuit, bread, muffin, cupcake, scone, pancake, macaroon, meringue, choux pastry and soufflé. The cake made using such an ingredient may be pound cake, sponge cake, yellow cake, or angel food cake. The composition may further comprise one or more components selected from the group consisting of a sweetening agent, a gum, a hydrocolloid, a starch, a fiber, a plant protein, algal protein, a coloring agent and a flavoring extract.

The composition may provide one or more characteristics suitable for an egg-like dish, and wherein the characteristic may be selected from the group consisting of foaming, coagulation, binding, structure, texture, film-formation, nutritional profile, cholesterol free and protein fortification. In some embodiments, provided herein are egg-like dishes comprising the ingredient composition described herein. The egg-like dish may be selected from the group consisting of scramble, omelet, patty, soufflé, quiche and frittata. The egg-like dish may be vegan, vegetarian, halal or kosher.

The composition may provide one or more characteristics suitable for a processed meat product or meat-like product, and wherein the characteristic may be selected from the group consisting of high protein content, binding, and sensory neutrality. In some embodiments, provided herein are meat-like products, comprising the ingredient compositions disclosed herein.

The meat-like product may be selected from the group consisting of a burger, patty, sausage, hot dog, sliced deli meat, jerky, bacon, nugget and ground meat-like mixture or formed meat or meat-like composition.

The recombinant ovalbumin may provide the characteristic of binding suitable for adhesion of a food coating. A food coating may comprise the ingredients described herein. The food coating may be a batter or a breading. The recombinant ovalbumin may further provide the characteristic of crunchy texture to the food coating when cooked, baked or fried.

The recombinant ovalbumin may provide the characteristic suitable for a confectionary selected from the group consisting of odor neutrality, flavor, mouthfeel, texture, nutritional value and protein fortification. A confectionary product may comprise the ingredient compositions described herein, as defined in the claims. The recombinant ovalbumin may provide a firm or chewy texture to the confectionary. The confectionary may be a gummy, a taffy or a nougat.

The recombinant ovalbumin may provide a characteristic suitable for a dairy-like beverage selected from the group consisting of odor neutrality, flavor, mouthfeel, foaming, frothiness, texture, and nutritional value. A dairy-like beverage may comprise the ingredient compositions described herein, as defined in the claims. The beverage may be selected from the group consisting of smoothie, milkshake, "egg-nog", and coffee beverage.

Recombinant ovalbumin may provide a characteristic suitable for a dessert product selected from the group consisting of creamy texture, low fat content, odor neutrality, flavor, mouthfeel, texture, binding, and nutritional value. A dessert product may comprise the ingredient compositions described herein. The dessert product may be selected from the group consisting of a mousse, a cheesecake, a custard, a pudding, a popsicle, a frozen dessert, and an ice cream. The dessert product may be vegan, vegetarian or dairy-free.

The recombinant ovalbumin may provide a characteristic suitable for a sauce or dressing selected from the group consisting of binding, emulsifying, odor neutrality, and mouthfeel. A sauce or dressing may comprise the ingredient compositions described herein. The sauce or dressing may be selected from the group consisting of salad dressing, mayonnaise, commercial mayonnaise substitutes, alfredo sauce, and hollandaise sauce.

The recombinant ovalbumin may provide a characteristic suitable for a snack food selected from the group consisting of binding, protein supplementation, flavor neutrality, odor neutrality, and mouth feel. A snack food may comprise the ingredient compositions described herein. The snack food may be a protein bar, a nutrition bar or a granola bar. The ingredient composition may further comprise one or more additional components selected from the group consisting of a sweetener, a gum, a plant protein, algal protein, a flavoring, a colorant, a thickener, an acidulant and an emulsifier.

Disclosed herein are methods of producing an egg white replacer. The egg-white replacer may comprise providing a recombinant ovalbumin; mixing the recombinant ovalbumin with at least one additional component to form the egg white replacer. The recombinant ovalbumin may provide at least one egg white characteristic selected from the group consisting of gelling, foaming, whipping, fluffing, binding, springiness, aeration, creaminess and cohesiveness to the egg white replacer. The at least one egg white characteristic may be the same or better than a native egg provided in the same amount or concentration (weight/volume).

The method may further comprise producing the recombinant ovalbumin in a heterologous host cell, wherein the host cell may be E.coli, yeast, filamentous fungus, or Trichoderma. The yeast or filamentous fungus may be selected from the group consisting of a Saccharomyces species and a Pichia species. The recombinant ovalbumin may be secreted from the host cell. The recombinant ovalbumin may be glycosylated by the host cell and wherein the glycosylation of the ovalbumin may be not identical to ovalbumin isolated from chicken egg.

The method may further comprise treating the secreted ovalbumin with a deglycosylation enzyme. The deglycosylation enzyme may be expressed by the host cell.

The host may comprise a nucleic acid sequence encoding the recombinant ovalbumin, and the recombinant ovalbumin has an amino acid sequence of an ovalbumin from an avian species. The host may comprise a nucleic acid sequence encoding the recombinant ovalbumin, and the recombinant ovalbumin has an amino acid sequence of an ovalbumin that has at least 95% sequence identity with an ovalbumin from an avian species. The avian species may be chicken, duck, goose, ostrich, or quail.

The ovalbumin from the avian species may be selected from the group consisting of SEQ ID NO. 1-74.

In some embodiments, disclosed herein is a recombinant protein composition for use as an egg-white replacer. The composition can comprise a recombinant ovalbumin and at least one additional component. The recombinant ovalbumin may provide at least one egg white characteristic selected from the group consisting of gelling, foaming, whipping, fluffing, binding, springiness, aeration, creaminess and cohesiveness to the composition. The at least one egg white characteristic may be the same or better than a native egg compared at the same amount or concentration (weight/volume).

The recombinant ovalbumin may have an amino acid sequence of an ovalbumin from an avian species. The recombinant ovalbumin may have an amino acid sequence of an ovalbumin that has at least 95% sequence identity with an ovalbumin from an avian species.

The avian species may be chicken, duck, goose, ostrich, or quail. The ovalbumin from the avian species may be selected from the group consisting of SEQ ID NO. 1-74.

An animal nutrition composition may comprise a recombinant ovalbumin (rOVA). The rOVA may be in a form selected from whole cell extract, fractionated cell extract and isolated protein. The composition may be comprised within a pet food, an animal feed, a chewy treat, bone broth, smoothie or other liquid for animal nutrition and a solid nutritional supplement suitable for animal consumption.

Additionally, any composition, food product, ingredient, use, or method disclosed herein is applicable to any herein-disclosed composition, food product, ingredient, use, or method. In other words, any aspect or embodiment described herein can be combined with any other aspect or embodiment as disclosed herein.

**BRIEF DESCRIPTION OF THE DRAWINGS**

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIGs. 1A-B illustrate glycosylation patterns of native OVA and rOVA produced in P. pastoris respectively.
FIG. 2 illustrates pound cakes and their cross-sections made using rOVA compared to cakes made using eggs.
FIG. 3 illustrates meringues made using rOVA compared to meringues made using eggs.
FIG. 4 illustrates heat coagulation and foaming properties of whole egg, egg white and native OVA solutions.
FIG. 5 illustrates heat coagulation and foaming properties of egg white and native OVA compared to rOVA.
FIG. 6A illustrates gel electrophoresis migration of glycosylated native and recombinant OVA. Also shown are deglycosylated recombinant OVA treated with EndoH and PNGaseF enzymes.
FIG. 6B illustrates a chromatogram depicting glycosylation patterns of rOVA produced in P. pastoris.
FIG. 7 illustrates gelation results before and after foaming of various OVA samples compared to egg white.
FIG. 8 illustrates film formation using nOVA, rOVA, whole egg wash and a commercial egg-white substitute.
FIGs. 9A-B illustrates emulsification results of nOVA, rOVA and egg white protein at acidic and neutral pH.
FIG. 10 illustrates foaming of rOVA and control samples in an alcohol-based drink.
FIG. 11 illustrates egg patties made using nOVA, rOVA and egg white proteins.
FIG. 12 illustrates meringues made using rOVA samples and egg white proteins.
FIG. 13 illustrates protein bars made with egg white proteins (EWP), nOVA and rOVA at different protein inclusion levels.

**DETAILED DESCRIPTION OF THE INVENTION**

Disclosed herein are compositions and methods of making compositions for non-animal-based sources of proteins which provide nutritional as well as functional properties to food ingredients and consumable products for ingestion by an animal, including a human, such as for daily diet, ingredients for human food and treats and for human and animal nutrition.

The compositions and methods disclosed herein contain fermentation-derived ovalbumin, produced through recombinant technology, i.e., a recombinant ovalbumin (rOVA). The compositions and methods for making compositions comprising rOVA can increase the protein content of a consumable or food ingredient, and also provide functional features for use in the preparation of food ingredients and consumable food products for animal and human ingestion.

In some embodiments, the rOVA provides one or more functional characteristics such as of gelling, foaming, whipping, fluffing, binding, springiness, aeration, coating, film forming, emulsification, browning, thickening, texturizing, humectant, clarification, and cohesiveness. The rOVA with such feature(s) can be a food ingredient that provides for production of an egg-less or animal-free food ingredient or food product.

As used herein "native" in the context of native egg white, native egg protein, native ovalbumin and native egg, refers to the egg white, egg protein, ovalbumin or whole egg, respectively, produced by an animal or collected from an animal, in particular an egg-laying animal such as a bird. The rOVA and compositions containing rOVA can be used in food ingredients and food products, such that the ingredient or product does not contain any native egg white, native egg protein, native ovalbumin or native egg. The ingredients or food products made using rOVA do not include any egg-white proteins other than rOVA. The rOVA and compositions containing rOVA can be used in food ingredients and food products, such that the ingredient or product does not contain any animal products.

The rOVA can (alone or with other ingredients) substitutes for the use of whole egg or egg white in the production of a food product. In some embodiments, the feature(s) provided by the rOVA is substantially the same or better than the same characteristic provided by a native egg white or native egg. For example, the rOVA and compositions containing rOVA can have gelling, foaming, whipping, fluffing, binding, springiness, aeration, coating, film forming, emulsification, browning, thickening, texturizing, preserving moisture (humectant), clarification, and cohesiveness, improved color, such as a whiter color, as compared to native egg white or native whole egg and compositions made with native egg white.

**Food ingredients and food products with rOVA**

Food ingredients and food products disclosed herein include compositions that comprise, consists essentially of, or consist of rOVA, where rOVA provides at least one functional feature to the composition, food ingredient, or food product. In some cases, at least one functional feature provided by the rOVA is comparable or substantially similar to a native egg or egg white or native OVA (nOVA). For instance, it may provide any one of gelling, foaming, whipping, fluffing, binding, springiness, aeration, coating, film forming, emulsification, browning, thickening, texturizing, preserving moisture (humectant), clarification, and cohesiveness comparable to a whole egg, egg-white or nOVA composition. In some embodiments, the at least one functional feature is provided by or provided substantially by the inclusion of rOVA in the food ingredient or food product, for example, in the absence of any other whole egg proteins or egg white proteins.

rOVA may be present at or at least at 2%, 3%, 4%, 5%, 6%, 7%, 8%, 9%, 10%, 11%, 12%, 13%, 14%, or 15% on a weight/weight (w/w) basis. These concentrations can be based on the dry weight of the composition. Additionally, or alternatively, the concentration of rOVA in such compositions is at most 15%, 10%, 5%, 4%, 3%, or 2% on a w/w basis.

Disclosed herein are consumable food compositions and methods of making such compositions where rOVA provides at least one feature of whole egg or egg-whites to a consumable food composition. rOVA may be added to a consumable food composition to increase the protein content, such as for added nutrition.

A consumable product can include one or more other proteins, such as a non-OVA protein or a non-recombinant protein. The rOVA can increase amount of protein content in a consumable product, and/or provide one or more egg-white like features. For example, the consumable composition can include a whey protein, a pea protein, a soy protein, an almond protein, an oat protein, a flax seed protein, or a vegetable protein. The consumable protein may include an extruded plant protein or a non-extruded plant protein.

The compositions and methods for making compositions may have an egg-white like property and increase the protein content in the composition. The compositions and methods for making compositions with an egg-white like property may increase the protein content, while not adversely affecting the stability, or one or more sensory qualities of the composition.

The consumable food compositions and methods for making consumable food compositions comprise rOVA and the addition of rOVA generates an egg-white like composition. The consumable food composition may be a finished product or an ingredient for making a finished product, e.g., a liquid or a powdered rOVA composition, in accordance with the claims.

rOVA protein may be used on its own or in combination with other components to form a composition, as described herein. rOVA may be used as an ingredient to form a composition and the rOVA ingredient (or rOVA starting composition to be added) may contain about or at least about 10%, 20%, 30%, 40%, 50%, 55%, 60%, 65%, 70%, 75%, 80%, 85%, 90%, 95%, 96%, 97%, 98%, or 99% rOVA by weight per total weight (w/w) and/or weight per total volume (w/v). In some cases, a composition described herein may contain up to about 10%, 20%, 30%, 40%, 50%, 55%, 60%, 65%, 70%, 75%, 80%, 85%, 90%, 95%, 96%, 97%, 98%, or 99% rOVA by w/w or w/v. About or at least about 10%, 20%, 30%, 40%, 50%, 55%, 60%, 65%, 70%, 75%, 80%, 85%, 90%, 95%, 96%, 97%, 98%, or 99% of the protein in a composition may be rOVA by weight per total weight (w/w) and/or weight per total volume (w/v). In some cases, up to or about 10%, 20%, 30%, 40%, 50%, 55%, 60%, 65%, 70%, 75%, 80%, 85%, 90%, 95%, 96%, 97%, 98%, or 99% of the protein in a composition may be rOVA by w/w or w/v.

A composition described herein may contain total protein at a concentration of about or at least 5, 6, 7, 8, 9, 10, 11, 12, 13, 13.2, 14, 15, 16, 17, 18, 19, 20, 21, 22, 23, 24, 25, 30, 35, 40, 45, 50, 55, 60, 65, 70, or 75 g total protein per 100 mL liquid (e.g., water). In some cases, a composition described herein may contain total protein at a concentration of about or at least 5, 6, 7, 8, 9, 10, 11, 12, 13, 13.2, 14, 15, 16, 17, 18, 19, 20, 21, 22, 23, 24, 25, 30, 35, 40, 45, 50, 55, 60, 65, 70, 75, 80, 85, 90, 95, or 100 g total protein per 100 g composition (e.g., powder).

A composition described herein may contain rOVA at a concentration of about or at least 5, 6, 7, 8, 9, 10, 11, 12, 13, 13.2, 14, 15, 16, 17, 18, 19, 20, 21, 22, 23, 24, 25, 30, 35, 40, 45, 50, 55, 60, 65, 70, or 75 g per 100 mL liquid (e.g., water). In some cases, a composition described herein may contain rOVA at a concentration of about or at least 5, 6, 7, 8, 9, 10, 11, 12, 13, 13.2, 14, 15, 16, 17, 18, 19, 20, 21, 22, 23, 24, 25, 30, 35, 40, 45, 50, 55, 60, 65, 70, 75, 80, 85, 90, 95, or 100 g total protein per 100 g composition (e.g., powder)

A composition described herein may contain total protein at a concentration of about or at least 0.1, 0.2, 0.3, 0.5, 0.7, 1.0, 1.2, 1.5, 1.7, 2.0, 2.2, 2.5, 2.7, 3.0, 3.2, 3.5, 3.7, 4.0, 4.2, 4.5, 4.7 or 5g total protein per 100 mL liquid (e.g., water). In some cases, a composition described herein may contain total protein at a concentration of about or at least 0.1, 0.2, 0.3, 0.5, 0.7, 1.0, 1.2, 1.5, 1.7, 2.0, 2.2, 2.5, 2.7, 3.0, 3.2, 3.5, 3.7, 4.0, 4.2, 4.5, 4.7 or 5g total protein per 100 g composition (e.g., powder).

A composition described herein may contain rOVA at a concentration of about or at least 0.1, 0.2, 0.3, 0.5, 0.7, 1.0, 1.2, 1.5, 1.7, 2.0, 2.2, 2.5, 2.7, 3.0, 3.2, 3.5, 3.7, 4.0, 4.2, 4.5, 4.7 or 5g per 100 mL liquid (e.g., water). In some cases, a composition described herein may contain rOVA at a concentration of about or at least 0.1, 0.2, 0.3, 0.5, 0.7, 1.0, 1.2, 1.5, 1.7, 2.0, 2.2, 2.5, 2.7, 3.0, 3.2, 3.5, 3.7, 4.0, 4.2, 4.5, 4.7 or 5g per 100 g composition (e.g., powder).

The rOVA consumable composition may be a liquid composition. In such cases, the concentration of rOVA in the liquid composition may be between 0.1% to 90%. The concentration of rOVA in the liquid composition may be at least 0.1%. The concentration of rOVA in the liquid composition may be at most 90%. The concentration of rOVA in the liquid composition may be from 0.1% to 1%, 0.1% to 5%, 0.1% to 10%, 0.1% to 15%, 0.1% to 20%, 0.1% to 25%, 0.1% to 30%, 0.1% to 35%, 0.1% to 40%, 1% to 5%, 1% to 10%, 1% to 15%, 1% to 20%, 1% to 25%, 1% to 30%, 1% to 35%, 1% to 40%, 5% to 10%, 5% to 15%, 5% to 20%, 5% to 25%, 5% to 30%, 5% to 35%, 5% to 40%, 10% to 15%, 10% to 20%, 10% to 25%, 10% to 30%, 10% to 35%, 10% to 40%, 15% to 20%, 15% to 25%, 15% to 30%, 15% to 35%, 15% to 40%, 20% to 25%, 20% to 30%, 20% to 35%, 20% to 40%, 25% to 30%, 25% to 35%, 25% to 40%, 30% to 35%, 30% to 40%, 35% to 40%, 40% to 45%, 45% to 50%, 50% to 55%, 55% to 60%, 60% to 65%, 65% to 70%, 70% to 75%, 75% to 80%, 80% to 85%, 85% to 90%, or 90% to 95% in weight per total volume (w/v). The concentration of rOVA in the liquid composition may be about 0.1%, 1%, 5%, 10%, 15%, 20%, 25%, 30%, 35%, 40%, 45%, 50%, 55%, 60%, 65%, 70%, 75%, 80%, 85%, 90%, or 95% w/v. The concentration of rOVA in the liquid composition may be at least 0.1%, 1%, 5%, 10%, 15%, 20%, 25%, 30%, 35%, 40%, 45%, 50%, 55%, 60%, 65%, 70%, 75%, 80%, 85%, 90%, or 95% w/v. The concentration of rOVA in the liquid composition may be at most 1%, 5%, 10%, 15%, 20%, 25%, 30%, 35% 40%, 45%, 50%, 55%, 60%, 65%, 70%, 75%, 80%, 85%, 90%, or 95% w/v. rOVA may be the sole protein in the liquid composition. In other cases, a liquid composition may comprise proteins other than rOVA.

The rOVA consumable composition may be a solid composition. In such cases, the concentration of rOVA in the solid composition may be between 0.1% to 70%. The concentration of rOVA in the solid composition may be at least 0.1%. The concentration of rOVA in the solid composition may be at most 70%. The concentration of rOVA in the solid composition may be 0.1% to 1%, 0.1% to 10%, 0.1% to 20%, 0.1% to 30%, 0.1% to 40%, 0.1% to 50%, 0.1% to 60%, 0.1% to 70%, 1% to 10%, 1% to 20%, 1% to 30%, 1% to 40%, 1% to 50%, 1% to 60%, 1% to 70%, 10% to 20%, 10% to 30%, 10% to 40%, 10% to 50%, 10% to 60%, 10% to 70%, 20% to 30%, 20% to 40%, 20% to 50%, 20% to 60%, 20% to 70%, 30% to 40%, 30% to 50%, 30% to 60%, 30% to 70%, 40% to 50%, 40% to 60%, 40% to 70%, 50% to 60%, 50% to 70%, or 60% to 70% weight per total weight (w/w) and/or weight per total volume (w/v). The concentration of rOVA in the solid composition may be 0.1%, 1%, 10%, 20%, 30%, 40%, 50%, 60%, or 70% w/w or w/v. The concentration of rOVA in the solid composition may be at least 0.1%, 1%, 10%, 20%, 30%, 40%, 50% or 60% w/w or w/v. The concentration of rOVA in the solid composition may be at most 1%, 10%, 20%, 30%, 40%, 50%, 60%, or 70% w/w or w/v.

The rOVA consumable composition may be a powdered composition. In such cases, the concentration of rOVA in the powder composition may be between 15% to 99% weight per total weight (w/w) and/or weight per total volume (w/v). The concentration of rOVA in the powder composition may be at least 15% w/w or w/v. In embodiments, the concentration of rOVA in the powder composition may be at most 99% w/w or w/v. The concentration of rOVA in the powder composition may be 15% to 30%, 15% to 45%, 15% to 60%, 15% to 75%, 15% to 80%, 15% to 85%, 15% to 90%, 15% to 95%, 15% to 99%, 30% to 45%, 30% to 60%, 30% to 75%, 30% to 80%, 30% to 85%, 30% to 90%, 30% to 95%, 30% to 99%, 45% to 60%, 45% to 75%, 45% to 80%, 45% to 85%, 45% to 90%, 45% to 95%, 45% to 99%, 60% to 75%, 60% to 80%, 60% to 85%, 60% to 90%, 60% to 95%, 60% to 99%, 75% to 80%, 75% to 85%, 75% to 90%, 75% to 95%, 75% to 99%, 80% to 85%, 80% to 90%, 80% to 95%, 80% to 99%, 85% to 90%, 85% to 95%, 85% to 99%, 90% to 95%, 90% to 99%, or 95% to 99% w/w or w/v. The concentration of rOVA in the powder composition may be about 15%, 30%, 45%, 60%, 75%, 80%, 85%, 90%, 95%, or 99% w/w or w/v. The concentration of rOVA in the powder composition may be at least 15%, 30%, 45%, 60%, 75%, 80%, 85%, 90% or 95% w/w or w/v. The concentration of rOVA in the powder composition may be at most 30%, 45%, 60%, 75%, 80%, 85%, 90%, 95%, or 99% w/w or w/v. rOVA may be the sole protein in the powder composition. A powder composition may comprise proteins other than rOVA.

In some cases, a powder composition may be a concentrate which comprises at least 70% rOVA w/w. In some cases, a powder composition may be a concentrate which comprises at least 80% rOVA w/w. In some cases, a powder composition may be an isolate which comprises at least 90% rOVA w/w. In some cases, a powder composition may be an isolate which comprises at least 95% rOVA w/w.

The rOVA consumable composition may be a concentrated liquid composition. In such cases, the concentration of rOVA in the concentrated liquid composition may be between 10% to 60% weight per total weight (w/w) and/or weight per total volume (w/v). The concentration of rOVA in the concentrated liquid may be at least 10% w/w or w/v. The concentration of rOVA in the concentrated liquid may be at most 60% w/w or w/v. The concentration of rOVA in the concentrated liquid may be 10% to 20%, 10% to 30%, 10% to 40%, 10% to 50%, 10% to 60%, 20% to 30%, 20% to 40%, 20% to 50%, 20% to 60%, 30% to 40%, 30% to 50%, 30% to 60%, 40% to 50%, 40% to 60%, or 50% to 60% w/w or w/v. The concentration of rOVA in the concentrated liquid may be about 10%, 20%, 30%, 40%, 50%, or 60% w/w or w/v. The concentration of rOVA in the concentrated liquid may be at least 10%, 20%, 30%, 40% or 50% w/w or w/v. The concentration of rOVA in the concentrated liquid may be at most 20%, 30%, 40%, 50%, or 60% w/w or w/v. The liquid may include any consumable solvent, e.g., water, dairy, oil, or other cooking base.

The rOVA consumable composition may be a prepared food for example, as a baked good, a salad dressing, an egg-like dish (such as an egg-patty or scramble), a dessert or dairy-like product or a meat-analog (such as a vegan meat patty, sausage or hot dog). rOVA may be present at or at least at 2%, 3%, 4%, 5%, 6%, 7%, 8%, 9%, 10%, 11%, 12%, 13%, 14%, or 15% on a weight/weight (w/w) basis. Additionally, or alternatively, the concentration of rOVA in such compositions is at most 15%, 10%, 5%, 4%, 3%, or 2% on a w/w basis.

**Features and characteristics of rOVA compositions and food ingredients and food products containing rOVA**

The rOVA containing compositions herein can provide one or more functional features to food ingredients and food products. In some embodiments, the rOVA provides a nutritional feature such as protein content, protein fortification and amino acid content to a food ingredient or food product. The nutritional feature provided by rOVA in the composition may be comparable or substantially similar to an egg, egg white or native OVA (nOVA). The nutritional feature provided by rOVA in the composition may be better than that provided by a native whole egg or native egg white. In some cases, rOVA provides the one or more functional features of egg-white in absence of any other egg-white proteins.

rOVA compositions disclosed herein can provide foaming and foam capacity to a composition. For example, rOVA can be used for forming a foam to use in baked products, such as cakes, for meringues and other foods where rOVA can replace egg white to provide foam capacity. rOVA provides foaming and foam capacity of egg-white in absence of any other egg-white proteins.

A composition comprising rOVA may have a foam height greater than a foam height of an egg white or a composition comprising nOVA. In some cases, a composition comprising rOVA may have a foam height of about or at least 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white, nOVA compositions or a substitute egg white. In some cases, a composition comprising rOVA may have a foam height of up to 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white, nOVA compositions or a substitute egg white. Substitute egg whites may include products such as aquafaba, chia seeds, flax seeds, starches; apple sauce, banana puree; condensed milk, etc. which are commonly used as egg white substitutes.

A composition comprising rOVA may have a foam stability greater than a foam stability of an egg white, nOVA compositions or a substitute egg white. In some cases, a composition comprising rOVA may have a foam stability of about or at least 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white or a substitute egg white. In some cases, a composition comprising rOVA may have a foam stability of up to 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white. Foam stability may be calculated by measuring drainage of a foamed solution. The drainage may be measured in 10-minute increments for 30 minutes to gather data for foam stability. The drained volume after 30 minutes may be compared to the initial liquid volume (5mL) for instance, foam Stability (%):

$$(\text{Initial volume - drained volume}) / \text{initial volume} * 100.$$

A composition comprising rOVA may have a foam capacity greater than a foam capacity of an egg white, nOVA compositions or a substitute egg white. In some cases, a composition comprising rOVA may have a foam capacity of about or at least 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white, nOVA or a substitute egg white. In some cases, a composition comprising rOVA may have a foam capacity of up to 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 210%, 220%, 230%, 240%, 250%, 260%, 270%, 280%, 290%, 300%, 350%, 400%, 450%, or 500% relative to an egg white, nOVA compositions or a substitute egg white. Foam capacity may be determined by measuring the initial volume of foam following the whipping and compare against the initial volume of 5mL. Foam Capacity (%) = (volume of foam / initial volume)* 100.

A liquid composition may foam faster than a composition comprising egg whites, nOVA or a substitute egg white. In some cases, an rOVA composition foams at least 1%, 5%, 10%, 15%, 20%, 25%, 30%, 35%, 40%, 45%, 50%, 55%, 60%, 65%, 70%, 75%, 80%, 85%, 90%, 95%, 100%, faster than an egg white, nOVA or substitute egg-white composition. In some cases, an rOVA composition foams up to 1%, 5%, 10%, 15%, 20%, 25%, 30%, 35%, 40%, 45%, 50%, 55%, 60%, 65%, 70%, 75%, 80%, 85%, 90%, 95%, 100% faster than an egg white, nOVA or substitute egg-white composition.

A composition comprising rOVA may have a gel strength greater than a gel strength of an egg white, nOVA composition or a egg white substitutes. In some cases, the rOVA composition may have a gel strength within the range from 100 g to 1500 g, from 500 g to 1500 g, or from 700 g to 1500 g. In some cases, an rOVA composition has a gel strength of about or at least 10, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, or 1500 g. In some cases, an rOVA composition has a gel strength of up to 10, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, or 1500 g. In some cases, an rOVA composition has a gel strength of about or at least 50%, 55%, 60%, 65%, 70%, 75%, 80%, 85%, 90%, 95%, 100% relative to an egg white, nOVA or egg white substitutes. In some cases, an rOVA composition has a gel strength of up to 50%, 55%, 60%, 65%, 70%, 75%, 80%, 85%, 90%, 95%, 100% relative to an egg white, nOVA or egg white substitutes.

rOVA compositions disclosed herein can provide structure, texture or a combination of structure and texture. In some embodiments, rOVA is added to a food ingredient or food product for baking and the rOVA provides structure, texture or a combination of structure and texture to the baked product. rOVA is used in such baked products in place of native egg white, native egg or native egg protein. The addition of rOVA to baked products can also provide protein fortification to improve the nutritional content. rOVA provides the structure and/or texture of egg-white in absence of any other egg-white proteins.

rOVA compositions disclosed herein can be compatible with gluten formation, such that the rOVA can be used where gluten formation provides structure, texture and/or form to a food ingredient or food product.

Exemplary baked products in which rOVA can be used as an ingredient include, but are not limited to cake, cookie, bread, bagel, biscuits, muffin, cupcake, scone, pancake, macaroon, choux pastry, meringue, and soufflé. For example, rOVA can be used as an ingredient to make cakes such as pound cake, sponge cake, yellow cake, or angel food cake, where such cakes do not contain any native egg white, native whole egg or native egg protein. Along with rOVA, baked products may contain additional ingredients such as flour, sweetening agents, gum, hydrocolloids, starches, fibers, flavorings (such as flavoring extracts) and other protein sources. In some embodiments, a baked product may include rOVA and at least one fat or oil, at least one grain starch, and optionally at least one sweetener. Grain starch for use in such compositions include flours such as wheat flour, rice flour, corn flour, millet flour, spelt flour, and oat flour, and starches such as from corn, potato, sorghum, and arrowroot. Oil and fat for use in such compositions include plant-derived oils and fats, such as olive oil, corn oil, avocado oil, nut oils (e.g., almond, walnut and peanut) and safflower oil. rOVA may provide such baked goods with at least one characteristic of an egg white such as binding, springiness, aeration, browning, texturizing, humectant, and cohesiveness of the baked product. The baked product does not comprise any natural egg white or natural egg, and/or does not include any other egg white derived proteins except rOVA. In some cases, rOVA is provided to the baked composition as an ingredient, such as starting with a concentrate, isolate or powder form of rOVA. In some cases, the rOVA provided as an ingredient for baked products is at a pH range between about 3.5 and 7.0. In some cases, a sweetener is included in the baked product such as a sugar, syrup, honey or sugar-substitute.

rOVA compositions disclosed herein are used to prepare egg-less food products, such as food products made where native whole egg or native egg white is a primary or featured ingredient such as scramble, omelet, patty, soufflé, quiche and frittata. In some embodiments, rOVA provides one or more functional features to the preparation including foaming, coagulation, binding, structure, texture, film-formation, nutritional profile, absence of cholesterol (i.e., cholesterol free) and protein fortification. Such egg-less preparations can be vegan, vegetarian, halal, or kosher, or a combination thereof. An egg-less preparation (also referred to as an egg-white substitute) may include rOVA and at least one fat or oil, a polysaccharide or polysaccharide-containing ingredient, and a starch. In some cases, the egg-less preparation may also include a flavoring agent (such as to provide a salty, sulfur-like or umami flavor), and/or a coloring agent (for example to provide yellow-like or off-white color to the baked product). In some cases, the inclusion or rOVA in the egg-less preparation provides a characteristic of natural (native) egg white such as hardness, adhesiveness, fracturability, cohesiveness, gumminess and chewiness when the composition is heated or cooked. Exemplary polysaccharide or polysaccharide-containing ingredients for such compositions include gellan gum, sodium alginate, and psyllium. Oil and fat for use in such compositions include plant-derived oils and fats, such as olive oil, corn oil, avocado oil, and safflower oil.

rOVA compositions disclosed herein can be used for a processed meat product or meat-like product, or for fish-like or shell-fish-like products. In such products, rOVA can provide one or more functional characteristics such as protein content and protein supplementations as well as binding, texturizing properties. Exemplary meat and meat-like products include burger, patty, sausage, hot dog, sliced deli meat, jerky, bacon, nugget and ground meat-like mixtures. Meat-like products can resemble beef, pork, chicken, lamb and other edible and consumed meats for humans and for other animals. Fish-like and shell-fish like products can resemble, for example, fish cakes, crab cakes, shrimp, shrimp balls, fish sticks, seafood meat, crab meat, fish fillets and clam strips. rOVA is used for a meat-like product (also referred to as a meat-analog and includes at least one fat or oil; and a plant-derived protein. Oil and fat for use in such compositions include plant-derived oils and fats, such as olive oil, corn oil, avocado oil, and safflower oil. Plant-derived proteins for use in meat analogs include soy protein, nut proteins, pea protein, lentil and other pulse proteins and whey protein. In some cases, such plant protein is extruded, in other cases, such plant protein is non-extruded protein. In some cases, a meat analog include rOVA at about 2% to 15% (w/w). In some cases for meat analog compositions, rOVA acts as a binding agent, a gelling agent or a combination of a binding and gelling agent for such compositions.

rOVA compositions disclosed herein can be employed in coatings for food products. For example, rOVA can provide binding or adhesion characteristics to adhere batter or breading to another food ingredient. rOVA can be used as an "egg-less egg wash" where the rOVA protein provides appearance, color and texture when coated onto other food ingredients or food products, such as baked products. In one example, the "egg-less egg wash" may be used to coat a baked good such that the baked good adheres to a coating (e.g., seed, salt, spice, and herb). The addition of rOVA as a coating to a food product can provide a crunchy texture or increase the hardness, for example, of the exterior of a food product such as when the product is cooked, baked or fried.

rOVA compositions disclosed herein include sauces and dressings, such as an eggless mayonnaise, commercial mayonnaise substitutes, gravy, sandwich spread, salad dressing or food sauce. Inclusion of rOVA in a sauce or dressing, and the like, can provide one or more characteristics such as binding, emulsifying, odor neutrality, and mouthfeel. In some cases, the amount of rOVA in a sauce or dressing may be substantially similar to the amount of whole egg, egg-white or nOVA used in a commercially available or commonly used recipe. Exemplary sauces and dressing include mayonnaise, commercial mayonnaise substitutes, alfredo sauce, and hollandaise sauce. The rOVA-containing sauce or dressing does not contain whole egg, egg white, or any other protein extracted from egg. In some cases, the sauce, dressing or other emulsified product made with rOVA includes at least one fat or oil and water. Exemplary fats and oils for such compositions include corn oil, safflower oil, nut oils, and avocado oil.

rOVA compositions can be used to prepare confectionaries such as eggless, animal-free, vegetarian and vegan confectionaries. rOVA can provide one or more functional features to the confectionary including odor neutrality, flavor, mouthfeel, texture, gelling, cohesiveness, foaming, frothiness, nutritional value and protein fortification. The prepared confectionary containing rOVA does not contain any native egg protein or native egg white. rOVA in such confectionaries can provide a firm or chewy texture. Exemplary confectionaries include a gummy, a taffy, a divinity candy, meringue, marshmallow, and a nougat. In some embodiments, a confectionary includes rOVA, at least one sweetener and optionally a consumable liquid. Exemplary sweetners include sugar, honey, sugar-substitutes and plant-derived syrups. In some cases, the rOVA is provided as an ingredient for making confectionaries at a pH between about 3.5 and about 7. The rOVA is present in the confectionary composition at about 2% to about 15% (w/v). In some embodiments, the confectionary is a food product such as a meringue, a whipped dessert, or a whipped topping. In some embodiments, rOVA in the confectionary provides foaming, whipping, fluffing or aeration to the food product, and/or provides gelation. In some cases, the confectionary is a liquid, such as a foamed drink. In some cases, the liquid may include a consumable alcohol (such as in a sweetened cocktail or after-dinner drink).

rOVA compositions herein can be used in dairy products, dairy-like products or dairy containing products. For example, rOVA can be used in preparations of beverages such as a smoothie, milkshake, "egg-nog", and coffee beverage. In some embodiments, rOVA is added to additional ingredients where at least one ingredient is a dairy ingredient or dairy-derived ingredient (such as milk, cream, whey, and butter). rOVA is added to additional ingredients to create a beverage that does not contain any native egg protein, native egg white or native egg. In some embodiments, rOVA is an ingredient in a beverage that does not contain any animal-derived ingredients, such as one that does not contain any native egg-derived or any dairy-derived ingredients. Examples of such non-dairy derived drinks include nut milks, such as soy milk or almond milk. rOVA can also be used to create beverage additions, such as creamer or "milk" to provide protein, flavor, texture and mouthfeel to a beverage such as a coffee, tea, alcohol-based beverages or cocoa.

In some embodiments herein, rOVA can be used to prepare a dairy-like product such as yogurt, cheese or butter. Dairy products with rOVA can include other animal-based dairy components or proteins. In some embodiments, dairy products prepared with rOVA do not include any animal-based ingredients.

Preparations of dessert products can be prepared using rOVA. In dessert products rOVA can provide one or more characteristics such as creamy texture, low fat content, odor neutrality, flavor, mouthfeel, texture, binding, and nutritional value. rOVA may be present in an ingredient or set of ingredients that is used to prepare a dessert product. Exemplary dessert products suitable for preparation with rOVA include a mousse, a cheesecake, a custard, a pudding, a popsicle and an ice cream. In some embodiments, dessert products prepared to include rOVA are vegan, vegetarian or dairy-free.

rOVA can be used to prepare a snack food, such as a protein bar, an energy bar, a nutrition bar or a granola bar. The rOVA can provide characteristics to the snack food including one or more of binding, protein supplementation, flavor neutrality, odor neutrality, coating and mouth feel.

rOVA can be used for nutritional supplements such as in parenteral nutrition, protein drink supplements, protein shakes where rOVA provides a high protein supplement.

rOVA compositions is used as an egg-replacer and an egg white-replacer. rOVA can be mixed or combined with at least one additional component to form the egg white replacer. rOVA can provide one or more characteristics to the egg-replacer or egg white-replacer, such as gelling, foaming, whipping, fluffing, binding, springiness, aeration, creaminess and cohesiveness. In some embodiments, characteristic is the same or better than a native egg or native egg white provided in the same amount or concentration (w/w or w/v). The egg-replacer or egg white-replacer does not contain any egg, egg white, protein extracted or isolated from egg.

The rOVA-containing food ingredient and food products, such as described herein, can contain additional ingredients or components. For example, rOVA compositions can be prepared with an additional component such as one or more of a sweetener, a gum, a flavoring, a thickener, an acidulant and an emulsifier. Other ingredients such as flour, grains, oils and fats, fiber, fruit and vegetables can be combined with rOVA. Such rOVA compositions can be vegan, vegetarian, halal, kosher and animal-free, or a combination thereof. In some embodiments, rOVA can be a food ingredient or prepared for a food product that is normally animal based or normally contains animal-derived components, such as meat, dairy or eggs.

Compositions including rOVA including food ingredients and food products can be compatible with one or more steps of consumables preparation such as heated, baked, grilled, roasted, braised, microwaved, broiled, boiled, steamed, extruded, deep fried, or pan-fried, or processed using ohmic heating, Sue Vide, freezing, chilling, blanching, packaging, canning, bleaching, enriching, drying, pressing, grinding, mixing, par cooking, cooking, proofing, marinating, cutting, slicing, dicing, crushing, shredding, chopping, shaking, coring, spiralizing, rolling, juicing, straining, filtering, kneading, whisking, beating, whipping, grating, stuffing, peeling, smoking, curing, salting, preserving, pickling, fermenting, homogenizing, pasteurizing, sterilizing, irradiating, cold plasma processing, high pressure processing, pulse electric field processing, microwave assisted thermal sterilization, stabilizing, blending, pureeing, fortifying, refining, hydrogenating, aging, extending shelf life, or adding enzymes.

Food ingredients and food products prepared with rOVA can be essentially free of any microbial cells or microbial cell debris. For instance, rOVA may be secreted from a microbial host cell and isolated from microbial cells, culture media and/or microbial cell debris.

In some embodiments, rOVA may be prepared as a whole cell extract or fractionated extract such that an rOVA composition contains microbial cells and/or microbial cell components.

In one embodiment, an rOVA composition is prepared for animal consumption where the rOVA is present in a whole cell extract or fractionated extract such that an rOVA composition contains microbial cells and/or microbial cell components. In some embodiments, an rOVA composition is prepared for animal consumption where rOVA is isolated from microbial cells, culture media and microbial cell debris. Exemplary compositions for animal consumption can include a pet food, an animal feed, a chewy treat, bone broth, smoothie or other liquid for animal nutrition and a solid nutritional supplement suitable for animal consumption. In these cases, the microbial cell extract or microbial cell debris may provide additional nutritional value.

Animals which may consume rOVA compositions can include companion animals (e.g., dog, cat, horse), farm animals, exotic animals (lion, tiger, zebra) as well as livestock (such as cow, pig, sheep, goat). rOVA compositions as described herein can also be used for aquaculture (such as for fish and shell fish) and for avian nutrition (such as for bird pets, zoo birds, wild birds, fowl and birds raised for human and animal food).

In some embodiments of the consumable food compositions described herein, the composition is essentially free of animal-derived components, whey protein, caseinate, fat, lactose, hydrolyzed lactose, soy protein, collagen, hydrolyzed collagen, or gelatin, or any combination thereof. A composition described herein may be essentially free of cholesterol, glucose, fat, saturated fat, trans fat, or any combination thereof. In some cases, a composition described herein comprises less than 10%, 5%, 4%, 3%, 2%, 1%, or 0.5% fat by dry weight. In some embodiments, the composition may be fat-containing (e.g., such as a mayonnaise and commercial mayonnaise substitutes) and such composition may include up to about 60% fat or a reduced-fat composition (e.g., reduced fat mayonnaise and commercial mayonnaise substitutes) and such composition may include lesser percentages of fat. A composition that free of an animal-derived component can be considered vegetarian and/or vegan.

In some embodiments, an rOVA powder composition comprises less than 5% ash. The term "ash" is an art-known term and represents inorganics such as one or more ions, elements, minerals, and/or compounds In some cases, the rOVA powder composition comprises less than 5%, 4.5%, 4%, 3.5%, 3%, 2.5%, 2%, 1.5%, 1%, 0.75%, 0.5%, 0.25% or 0.1% ash weight per total weight (w/w) and/or weight per total volume (w/v).

In some embodiments, the moisture content of an rOVA powder composition may be less than 15%. The rOVA powder composition may have less than 15%, 12%, 10%, 8%, 6%, 5%, 3%, 2% or 1% moisture weight per total weight (w/w) and/or weight per total volume (w/v). In some embodiments, the carbohydrate content of an rOVA powder composition may be less than 30%. The rOVA powder composition may have less than 30%, 27%, 25%, 22%, 20%, 17%, 15%, 12%, 10%, 8%, 5%, 3% or 1% carbohydrate content w/w or w/v.

**Sensory Neutrality and Improved Sensory Appeal**

In some embodiments, in addition to the egg-white like properties, the addition of rOVA to a consumable food composition provides increased protein nutritional content, sensory neutrality or an improved sensory appeal as compared to other proteins in such compositions. As used herein "sensory neutrality" refers to the absence of a strong or distinctive taste, odor (smell) or combination of taste and smell, as well as texture, mouth-feel, aftertaste and color. A sensory panel such as one described in Kemp et al. 2009 may be used by a trained sensory analyst. Sensory neutrality may provide an improved sensory appeal to a taster, such as a tester of foods or a consumer, when a consumable food composition containing rOVA is compared with another like composition that has a different protein such as nOVA, whey protein, pea protein, soy protein, whole egg or egg white protein at the same concentration.

In some embodiments, rOVA when added to a consumable food composition is substantially odorless, such as measured by a trained sensory analyst, in comparison with different solutions/products with a different protein component present in an equal concentration to the rOVA containing solution/product, for example, in the comparison is whey, soy, collagen, pea, egg white solid isolates and/or nOVA. In some embodiments of the rOVA compositions described herein, such compositions are essentially odorless at a protein concentration between about 0.5-1%, 1%-5%, 5-10%, 10-15%, 15-20%, 20-25%, 25-30% rOVA weight per total weight (w/w) and/or weight per total volume (w/v) or at a protein concentration of about 0.1, 1, 5, 10, 11, 12, 13, 14, 15, 16, 17, 18, 19, 20, 21, 22, 23, 24, 25, 26, 27, 28, 29 or 30 g of total rOVA protein per 100 mL solution (e.g., per 100 mL water).

In some embodiments, the addition of rOVA to a consumable food composition also provides a neutral taste in addition to the characteristics such as egg-white like properties and increased protein nutrition content. A neutral taste can be measured for example, by a trained sensory analyst in comparison with solutions containing a different protein present in an equal concentration to the rOVA, for example, whey, soy, collagen, pea, whole egg, and egg white solid isolates (including native OVA).

In some embodiments, the addition of rOVA provides a reduction in a certain odor and/or taste that is associated with other proteins or egg-whites. For example, addition of rOVA has less of an "egg-like" odor or taste as compared to the addition of whole egg, fractionated egg or egg-white to a consumable food composition. In some embodiments, addition of rOVA has less of a metallic odor or taste as compared to other protein sources.

In some embodiments, the addition of rOVA has an improved mouth-feel as compared to the addition of other protein sources used to produce egg-white like properties. For example, the addition of rOVA is less grainy or has less precipitates or solids as compared to other protein sources.

In some embodiments, the addition of rOVA has an improved texture, for example, as compared to other available supplemental protein sources.

A consumable composition with rOVA may also have an improved sensory appeal as compared to the composition without rOVA or with a different protein present in an equal concentration to the rOVA. Such improved sensory appeal may relate to taste and/or smell. Taste and smell can be measured, for example, by a trained sensory analyst. In some instances, a sensory analyst compares a consumable composition with rOVA to one without it or with a different protein or protein source in an equivalent amount.

As described herein, a consumable composition herein can be in a liquid form. A liquid form can be an intermediate product such as soluble rOVA solution. In some cases, a liquid form can be a final product, such as a beverage comprising rOVA. Example of different types of beverages contemplated herein include: a juice, a soda, a soft drink, a flavored water, a protein water, a fortified water, a carbonated water, a nutritional drink, an energy drink, a sports drink, a recovery drink, an alcohol-based drink, a heated drink, a coffee-based drink, a tea-based drink, a plant-based milk, a nut milk, a milk based drink, a non-dairy, plant based mild drink, infant formula drink, and a meal replacement drink.

## pH of Compositions

The pH of an rOVA composition may be 3.5 to 8. The pH of an rOVA composition may be at least 3.5. The pH of an rOVA composition may be at most 8. The pH of an rOVA composition may be 3.5 to 4, 3.5 to 4.5, 3.5 to 5, 3.5 to 5.5, 3.5 to 6, 3.5 to 6.5, 3.5 to 7, 3.5 to 7.5, 3.5 to 8, 4 to 4.5, 4 to 5, 4 to 5.5, 4 to 6, 4 to 6.5, 4 to 7, 4 to 7.5, 4 to 8, 4.5 to 5, 4.5 to 5.5, 4.5 to 6, 4.5 to 6.5, 4.5 to 7, 4.5 to 7.5, 4.5 to 8, 5 to 5.5, 5 to 6, 5 to 6.5, 5 to 7, 5 to 7.5, 5 to 8, 5.5 to 6, 5.5 to 6.5, 5.5 to 7, 5.5 to 7.5, 5.5 to 8, 6 to 6.5, 6 to 7, 6 to 7.5, 6 to 8, 6.5 to 7, 6.5 to 7.5, 6.5 to 8, 7 to 7.5, 7 to 8, or 7.5 to 8. The pH of an rOVA composition may be 3.5, 4, 4.5, 5, 5.5, 6, 6.5, 7, 7.5, or 8. An rOVA composition with a pH between 3.5 to 7 may have one or more improved functionalities as compared to nOVA, egg white or egg-white substitute compositions.

The pH of an rOVA composition may be 2 to 3.5. The pH of an rOVA composition may be at least 2. The pH of an rOVA composition may be at most 3.5. The pH of an rOVA composition may be 2 to 2.5, 2 to 3, 2 to 3.5, 2.5 to 3, 2.5 to 3.5, or 3 to 3.5. The pH of an rOVA composition may be 2, 2.5, 3, or 3.5.

The pH of an rOVA composition may be 7 to 12. The pH of an rOVA composition may be at least 7. The pH of an rOVA composition may be at most 12. The pH of an rOVA composition may be 7 to 7.5, 7 to 8, 7 to 8.5, 7 to 9, 7 to 9.5, 7 to 10, 7 to 10.5, 7 to 11, 7 to 11.5, 7 to 12, 7.5 to 8, 7.5 to 8.5, 7.5 to 9, 7.5 to 9.5, 7.5 to 10, 7.5 to 10.5, 7.5 to 11, 7.5 to 11.5, 7.5 to 12, 8 to 8.5, 8 to 9, 8 to 9.5, 8 to 10, 8 to 10.5, 8 to 11, 8 to 11.5, 8 to 12, 8.5 to 9, 8.5 to 9.5, 8.5 to 10, 8.5 to 10.5, 8.5 to 11, 8.5 to 11.5, 8.5 to 12, 9 to 9.5, 9 to 10, 9 to 10.5, 9 to 11, 9 to 11.5, 9 to 12, 9.5 to 10, 9.5 to 10.5, 9.5 to 11, 9.5 to 11.5, 9.5 to 12, 10 to 10.5, 10 to 11, 10 to 11.5, 10 to 12, 10.5 to 11, 10.5 to 11.5, 10.5 to 12, 11 to 11.5, 11 to 12, or 11.5 to 12. The pH of an rOVA composition may be 7, 7.5, 8, 8.5, 9, 9.5, 10, 10.5, 11, 11.5, or 12.

In some embodiments, the pH of rOVA may be adjusted prior to its inclusion in a composition or its use as an ingredient. In some embodiments, the pH of rOVA is adjusted during the purification and/or isolation processes. In some embodiments, the pH of the rOVA for use in an ingredient or in production of a food product composition is adjusted to between about 3.5 to about 7.0. In some cases, the pH of rOVA may be adjusted to more than one pH during the production process. For example rOVA may be expressed in a host cell such as a a microbial cell, and in some cases the rOVA is secreted by the host cell into the growth media (e.g., liquid media). rOVA is separated from the host cells and such separation step may be performed at a selected pH, for example at a pH of about 3.5. In some cases, the rOVA at such separation pH may not be soluble or may not be fully soluble and the pH is adjusted to a higher pH, such as about pH 12. The rOVA may then be adjusted to a final pH between about 3.5 and about 7.0. Separation of rOVA from other components of the host cells or other components of the liquid media can include one or more of ion exchange chromatography, such as cation exchange chromatography and/or anion exchange chromatography, filtration and ammonium sulfate precipitation.

## Additional components of compositions

The consumable food compositions containing rOVA disclosed herein and the methods of making such compositions may including adding or mixing the rOVA with one or more ingredients. For example, food additives may be added in or mixed with the compositions. Food additives can add volume and/or mass to a composition. A food additive may improve functional performance and/or physical characteristics. For example, a food additive may prevent gelation or increased viscosity due to the lipid portion of the lipoproteins in the freeze-thaw cycle. An anticaking agent may be added to make a free-flowing composition. Carbohydrates can be added to increase resistance to heat damage, e.g., less protein denaturation during drying and improve stability and flowability of dried compositions. Food additives include, but are not limited to, food coloring, pH adjuster, natural flavoring, artificial flavoring, flavor enhancer, batch marker, food acid, filler, anticaking agent (e.g., sodium silico aluminate), anti greening agent (e.g., citric acid), food stabilizer, foam stabilizer or binding agent, antioxidant, acidity regulatory, bulking agent, color retention agent, whipping agent (e.g., ester-type whipping agent, triethyl citrate, sodium lauryl sulfate), emulsifier (e.g., lecithin), humectant, thickener, excipient, solid diluent, salts, nutrient, sweetener, glazing agent, preservative, vitamin, dietary elements, carbohydrates, polyol, gums, starches, flour, oil, or bran.

Food coloring includes, but is not limited to, FD&C Yellow #5, FD&C Yellow #6, FD&C Red #40, FD&C Red #3, FD&C Blue No. 1, FD&C Blue No. 2, FD&C Green No. 3, carotenoids (e.g., saffron, β-carotene), anthocyanins, annatto, betanin, butterfly pea, caramel coloring, chlorophyllin, elderberry juice, lycopene, carmine, pandan, paprika, turmeric, curcuminoids, quinoline yellow, carmoisine, Ponceau 4R, Patent Blue V, and Green S.

Ingredients for pH adjustment include, but are not limited to, Tris buffer, potassium phosphate, sodium hydroxide, potassium hydroxide, citric acid, sodium citrate, sodium bicarbonate, and hydrochloric acid.

Salts include, but are not limited, to acid salts, alkali salts, organic salts, inorganic salts, phosphates, chloride salts, sodium salts, sodium chloride, potassium salts, potassium chloride, magnesium salts, magnesium chloride, magnesium perchlorate, calcium salts, calcium chloride, ammonium chloride, iron salts, iron chlorides, zinc salts, and zinc chloride.

Nutrient includes, but is not limited to, macronutrient, micronutrient, essential nutrient, non-essential nutrient, dietary fiber, amino acid, essential fatty acids, omega-3 fatty acids, and conjugated linoleic acid.

Sweeteners include, but are not limited to, sugar substitute, artificial sweetener, acesulfame potassium, advantame, alitame, aspartame, sodium cyclamate, dulcin, glucin, neohesperidin dihydrochalcone, neotame, P-4000, saccharin, aspartame-acesulfame salt, sucralose, brazzein, curculin, glycyrrhizin, glycerol, inulin, mogroside, mabinlin, malto-oligosaccharide, mannitol, miraculin, monatin, monellin, osladin, pentadin, stevia, trilobatin, and thaumatin.

Carbohydrates include, but are not limited to, sugar, sucrose, glucose, fructose, galactose, lactose, maltose, mannose, allulose, tagatose, xylose, arabinose, high fructose corn syrup, high maltose corn syrup, corn syrup (e.g., glucose-free corn syrup), sialic acid, monosaccharides, disaccharides, polysaccharides (e.g., polydextrose, maltodextrin), and starch.

Polyols include, but are not limited to, xylitol, maltitol, erythritol, sorbitol, threitol, arabitol, hydrogenated starch hydrolysates, isomalt, lactitol, mannitol, and galactitol (dulcitol).

Gums include, but are not limited to, gum arabic, gellan gum, guar gum, locust bean gum, acacia gum, cellulose gum, and xanthan gum.

Vitamins include, but are not limited to, niacin, riboflavin, pantothenic acid, thiamine, folic acid, vitamin A, vitamin B6, vitamin B12, vitamin D, vitamin E, lutein, zeaxanthin, choline, inositol, and biotin.

Dietary elements include, but are not limited to, calcium, iron, magnesium, phosphorus, potassium, sodium, zinc, copper, manganese, selenium, chlorine, iodine, sulfur, cobalt, molybdenum, nickel, and bromine.

## rOVA protein and production of rOVA protein

rOVA can have an amino acid sequence from any species. For example, an rOVA can have an amino acid sequence of OVA from a bird or a reptile or other egg-laying species. An rOVA having an amino acid sequence from an avian can be selected from the group consisting of: poultry, fowl, waterfowl, game bird, chicken, quail, turkey, duck, ostrich, goose, gull, guineafowl, pheasant, emu, and any combination thereof. An rOVA can have an amino acid sequence derived from a single species, such as *Gallus gallus domesticus.* Alternatively, an rOVA can have an amino acid sequence derived from two or more species, and as such be a hybrid.

Exemplary OVA amino acid sequences contemplated herein are provided in Table 1 below as SEQ ID NOs: 1-74.

*Table 1. OVA Sequences*

| Name | SEQ ID | Sequence |
|------|--------|----------|
| Chicken Ovalbumin with bolded signal sequence | 1 | **MRFPSIFTAVLFAASSALAAPVNTTTEDETAQIPAEAVIGYSDLEGDFDVAVLP FSNSTNNGLLFINTTIASIAAKEEGVSLDKR***EAEA*GSIGAASMEFCFDVFKELKV HHANENIFYCPIAIMSALAMVYLGAKDSTRTQINKVVRFDKLPGFGDSIEAQCGTS VNVHSSLRDILNQITKPNDVYSFSLASRLYAEERYPILPEYLQCVKELYRGGLEPIN FQTAADQARELINSWVESQTNGIIRNVLQPSSVDSQTAMVLVNAIVFKGLWEKAF KDEDTQAMPFRVTEQESKPVQMMYQIGLFRVASMASEKMKILELPFASGTMSML VLLPDEVSGLEQLESIINFEKLTEWTSSNVMEERKIKVYLPRMKMEEKYNLTSVL MAMGITDVFSSSANLSGISSAESLKISQAVHAAHAEINEAGREVVGSAEAGVDAA SVSEEFRADHPFLFCIKHIATNAVLFFGRCVSP |
| Chicken OVA sequence as secreted from pichia | 2 | EAEAGSIGAASMEFCFDVFKELKVHHANENIFYCPIAIMSALAMVYLGAKDSTRT QINKVVRFDKLPGFGDSIEAQCGTSVNVHSSLRDILNQITKPNDVYSFSLASRLYA EERYPILPEYLQCVKELYRGGLEPINFQTAADQARELINSWVESQTNGIIRNVLQPS SVDSQTAMVLVNAIVFKGLWEKAFKDEDTQAMPFRVTEQESKPVQMMYQIGLF RVASMASEKMKILELPFASGTMSMLVLLPDEVSGLEQLESIINFEKLTEWTSSNV MEERKIKVYLPRMKMEEKYNLTSVLMAMGITDVFSSSANLSGISSAESLKISQAV HAAHAEINEAGREVVGSAEAGVDAASVSEEFRADHPFLFCIKHIATNAVLFFGRC VSP |
| Predicted Ovalbumin [*Achromobacter denitrificans*] | 3 | MRVPAQLLGLLLLWLPGARCGSIGAASMEFCFDVFKELKVHHANENIFYCPIAIM SALAMVYLGAKDSTRTQINKVVRFDKLPGFGDSIEAQCGTSVNVHSSLRDILNQIT KPNDVYSFSLASRLYAEERYPILPEYLQCVKELYRGGLEPINFQTAADQARELINS WVESQTNGIIRNVLQPSSVDSQTAMVLVNAIVFKGLWEKAFKDEDTQAMPFRVT EQESKPVQMMYQIGLFRVASMASEKMKILELPFASGTMSMLVLLPDEVSGLEQL ESIINFEKLTEWTSSNVMEERKIKVYLPRMKMEEKYNLTSVLMAMGITDVFSSSA NLSGISSAESLKISQAVHAAHAEINEAGREVVGSAEAGVDAASVSEEFRADHPFLF CIKHIATNAVLFFGRCVSPLEIKRAAAHHHHHH |
| OLLAS epitopetagged ovalbumin | 4 | MTSGFANELGPRLMGKLTMGSIGAASMEFCFDVFKELKVHHANENIFYCPIAIMS ALAMVYLGAKDSTRTQINKVVRFDKLPGFGDSIEAQCGTSVNVHSSLRDILNQIT KPNDVYSFSLASRLYAEERYPILPEYLQCVKELYRGGLEPINFQTAADQARELINS WVESQTNGIIRNVLQPSSVDSQTAMVLVNAIVFKGLWEKTFKDEDTQAMPFRVT EQESKPVQMMYQIGLFRVASMASEKMKILELPFASGTMSMLVLLPDEVSGLEQL ESIINFEKLTEWTSSNVMEERKIKVYLPRMKMEEKYNLTSVLMAMGITDVFSSSA NLSGISSAESLKISQAVHAAHAEINEAGREVVGSAEAGVDAASVSEEFRADHPFLF CIKHIATNAVLFFGRCVSPSR |

(continued)

| Name | SEQ ID | Sequence |
|------|--------|----------|
| Serpin family protein [*Achromobacter denitrificans*] | 5 | MGGRRVRWEVYISRAGYVNRQIAWRRHHRSLTMRVPAQLLGLLLLWLPGARCG SIGAASMEFCFDVFKELKVHHANENIFYCPIAIMSALAMVYLGAKDSTRTQINKV VRFDKLPGFGDSIEAQCGTSVNVHSSLRDILNQITKPNDVYSFSLASRLYAEERYPI LPEYLQCVKELYRGGLEPINFQTAADQARELINSWVESQTNGIIRNVLQPSSVDSQ TAMVLVNAIVFKGLWEKAFKDEDTQAMPFRVTEQESKPVQMMYQIGLFRVASM ASEKMKILELPFASGTMSMLVLLPDEVSGLEQLESIINFEKLTEWTSSNVMEERKI KVYLPRMKMEEKYNLTSVLMAMGITDVFSSSANLSGISSAESLKISQAVHAAHAE INEAGREVVGSAEAGVDAASVSEEFRADHPFLFCIKHIATNAVLFFGRCVSPLEIK RAAAHHHHHH |
| PREDICTED: ovalbumin isoform X1 [*Meleagris gallopavo*] | 6 | MGSIGAVSMEFCFDVFKELKVHHANENIFYSPFTIISALAMVYLGAKDSTRTQINK VVRFDKLPGFGDSVEAQCGTSVNVHSSLRDILNQITKPNDVYSFSLASRLYAEETY PILPEYLQCVKELYRGGLESINFQTAADQARGLINSWVESQTNGMIKNVLQPSSV DSQTAMVLVNAIVFKGLWEKAFKDEDTQAIPFRVTEQESKPVQMMYQIGLFKVA SMASEKMKILELPFASGTMSMWVLLPDEVSGLEQLETTISFEKMTEWISSNIMEER RIKVYLPRMKMEEKYNLTSVLMAMGITDLFSSSANLSGISSAGSLKISQAVHAAY AEIYEAGREVIGSAEAGADATSVSEEFRVDHPFLYCIKHNLTNSILFFGRCISP |
| Ovalbumin precursor [*Meleagris gallopavo*] | 7 | MGSIGAVSMEFCFDVFKELKVHHANENIFYSPFTIISALAMVYLGAKDSTRTQINK VVRFDKLPGFGDSVEAQCGTSVNVHSSLRDILNQITKPNDVYSFSLASRLYAEETY PILPEYLQCVKELYRGGLESINFQTAADQARGLINSWVESQTNGMIKNVLQPSSV DSQTAMVLVNAIVFKGLWEKAFKDEDTQAIPFRVTEQESKPVQMMYQIGLFKVA SMASEKMKILELPFASGTMSMWVLLPDEVSGLEQLETTISFEKMTEWISSNIMEER RIKVYLPRMKMEEKYNLTSVLMAMGITDLFSSSANLSGISSAGSLKISQAAHAAY AEIYEAGREVIGSAEAGADATSVSEEFRVDHPFLYCIKHNLTNSILFFGRCISP |
| Hypothetical protein [*Bambusicola thoracicus*] | 8 | YYRVPCMVLCTAFHPYIFIVLLFALDNSEFTMGSIGAVSMEFCFDVFKELRVHHPN ENIFFCPFAIMSAMAMVYLGAKDSTRTQINKVIRFDKLPGFGDSTEAQCGKSANV HSSLKDILNQITKPNDVYSFSLASRLYADETYSIQSEYLQCVNELYRGGLESINFQT AADQARELINSWVESQTNGIIRNVLQPSSVDSQTAMVLVNAIVFRGLWEKAFKDE DTQTMPFRVTEQESKPVQMMYQIGSFKVASMASEKMKILELPLASGTMSMLVLL PDEVSGLEQLETTISFEKLTEWTSSNVMEERKIKVYLPRMKMEEKYNLTSVLMA MGITDLFRSSANLSGISLAGNLKISQAVHAAHAEINEAGRKAVSSAEAGVDATSV SEEFRADRPFLFCIKHIATKVVFFFGRYTSP |

(continued)

| Name | SEQ ID | Sequence |
|---|---|---|
| Egg albumin | 9 | MGSIGAASMEFCFDVFKELKVHHANDNMLYSPFAILSTLAMVFLGAKDSTRTQIN KVVHFDKLPGFGDSIEAQCGTSVNVHSSLRDILNQITKQNDAYSFSLASRLYAQET YTVVPEYLQCVKELYRGGLESVNFQTAADQARGLINAWVESQTNGIIRNILQPSS VDSQTAMVLVNAIAFKGLWEKAFKAEDTQTIPFRVTEQESKPVQMMYQIGSFKV ASMASEKMKILELPFASGTMSMLVLLPDDVSGLEQLESIISFEKLTEWTSSSIMEER KVKVYLPRMKMEEKYNLTSLLMAMGITDLFSSSANLSGISSVGSLKISQAVHAAH AEINEAGRDVVGSAEAGVDATEEFRADHPFLFCVKHIETNAILLFGRCVSP |
| Ovalbumin isoform X2 [*Numida meleagris*] | 10 | MASIGAVSTEFCVDVYKELRVHHANENIFYSPFTIISTLAMVYLGAKDSTRTQINK VVRFDKLPGFGDSIEAQCGTSVNVHSSLRDILNQITKPNDVYSFSLASRLYAEETY PILPEYLQCVKELYRGGLESINFQTAADQARELINSWVESQTSGIIKNVLQPSSVNS QTAMVLVNAIYFKGLWERAFKDEDTQAIPFRVTEQESKPVQMMSQIGSFKVASV ASEKVKILELPFVSGTMSMLVLLPDEVSGLEQLESTISTEKLTEWTSSSIMEERKIK VFLPRMRMEEKYNLTSVLMAMGMTDLFSSSANLSGISSAESLKISQAVHAAYAEI YEAGREVVSSAEAGVDATSVSEEFRVDHPFLLCIKHNPTNSILFFGRCISP |
| Ovalbumin isoform X1 [*Numida meleagris*] | 11 | MALCKAFHPYIFIVLLFDVDNSAFTMASIGAVSTEFCVDVYKELRVHHANENIFYS PFTIISTLAMVYLGAKDSTRTQINKVVRFDKLPGFGDSIEAQCGTSVNVHSSLRDIL NQITKPNDVYSFSLASRLYAEETYPILPEYLQCVKELYRGGLESINFQTAADQARE LINSWVESQTSGIIKNVLQPSSVNSQTAMVLVNAIYFKGLWERAFKDEDTQAIPFR VTEQESKPVQMMSQIGSFKVASVASEKVKILELPFVSGTMSMLVLLPDEVSGLEQ LESTISTEKLTEWTSSSIMEERKIKVFLPRMRMEEKYNLTSVLMAMGMTDLFSSSA NLSGISSAESLKISQAVHAAYAEIYEAGREVVSSAEAGVDATSVSEEFRVDHPFLL CIKHNPTNSILFFGRCISP |
| PREDICTED: Ovalbumin isoform X2 [*Coturnix japonica*] | 12 | MGSIGAASMEFCFDVFKELKVHHANDNMLYSPFAILSTLAMVFLGAKDSTRTQIN KVVHFDKLPGFGDSIEAQCGTSANVHSSLRDILNQITKQNDAYSFSLASRLYAQET YTVVPEYLQCVKELYRGGLESVNFQTAADQARGLINAWVESQTNGIIRNILQPSS VDSQTAMVLVNAIAFKGLWEKAFKAEDTQTIPFRVTEQESKPVQMMHQIGSFKV ASMASEKMKILELPFASGTMSMLVLLPDDVSGLEQLESTISFEKLTEWTSSSIMEE RKVKVYLPRMKMEEKYNLTSLLMAMGITDLFSSSANLSGISSVGSLKISQAVHAA YAEINEAGRDVVGSAEAGVDATEEFRADHPFLFCVKHIETNAILLFGRCVSP |

(continued)

| Name | SEQ ID | Sequence |
|---|---|---|
| PREDICTED: ovalbumin isoform X1 [*Coturnix japonica*] | 13 | MGLCTAFHPYIFIVLLFALDNSEFTMGSIGAASMEFCFDVFKELKVHHANDNMLY SPFAILSTLAMVFLGAKDSTRTQINKVVHFDKLPGFGDSIEAQCGTSANVHSSLRD ILNQITKQNDAYSFSLASRLYAQETYTVVPEYLQCVKELYRGGLESVNFQTAADQ ARGLINAWVESQTNGIIRNILQPSSVDSQTAMVLVNAIAFKGLWEKAFKAEDTQTI PFRVTEQESKPVQMMHQIGSFKVASMASEKMKILELPFASGTMSMLVLLPDDVS GLEQLESTISFEKLTEWTSSSIMEERKVKVYLPRMKMEEKYNLTSLLMAMGITDL FSSSANLSGISSVGSLKISQAVHAAYAEINEAGRDVVGSAEAGVDATEEFRADHPF LFCVKHIETNAILLFGRCVSP |
| Egg albumin | 14 | MGSIGAASMEFCFDVFKELKVHHANDNMLYSPFAILSTLAMVFLGAKDSTRTQIN KVVHFDKLPGFGDSIEAQCGTSANVHSSLRDILNQITKQNDAYSFSLASRLYAQET YTVVPEYLQCVKELYRGGLESVNFQTAADQARGLINAWVESQTNGIIRNILQPSS VDSQTAMVLVNAIAFKGLWEKAFKAEDTQTIPFRVTEQESKPVQMMHQIGSFKV ASMASEKMKILELPFASGTMSMLVLLPDDVSGLEQLESTISFEKLTEWTSSSIMEE RKVKVYLPRMKMEEKYNLTSLLMAMGITDLFSSSANLSGISSVGSLKIPQAVHAA YAEINEAGRDVVGSAEAGVDATEEFRADHPFLFCVKHIETNAILLFGRCVSP |
| ovalbumin [*Anas platyrhynchos*] | 15 | MGSIGAASTEFCFDVFRELRVQHVNENIFYSPFSIISALAMVYLGARDNTRTQIDK VVHFDKLPGFGESMEAQCGTSVSVHSSLRDILTQITKPSDNFSLSFASRLYAEETY AILPEYLQCVKELYKGGLESISFQTAADQARELINSWVESQTNGIIKNILQPSSVDS QTTMVLVNAIYFKGMWEKAFKDEDTQAMPFRMTEQESKPVQMMYQVGSFKVA MVTSEKMKILELPFASGMMSMFVLLPDEVSGLEQLESTISFEKLTEWTSSTMMEE RRMKVYLPRMKMEEKYNLTSVFMALGMTDLFSSSANMSGISSTVSLKMSEAVH AACVEIFEAGRDVVGSAEAGMDVTSVSEEFRADHPFLFFIKHNPTNSILFFGRWM SP |
| PREDICTED: ovalbumin-like [*Anser cygnoides domesticus*] | 16 | MGSIGAASTEFCFDVFRELKVQHVNENIFYSPLSIISALAMVYLGARDNTRTQIDQ VVHFDKIPGFGESMEAQCGTSVSVHSSLRDILTEITKPSDNFSLSFASRLYAEETYT ILPEYLQCVKELYKGGLESISFQTAADQARELINSWVESQTNGIIKNILQPSSVDSQ TTMVLVNAIYFKGMWEKAFKDEDTQTMPFRMTEQESKPVQMMYQVGSFKLAT VTSEKVKILELPFASGMMSMCVLLPDEVSGLEQLETTISFEKLTEWTSSTMMEER RMKVYLPRMKMEEKYNLTSVFMALGMTDLFSSSANMSGISSTVSLKMSEAVHA ACVEIFEAGRDVVGSAEAGMDVTSVSEEFRADHPFLFFIKHNPSNSILFFGRWISP |

(continued)

| Name | SEQ ID | Sequence |
|------|--------|----------|
| PREDICTED: Ovalbumin-like [*Aquila chrysaetos canadensis*] | 17 | MGSIGAASTEFCFDVFKELKVQHVNENIFYSPLTIISALSMVYLGARENTRAQIDK VLHFDKMPGFGDTIESQCGTSVSIHTSLKDMFTQITKPSDNYSLSFASRLYAEETY PILPEYLQCVKELYKGGLETISFQTAAEQARELINSWVESQTNGMIKNILQPSSVDP QTKMVLVNAIYFKGVWEKAFKDEDTQEVPFRVTEQESKPVQMMYQIGSFKVAV MASEKMKILELPYASGQLSMLVLLPDDVSGLEQLESAITFEKLMAWTSSTTMEER KMKVYLPRMKIEEKYNLTSVLMALGVTDLFSSSANLSGISSAESLKISKAVHEAF VEIYEAGSEVVGSTEAGMEVTSVSEEFRADHPFLFLIKHNPTNSILFFGRCFSP |
| PREDICTED: Ovalbumin-like [*Haliaeetus albicilla*] | 18 | MGSIGAASTEFCFDVFKELKVQHVNENIFYSPLTIISALSMVYLGARENTRTQIDK VLHFDKMTGFGDTVESQCGTSVSIHTSLKDIFTQITKPSDNYSLSLASRLYAEETYP ILPEYLQCVKELYKGGLETVSFQTAAEQARELINSWVESQTNGMIKNILQPSSVDP QTKMVLVNAIYFKGVWEKAFKDEDTQEVPFRVTEQESKPVQMMYQIGSFKVAV MASEKMKILELPYASGQLSMLVLLPDDVSGLEQLESAITSEKLMEWTSSTTMEER KMKVYLPRMKIEEKYNLTSVLMALGVTDLFSSSADLSGISSAESLKISKAVHEAF VEIYEAGSEVVGSTEGGMEVTSVSEEFRADHPFLFLIKHKPTNSILFFGRCFSP |
| PREDICTED: Ovalbumin-like [*Haliaeetus leucocephalus*] | 19 | MGSIGAASTEFCFDVFKELKVQHVNENIFYSPLTIISALSMVYLGARENTRTQIDK VLHFDKMTGFGDTVESQCGTSVSIHTSLKDIFTQITKPSDNYSLSLASRLYAEETYP ILPEYLQCVKELYKGGLETVSFQTAAEQARELINSWVESQTNGMIKNILQPSSVDP QTKMVLVNAIYFKGVWEKAFKDEDTQEVPFRVTEQESKPVQMMYQIGSFKVAV MASEKMKILELPYASGQLSMLVLLPDDVSGLEQLESAITSEKLMEWTSSTTMEER KMKVYLPRMKIEEKYNLTSVLMALGVTDLFSSSADLSGISSAESLKISKAVHEAF VEIYEAGSEVVGSTEGGMEVTSFSEEFRADHPFLFLIKHKPTNSILFFGRCFSP |
| PREDICTED: Ovalbumin [*Fulmarus glacialis*] | 20 | MGSIGAASTEFCFDVFKELKVQHVNENIFYSPLSIISALSMVYLGARENTRAQIDK VVHFDKITGFGETIESQCGTSVSVHTSLKDMFTQITKPSDNYSLSFASRLYAEETYP ILPEYLQCVKELYKGGLETTSFQTAADQARELINSWVESQTNGMIKNILQPGSVDP QTEMVLVNAIYFKGMWEKAFKDEDTQAVPFRMTEQESKTVQMMYQIGSFKVAV MASEKMKILELPYASGELSMLVMLPDDVSGLEQLETAITFEKLMEWTSSNMMEE RKMKVYLPRMKMEEKYNLTSVLMALGVTDLFSSSANLSGISSAESLKMSEAVHE AFVEIYEAGSEVVGSTGAGMEVTSVSEEFRADHPFLFLIKHNPTNSILFFGRCFSP |
| PREDICTED: Ovalbumin-like [*Chlamydotis macqueenii*] | 21 | MGSIGAASTEFCFDVFKELRVQHVNENVCYSPLIIISALSLVYLGARENTRAQIDK VVHFDKITGFGESIESQCGTSVSVHTSLKDMFNQITKPSDNYSLSVASRLYAEERY PILPEYLQCVKELYKGGLESISFQTAADQAREAINSWVESQTNGMIKNILQPSSVD PQTEMVLVNAIYFKGMWQKAFKDEDTQAVPFRISEQESKPVQMMYQIGSFKVAV MAAEKMKILELPYASGELSMLVLLPDEVSGLEQLENAITVEKLMEWTSSSPMEER IMKVYLPRMKIEEKYNLTSVLMALGITDLFSSSANLSGISAEESLKMSEAVHQAFA EISEAGSEVVGSSEAGIDATSVSEEFRADHPFLFLIKHNATNSILFFGRCFSP |

(continued)

| Name | SEQ ID | Sequence |
|------|--------|----------|
| PREDICTED: Ovalbumin like [*Nipponia nippon*] | 22 | MGSISAASTEFCFDVFKELKVQHVNENIFYSPLSIISALSMVYLGARENTRAQIEKV VHFDKITGFGESIESQCSTSVSVHTSLKDMFTQITKPSDNYSLSFASRFYAEETYPIL PEYLQCVKELYKGGLETINFRTAADQARELINSWVESQTNGMIKNILQPGSVDPQ TDMVLVNAIYFKGMWEKAFKDEDTQALPFRVTEQESKPVQMMYQIGSFKVAVL ASEKVKILELPYASGQLSMLVLLPDDVSGLEQLETAITVEKLMEWTSSNNMEERK IKVYLPRIKIEEKYNLTSVLMALGITDLFSSSANLSGISSAESLKVSEAIHEAFVEIYE AGSEVAGSTEAGIEVTSVSEEFRADHPFLFLIKHNATNSILFFGRCFSP |
| PREDICTED: Ovalbumin-like isoform X2 [*Gavia stellata*] | 23 | MVSIGAASTEFCFDVFKELKVQHVNENIFYSPLSIISALSMVYLGARENTRAQIDK VVHFDKITGFEETIESQCSTSVSVHTSLKDMFTQITKPSDNYSLSFASRLYAEETYPI LPEYLQCVKELYKGGLETISFQTAADQARELINSWVESQTDGMIKNILQPGSVDP QTEMVLVNAIYFKGMWEKAFKDEDTQAVPFRMTEQESKPVQMMYQIGSFKVAV MASEKMKILELPYASGGMSMLVMLPDDVSGLEQLETAITFEKLMEWTSSNMME ERKMKVYLPRMKMEEKYNLTSVLMALGMTDLFSSSANLSGISSAESLKMSEAVH EAFVEIYEAGSEAVGSTGAGMEVTSVSEEFRADHPFLFLIKHNPTNSILFFGRCFSP |
| PREDICTED: Ovalbumin [*Pelecanus crispus*] | 24 | MGSIGAASTEFCFDVFKELKVQHVNENIFYSPLSIISALSMVYLGARENTRAQIDK VVHFDKITGFGEPIESQCGISVSVHTSLKDMITQITKPSDNYSLSFASRLYAEETYPI LPEYLQCVKELYKGGLETISFQTAADQARELINSWVENQTNGMIKNILQPGSVDP QTEMVLVNAVYFKGMWEKAFKDEDTQAVPFRMTEQESKPVQMMYQIGSFKVA VMASEKIKILELPYASGELSMLVLLPDDVSGLEQLETAITLDKLTEWTSSNAMEER KMKVYLPRMKIEKKYNLTSVLIALGMTDLFSSSANLSGISSAESLKMSEAIHEAFL EIYEAGSEVVGSTEAGMEVTSVSEEFRADHPFLFLIKHNPTNSILFFGRCLSP |
| PREDICTED: Ovalbumin-like [*Charadrius vociferus*] | 25 | MGSIGAASTEFCFDVFKELKVQHVNENIFYSPLTIISALSMVYLGARENTRAQIDK VVHFDKIPGFGDTTESQCGTSVSVHTSLKDMFTQITKPSDNYSVSFASRLYAEETY PILPEFLECVKELYKGGLESISFQTAADQARELINSWVESQTNGMIKNILQPGSVDS QTEMVLVNAIYFKGMWEKAFKDEDTQTVPFRMTEQETKPVQMMYQIGTFKVAV MPSEKMKILELPYASGELCMLVMLPDDVSGLEELESSITVEKLMEWTSSNMMEE RKMKVFLPRMKIEEKYNLTSVLMALGMTDLFSSSANLSGISSAEPLKMSEAVHEA FIEIYEAGSEVVGSTGAGMEITSVSEEFRADHPFLFLIKHNPTNSILFFGRCVSP |
| PREDICTED: Ovalbumin-like [*Eurypyga helias*] | 26 | MGSIGAVSTEFCFDVFKELKVQHVNENIFYSPLSIISALSMVYLGARENTRAQIDK VVHFDKITGSGETIEAQCGTSVSVHTSLKDMFTQITKPSENYSVGFASRLYADETY PIIPEYLQCVKELYKGGLEMISFQTAADQARELINSWVESQTNGMIKNILQPGSVD PQTEMILVNAIYFKGVWEKAFKDEDTQAVPFRMTEQESKPVQMMYQFGSFKVA AMAAEKMKILELPYASGALSMLVLLPDDVSGLEQLESAITFEKLMEWTSSNMME EKKIKVYLPRMKMEEKYNFTSVLMALGMTDLFSSSANLSGISSADSLKMSEVVH EAFVEIYEAGSEVVGSTGSGMEAASVSEEFRADHPFLFLIKHNPTNSILFFGRCFSP |

(continued)

| Name | SEQ ID | Sequence |
|------|--------|----------|
| PREDICTED: Ovalbumin-like isoform X1 [*Gavia stellata*] | 27 | MVSIGAASTEFCFDVFKELKVQHVNENIFYSPLSIISALSMVYLGARENTRAQIDK VVHFDKITGFEETIESQVQKKQCSTSVSVHTSLKDMFTQITKPSDNYSLSFASRLY AEETYPILPEYLQCVKELYKGGLETISFQTAADQARELINSWVESQTDGMIKNILQ PGSVDPQTEMVLVNAIYFKGMWEKAFKDEDTQAVPFRMTEQESKPVQMMYQIG SFKVAVMASEKMKILELPYASGGMSMLVMLPDDVSGLEQLETAITFEKLMEWTS SNMMEERKMKVYLPRMKMEEKYNLTSVLMALGMTDLFSSSANLSGISSAESLK MSEAVHEAFVEIYEAGSEAVGSTGAGMEVTSVSEEFRADHPFLFLIKHNPTNSILF FGRCFSP |
| PREDICTED: Ovalbumin -like [*Egretta garzetta*] | 28 | MGSIGAASGEFCFDVFKELKVQHVNENIFYSPLSIISALSMVYLGARENTRAQIDK VVHFDKIIGFGESIESQCGTSVSVHTSLKDMFAQITKPSDNYSLSFASRLYAEETFPI LPEYLQCVKELYKGGLETLSFQTAADQARELINSWVESQTNGMIKDILQPGSVDP QTEMVLVNAIYFKGVWEKAFKDEDTQTVPFRMTEQESKPVQMMYQIGSFKVAV VAAEKIKILELPYASGALSMLVLLPDDVSSLEQLETAITFEKLTEWTSSNIMEERKI KVYLPRMKIEEKYNLTSVLMDLGITDLFSSSANLSGISSAESLKVSEAIHEAIVDIY EAGSEVVGSSGAGLEGTSVSEEFRADHPFLFLIKHNPTSSILFFGRCFSP |
| PREDICTED: Ovalbumin-like [*Balearica regulorum gibbericeps*] | 29 | MGSIGAASTEFCFDVFKELKVQHVNENIFYSPLSIISALSMVYLGARENTRAQIDK VVHFDKITGSGEAIESQCGTSVSVHISLKDMFTQITKPSDNYSLSFASRLYAEETYP ILPEYLQCVKELYKEGLATISFQTAADQAREFINSWVESQTNGMIKNILQPGSVDP QTQMVLVNAIYFKGVWEKAFKDEDTQAVPFRMTKQESKPVQMMYQIGSFKVAV MASEKMKILELPYASGQLSMLVMLPDDVSGLEQIENAITFEKLMEWTNPNMMEE RKMKVYLPRMKMEEKYNLTSVLMALGMTDLFSSSANLSGISSAESLKMSEAVHE AFVEIYEAGSEVVGSTGAGIEVTSVSEEFRADHPFLFLIKHNPTNSILFFGRCFSP |
| PREDICTED: Ovalbumin-like [*Nestor notabilis*] | 30 | MGSIGEASTEFCIDVFRELKVQHVNENIFYSPLSIISALSMVYLGARENTRAQIDQV VHFDKITGFGDTVESQCGSSLSVHSSLKDIFAQITQPKDNYSLNFASRLYAEETYPI LPEYLQCVKELYKGGLETISFQTAADQARELINSWVESQTNGMIKNILQPSSVDPQ TEMVLVNAIYFKGVWEKAFKDEETQAVPFRITEQENRPVQIMYQFGSFKVAVVA SEKIKILELPYASGQLSMLVLLPDEVSGLEQLENAITFEKLTEWTSSDIMEEKKIKV FLPRMKIEEKYNLTSVLVALGIADLFSSSANLSGISSAESLKMSEAVHEAFVEIYEA GSEVVGSSGAGIEAASDSEEFRADHPFLFLIKHKPTNSILFFGRCFSP |
| PREDICTED: Ovalbumin-like [*Pygoscelis adeliae*] | 31 | MGSIGAASTEFCFDIFNELKVQHVNENIFYSPLSIISALSMVYLGARENTKAQIDKV VHFDKITGFGESIESQCSTSASVHTSFKDMFTQITKPSDNYSLSFASRLYAEETYPIL PEYSQCVKELYKGGLESISFQTAADQARELINSWVESQTNGMIKNILQPGSVDPQT ELVLVNAIYFKGTWEKAFKDKDTQAVPFRVTEQESKPVQMMYQIGSYKVAVIAS EKMKILELPYASGELSMLVLLPDDVSGLEQLETAITFEKLMEWTSSNMMEERKV KVYLPRMKIEEKYNLTSVLMALGMTDLFSPSANLSGISSAESLKMSEAIHEAFVEI YEAGSEVVGSTEAGMEVTSVSEEFRADHPFLFLIKCNLTNSILFFGRCFSP |

(continued)

| Name | SEQ ID | Sequence |
|------|--------|----------|
| Ovalbumin-like [*Athene cunicularia*] | 32 | MGSISTASTEFCFDVFKELKVQHVNENIFYSPLSIISALSMVYLGARENTRAQIEKV VHFDKITGFGESIESQCGTSVSVHTSLKDMLIQISKPSDNYSLSFASKLYAEETYPIL PEYLQCVKELYKGGLESINFQTAADQARQLINSWVESQTNGMIKDILQPSSVDPQ TEMVLVNAIYFKGIWEKAFKDEDTQEVPFRITEQESKPVQMMYQIGSFKVAVIAS EKIKILELPYASGELSMLIVLPDDVSGLEQLETAITFEKLIEWTSPSIMEERKTKVYL PRMKIEEKYNLTSVLMALGMTDLFSPSANLSGISSAESLKMSEAIHEAFVEIYEAG SEVVGSAEAGMEATSVSEFRVDHPFLFLIKHNPANIILFFGRCVSP |
| PREDICTED: Ovalbumin-like [*Calidris pugnax*] | 33 | MGSIGAASTEFCFDVFKELKVQHVNENIFYSPLTIISALSLVYLGARENTRAQIDKV FHFDKISGFGETTESQCGTSVSVHTSLKEMFTQITKPSDNYSVSFASRLYAEDTYPI LPEYLQCVKELYKGGLETISFQTAADQAREVINSWVESQTNGMIKNILQPGSVDS QTEMVLVNAIYFKGMWEKAFKDEDTQTMPFRITEQERKPVQMMYQAGSFKVAV MASEKMKILELPYASGEFCMLIMLPDDVSGLEQLENSFSFEKLMEWTTSNMMEE RKMKVYIPRMKMEEKYNLTSVLMALGMTDLFSSSANLSGISSAETLKMSEAVHE AFMEIYEAGSEVVGSTGSGAEVTGVYEEFRADHPFLFLVKHKPTNSILFFGRCVSP |
| PREDICTED: Ovalbumin [*Aptenodytes forsteri*] | 34 | MGSIGAASTEFCFDIFNELKVQHVNENIFYSPLSIISALSMVYLGARENTKAQIDKV VHFDKITGFGETIESQCSTSVSVHTSLKDTFTQITKPSDNYSLSFASRLYAEETYPIL PEYSQCVKELYKGGLETISFQTAADQARELINSWVESQTNGMIKNILQPGSVDPQT ELVLVNAIYFKGTWEKAFKDKDTQAVPFRVTEQESKPVQMMYQIGSYKVAVIAS EKMKILELPYASRELSMLVLLPDDVSGLEQLETAITFEKLMEWTSSNMMEERKVK VYLPRMKIEEKYNLTSVLMALGMTDLFSPSANLSGISSAESLKMSEAVHEAFVEIY EAGSEVVGSTGAGMEVTSVSEEFRADHPFLFLIKCNPTNSILFFGRCFSP |
| PREDICTED: Ovalbumin-like [*Pterocles gutturalis*] | 35 | MGSISAASAEFCLDVFKELKVQHVNENIFYSPLSIISALSMVYLGARENTRAQIDK VVHFDKITGSGETIEFQCGTSANIHPSLKDMFTQITRLSDNYSLSFASRLYAEERYP ILPEYLQCVKELYKGGLETISFQTAADQARELINSWVESQTNGMIKNILQPGSVNP QTEMVLVNAIYFKGLWEKAFKDEDTQTVPFRMTEQESKPVQMMYQVGSFKVAV MASDKIKILELPYASGELSMLVLLPDDVTGLEQLETSITFEKLMEWTSSNVMEERT MKVYLPHMRMEEKYNLTSVLMALGVTDLFSSSANLSGISSAESLKMSEAVHEAF VEIYESGSQVVGSTGAGTEVTSVSEEFRVDHPFLFLIKHNPTNSILFFGRCFSP |
| Ovalbumin-like [*Falco peregrinus*] | 36 | MGSIGAASVEFCFDVFKELKVQHVNENIFYSPLSIISALSMVYLGARENTKAQIDK VVHFDKIAGFGEAIESQCVTSASIHSLKDMFTQITKPSDNYSLSFASRLYAEEAYSI LPEYLQCVKELYKGGLETISFQTAADQARDLINSWVESQTNGMIKNILQPGAVDL ETEMVLVNAIYFKGMWEKAFKDEDTQTVPFRMTEQESKPVQMMYQVGSFKVA VMASDKIKILELPYASGQLSMVVVLPDDVSGLEQLEASITSEKLMEWTSSSIMEEK KIKVYFPHMKIEEKYNLTSVLMALGMTDLFSSSANLSGISSAEKLKVSEAVHEAF VEISEAGSEVVGSTEAGTEVTSVSEEFKADHPFLFLIKHNPTNSILFFGRCFSP |

(continued)

| Name | SEQ ID | Sequence |
|---|---|---|
| PREDICTED: Ovalbumin -like isoform X2 [*Phalacrocorax carbo*] | 37 | MGSIGAASSEFCFDIFKELKVQHVNENIFYSPLSIISALSMVYLGARENTRAQIDKV VPFDKITASGESIESQCSTSVSVHTSLKDIFTQITKSSDNHSLSFASRLYAEETYPILP EYLQCVKELYEGGLETISFQTAADQARELINSWIESQTNGRIKNILQPGSVDPQTE MVLVNAIYFKGMWEKAFKDEDTQAVPFRMTEQESKPVQVMHQIGSFKVAVLAS EKIKILELPYASGELSMLVLLPDDVSGLEQLETAITFEKLMEWTSPNIMEERKIKVF LPRMKIEEKYNLTSVLMALGITDLFSPLANLSGISSAESLKMSEAIHEAFVEISEAG SEVIGSTEAEVEVTNDPEEFRADHPFLFLIKHNPTNSILFFGRCFSP |
| PREDICTED: Ovalbumin-like [*Merops nubicus*] | 38 | MGSIGAASTEFCFDVFKELKAQYVNENIFYSPMTIITALSMVYLGSKENTRAQIAK VAHFDKITGFGESIESQCGASASIQFSLKDLFTQITKPSGNHSLSVASRIYAEETYPI LPEYLECMKELYKGGLETINFQTAANQARELINSWVERQTSGMIKNILQPSSVDS QTEMVLVNAIYFRGLWEKAFKVEDTQATPFRITEQESKPVQMMHQIGSFKVAVV ASEKIKILELPYASGRLTMLVVLPDDVSGLKQLETTITFEKLMEWTTSNIMEERKI KVYLPRMKIEEKYNLTSVLMALGLTDLFSSSANLSGISSAESLKMSEAVHEAFVEI YEAGSEVVASAEAGMDATSVSEEFRADHPFLFLIKDNTSNSILFFGRCFSP |
| PREDICTED: Ovalbumin-like [*Tauraco erythrolophus*] | 39 | MGSIGAASTEFCFDVFKELKGQHVNENIFFCPLSIVSALSMVYLGARENTRAQIVK VAHFDKIAGFAESIESQCGTSVSIHTSLKDMFTQITKPSDNYSLNFASRLYAEETYP IIPEYLQCVKELYKGGLETISFQTAADQAREIINSWVESQTNGMIKNILRPSSVHPQ TELVLVNAVYFKGTWEKAFKDEDTQAVPFRITEQESKPVQMMYQIGSFKVAAVT SEKMKILEVPYASGELSMLVLLPDDVSGLEQLETAITAEKLIEWTSSTVMEERKLK VYLPRMKIEEKYNLTTVLTALGVTDLFSSSANLSGISSAQGLKMSNAVHEAFVEIY EAGSEVVGSKGEGTEVSSVSDEFKADHPFLFLIKHNPTNSIVFFGRCFSP |
| PREDICTED: Ovalbumin -like [*Cuculus canorus*] | 40 | MGSIGAASTEFCFDVFKELKVHHVNENILYSPLAIISALSMVYLGAKENTRDQIDK VVHFDKITGIGESIESQCSTAVSVHTSLKDVFDQITRPSDNYSLAFASRLYAEKTYP ILPEYLQCVKELYKGGLETIDFQTAADQARQLINSWVEDETNGMIKNILRPSSVNP QTKIILVNAIYFKGMWEKAFKDEDTQEVPFRITEQETKSVQMMYQIGSFKVAEVV SDKMKILELPYASGKLSMLVLLPDDVYGLEQLETVITVEKLKEWTSSIVMEERITK VYLPRMKIMEKYNLTSVLTAFGITDLFSPSANLSGISSTESLKVSEAVHEAFVEIHE AGSEVVGSAGAGIEATSVSEEFKADHPFLFLIKHNPTNSILFFGRCFSP |
| Ovalbumin [*Antrostomus carolinensis*] | 41 | MGSIGAASTEFCLDVFKELKVQHVNENIFYSPLSIISALSMVYLGARENTRAQIDK VVHFDKITGFEDSIESQCGTSVSVHTSLKDMFTQITKPSDNYSVGFASRLYAAETY QILPEYSQCVKELYKGGLETINFQKAADQATELINSWVESQTNGMIKNILQPSSVD PQTQIFLVNAIYFKGMWQRAFKEEDTQAVPFRISEKESKPVQMMYQIGSFKVAVI PSEKIKILELPYASGLLSMLVILPDDVSGLEQLENAITLEKLMQWTSSNMMEERKI KVYLPRMRMEEKYNLTSVFMALGITDLFSSSANLSGISSAESLKMSDAVHEASVEI HEAGSEVVGSTGSGTEASSVSEEFRADHPYLFLIKHNPTDSIVFFGRCFSP |

(continued)

| Name | SEQ ID | Sequence |
|------|--------|----------|
| PREDICTED: Ovalbumin-like [*Opisthocomus hoazin*] | 42 | MGSIGAASTEFCFDVFKELKFQHVDENIFYSPLTIISALSMVYLGARENTRAQIDK VVHFDKIAGFEETVESQCGTSVSVHTSLKDMFAQITKPSDNYSLSFASRLYAEETY PILPEYLQCVKELYKGGLETISFQTAADQARDLINSWVESQTNGMIKNILQPSSVG PQTELILVNAIYFKGMWQKAFKDEDTQEVPFRMTEQQSKPVQMMYQTGSFKVA VVASEKMKILALPYASGQLSLLVMLPDDVSGLKQLESAITSEKLIEWTSPSMMEE RKIKVYLPRMKIEEKYNLTSVLMALGITDLFSPSANLSGISSAESLKMSQAVHEAF VEIYEAGSEVVGSTGAGMEDSSDSEEFRVDHPFLFFIKHNPTNSILFFGRCFSP |
| PREDICTED: Ovalbumin-like [*Lepidothrix coronata*] | 43 | MGSIGPLSVEFCCDVFKELRIQHPRENIFYSPVTIISALSMVYLGARDNTKAQIEKA VHFDKIPGFGESIESQCGTSLSIHTSLKDIFTQITKPSDNYTVGIASRLYAEEKYPILP EYLQCIKELYKGGLEPINFQTAAEQARELINSWVESQTNGMIKNILQPSSVNPETD MVLVNAIYFKGLWEKAFKDEDIQTVPFRITEQESKPVQMMFQIGSFRVAEITSEKI RILELPYASGQLSLWVLLPDDISGLEQLETAITFENLKEWTSSTKMEERKIKVYLPR MKIEEKYNLTSVLTSLGITDLFSSSANLSGISSAESLKVSSAFHEASVEIYEAGSKV VGSTGAEVEDTSVSEEFRADHPFLFLIKHNPSNSIFFFGRCFSP |
| PREDICTED: Ovalbumin [*Struthio camelus australis*] | 44 | MGSIGTASAEFCFDVFKELKVHHVNENIFYSPLSIISALSMVYLGARENTKTQMEK VIHFDKITGLGESMESQCGTGVSIHTALKDMLSEITKPSDNYSLSLASRLYAEQTY AILPEYLQCIKELYKESLETVSFQTAADQARELINSWIESQTNGVIKNFLQPGSVDS QTELVLVNAIYFKGMWEKAFKDEDTQEVPFRITEQESRPVQMMYQAGSFKVATV AAEKIKILELPYASGELSMLVLLPDDISGLEQLETTISFEKLTEWTSSNMMEDRNM KVYLPRMKIEEKYNLTSVLIALGMTDLFSPAANLSGISAAESLKMSEAIHAAYVEI YEADSEIVSSAGVQVEVTSDSEEFRVDHPFLFLIKHNPTNSVLFFGRCISP |
| PREDICTED: Ovalbumin-like [*Acanthisitta chloris*] | 45 | MGSIGAVSTEFSCDVFKELRIHHVQENIFYSPVTIISALSMIYLGARDSTKAQIEKA VHFDKIPGFGESIESQCGTSLSIHTSIKDMFTKITKASDNYSIGIASRLYAEEKYPILP EYLQCVKELYKGGLESISFQTAAEQAREIINSWVESQTNGMIKNILQPSSVDPQTDI VLVNAIYFKGLWEKAFRDEDTQTVPFKITEQESKPVQMMYQIGSFKVAEITSEKIK ILEVPYASGQLSLWVLLPDDISGLEKLETAITFENLKEWTSSTKMEERKIKVYLPR MKIEEKYNLTSVLTALGITDLFSSSANLSGISSAESLKVSEAFHEAIVEISEAGSKVV GSVGAGVDDTSVSEEFRADHPFLFLIKHNPTSSIFFFGRCFSP |
| PREDICTED: Ovalbumin-like [*Tyto alba*] | 46 | MGSIGAASTEFCFDVFKELKVQHVNENIFYSPLSIISALSMVYLGARENTRAQIDK VVHFDKIAGFGESTESQCGTSVSAHTSLKDMSNQITKLSDNYSLSFASRLYAEETY PILPEYSQCVKELYKGGLESISFQTAAYQARELINAWVESQTNGMIKDILQPGSVD SQTKMVLVNAIYFKGIWEKAFKDEDTQEVPFRMTEQETKPVQMMYQIGSFKVAV IAAEKIKILELPYASGQLSMLVILPDDVSGLEQLETAITFEKLTEWTSASVMEERKI KVYLPRMSIEEKYNLTSVLIALGVTDLFSSSANLSGISSAESLRMSEAIHEAFVETY EAGSTESGTEVTSASEEFRVDHPFLFLIKHKPTNSILFFGRCFSP |

(continued)

| Name | SEQ ID | Sequence |
|------|--------|----------|
| PREDICTED: Ovalbumin -like isoform X1 [*Phalacrocorax carbo*] | 47 | MGSIGAASSEFCFDIFKELKVQHVNENIFYSPLSIISALSMVYLGARENTRAQIDKV VPFDKITASGESIESQVQKIQCSTSVSVHTSLKDIFTQITKSSDNHSLSFASRLYAEE TYPILPEYLQCVKELYEGGLETISFQTAADQARELINSWIESQTNGRIKNILQPGSV DPQTEMVLVNAIYFKGMWEKAFKDEDTQAVPFRMTEQESKPVQVMHQIGSFKV AVLASEKIKILELPYASGELSMLVLLPDDVSGLEQLETAITFEKLMEWTSPNIMEE RKIKVFLPRMKIEEKYNLTSVLMALGITDLFSPLANLSGISSAESLKMSEAIHEAFV EISEAGSEVIGSTEAEVEVTNDPEEFRADHPFLFLIKHNPTNSILFFGRCFSP |
| Ovalbumin-like [*Pipra filicauda*] | 48 | MGSIGPLSVEFCCDVFKELRIQHARENIFYSPVTIISALSMVYLGARDNTKAQIEKA VHFDKIPGFGESIESQCGTSLSIHTSLKDIFTQITKPSDNYTVGIASRLYAEEKYPILP EYLQCIKELYKGGLEPISFQTAAEQARELINSWVESQTNGIIKNILQPSSVNPETDM VLVNAIYFKGLWEKAFKDEGTQTVPFRITEQESKPVQMMFQIGSFRVAEIASEKIR ILELPYASGQLSLWVLLPDDISGLEQLETAITFENLKEWTSSTKMEERKIKVYLPR MKIEEKYNLTSVLTSLGITDLFSSSANLSGISSAERLKVSSAFHEASMEINEAGSKV VGAGVDDTSVSEEFRVDRPFLFLIKHNPSNSIFFFGRCFSP |
| Ovalbumin [*Dromaius novaehollandiae*] | 49 | MGSIGAASTEFCFDMFKELKVHHVNENIIYSPLSIISILSMVFLGARENTKTQMEKV IHFDKITGFGESLESQCGTSVSVHASLKDILSEITKPSDNYSLSLASKLYAEETYPVL PEYLQCIKELYKGSLETVSFQTAADQARELINSWVETQTNGVIKNFLQPGSVDPQT EMVLVDAIYFKGTWEKAFKDEDTQEVPFRITEQESKPVQMMYQAGSFKVATVA AEKMKILELPYASGELSMFVLLPDDISGLEQLETTISIEKLSEWTSSNMMEDRKMK VYLPHMKIEEKYNLTSVLVALGMTDLFSPSANLSGISTAQTLKMSEAIHGAYVEIY EAGSEMATSTGVLVEAASVSEEFRVDHPFLFLIKHNPSNSILFFGRCIFP |
| Chain A, Ovalbumin | 50 | MGSIGAASTEFCFDMFKELKVHHVNENIIYSPLSIISILSMVFLGARENTKTQMEKV IHFDKITGFGESLESQCGTSVSVHASLKDILSEITKPSDNYSLSLASKLYAEETYPVL PEYLQCIKELYKGSLETVSFQTAADQARELINSWVETQTNGVIKNFLQPGSVDPQT EMVLVDAIYFKGTWEKAFKDEDTQEVPFRITEQESKPVQMMYQAGSFKVATVA AEKMKILELPYASGELSMFVLLPDDISGLEQLETTISIEKLSEWTSSNMMEDRKMK VYLPHMKIEEKYNLTSVLVALGMTDLFSPSANLSGISTAQTLKMSEAIHGAYVEIY EAGSEMATSTGVLVEAASVSEEFRVDHPFLFLIKHNPSNSILFFGRCIFPHHHHHH |
| Ovalbumin-like [*Corapipo altera*] | 51 | MGSIGPLSVEFCCDVFKELRIQHARENIFYSPVTIISALSMVYLGARDNTKAQIEKA VHFDKIPGFGESIESQCGTSLSIHTSLKDIFTQITKPSDNYTVGIASRLYAEEKYPILP EYLQCIKELYKGGLEPISFQTAAEQARELINSWVESQTNGMIKNILQPSAVNPETD MVLVNAIYFKGLWEKAFKDEGTQTVPFRITEQESKPVQMMFQIGSFRVAEITSEKI RILELPYASGQLSLWVLLPDDISGLEQLETAITFENLKEWTSSTKMEERKIKVYLPR MKIEEKYNLTSVLTSLGITDLFSSSANLSGISSAERLKVSSAFHEASMEIYEAGSKV VGSTGAGVDDTSVSEEFRVDRPFLFLIKHNPSNSIFFFGRCFSP |

(continued)

| Name | SEQ ID | Sequence |
|------|--------|----------|
| Ovalbumin-like protein [*Amazona aestiva*] | 52 | MEDQRGNTGFTMGSIGAASTEFCIDVFRELRVQHVNENIFYSPLTIISALSMVYLG ARENTRAQIDQVVHFDKIAGFGDTVESQCGSSPSVHNSLKTVXAQITQPRDNYSL NLASRLYAEESYPILPEYLQCVKELYNGGLETVSFQTAADQARELINSWVESQTN GIIKNILQPSSVDPQTEMVLVNAIYFKGLWEKAFKDEETQAVPFRITEQENRPVQM MYQFGSFKVAXVASEKIKILELPYASGQLSMLVLLPDEVSGLEQNAITFEKLTEW TSSDLMEERKIKVFFPRVKIEEKYNLTAVLVSLGITDLFSSSANLSGISSAENLKMS EAVHEAXVEIYEAGSEVAGSSGAGIEVASDSEEFRVDHPFLFLIXHNPTNSILFFGR CFSP |
| PREDICTED: Ovalbumin-like [*Melopsittacus undulatus*] | 53 | MGSIGAASTEFCIDVFRELRVQHVNENIFYSPLSIISALSMVYLGARENTRAQIDEV FHFDKIAGFGDTVDPQCGASLSVHKSLQNVFAQITQPKDNYSLNLASRLYAEESY PILPEYLQCVKELYNEGLETVSFQTGADQARELINSWVENQTNGVIKNILQPSSVD PQTEMVLVNAIYFKGLWQKAFKDEETQAVPFRITEQENRPVQMMYQFGSFKVAV VASEKVKILELPYASGQLSMWVLLPDEVSGLEQLENAITFEKLTEWTSSDLTEER KIKVFLPRVKIEEKYNLTAVLMALGVTDLFSSSANFSGISAAENLKMSEAVHEAF VEIYEAGSEVVGSSGAGIEAPSDSEEFRADHPFLFLIKHNPTNSILFFGRCFSP |
| Ovalbumin-like [*Neopelma chrysocephalum*] | 54 | MGSIGPLSVEFCCDVFKELRIQHARDNIFYSPVTIISALSMVYLGARDNTKAQIEKA VHFDKIPGFGESIESQCGTSLSVHTSLKDIFTQITKPRENYTVGIASRLYAEEKYPIL PEYLQCIKELYKGGLEPISFQTAAEQARELINSWVESQTNGMIKNILQPSSVNPETD MVLVNAIYFKGLWKKAFKDEGTQTVPFRITEQESKPVQMMFQIGSFRVAEITSEKI RILELPYASGQLSLWVLLPDDISGLEQLESAITFENLKEWTSSTKMEERKIKVYLPR MKIEEKYNLTSVLTSLGITDLFSSSANLSGISSAEKLKVSSAFHEASMEIYEAGNKV VGSTGAGVDDTSVSEEFRVDRPFLFLIKHNPSNSIFFFGRCFSP |
| PREDICTED: Ovalbumin-like [*Buceros rhinoceros silvestris*] | 55 | MGSIGAASAEFCVDVFKELKDQHVNNIVFSPLMIISALSMVNIGAREDTRAQIDKV VHFDKITGYGESIESQCGTSIGIYFSLKDAFTQITKPSDNYSLSFASKLYAEETYPIL PEYLKCVKELYKGGLETISFQTAADQARELINSWVESQTNGMIKNILQPSSVDPQT EMVLVNAIYFKGLWEKAFKDEDTQAVPFRITEQESKPVQMMYQIGSFKVAVIASE KIKILELPYASGQLSLLVLLPDDVSGLEQLESAITSEKLLEWTNPNIMEERKTKVYL PRMKIEEKYNLTSVLVALGITDLFSSSANLSGISSAEGLKLSDAVHEAFVEIYEAGR EVVGSSEAGVEDSSVSEEFKADRPFIFLIKHNPTNGILYFGRYISP |
| PREDICTED: Ovalbumin-like [*Cariama cristata*] | 56 | MGSIGAANTDFCFDVFKELKVHHANENIFYSPLSIVSALAMVYLGARENTRAQID KALHFDKILGFGETVESQCDTSVSVHTSLKDMLIQITKPSDNYSFSFASKIYTEETY PILPEYLQCVKELYKGGVETISFQTAADQAREVINSWVESHTNGMIKNILQPGSVD PQTKMVLVNAVYFKGIWEKAFKEEDTQEMPFRINEQESKPVQMMYQIGSFKLTV AASENLKILEFPYASGQLSMMVILPDEVSGLKQLETSITSEKLIKWTSSNTMEERKI RVYLPRMKIEEKYNLKSVLMALGITDLFSSSANLSGISSAESLKMSEAVHEAFVEI YEAGSEVTSSTGTEMEAENVSEEFKADHPFLFLIKHNPTDSIVFFGRCMSP |

(continued)

| Name | SEQ ID | Sequence |
|------|--------|----------|
| Ovalbumin [*Manacus vitellinus*] | 57 | MGSIGPLSVEFCCDVFKELRIQHARENIFYSPVTIISALSMVYLGARDNTKAQIEKA VHFDKIPGFGESIESQCGTSLSIHTSLKDIFTQITKPSDNYTVGIASRLYAEEKYPILP EYLQCIKELYKGGLEPISFQTAAEQARELINSWVESQTNGMIKNILQPSSVNPETD MVLVNAIYFKGLWEKAFKDESTQTVPFRITEQESKPVQMMFQIGSFRVAEIASEKI RILELPYASGQLSLWVLLPDDISGLEQLETAITFENLKEWTSSTKMEERKIKVYLPR MKIEEKYNLTSVLTSLGITDLFSSSANLSGISSAERLKVSSAFHEASMEIYEAGSRV VEAGVDDTSVSEEFRVDRPFLFLIKHNPSNSIFFFGRCFSP |
| Ovalbumin-like [*Empidonax traillii*] | 58 | MGSIGPVSTEFCCDIFKELRIQHARENIIYSPVTIISALSMVYLGARDNTKAQIEKAV HFDKIPGFGESIESQCGTSLSIHTSLKDILTQITKPSDNYTVGIASRLYAEEKYPILSE YLQCIKELYKGGLEPISFQTAAEQARELINSWVESQTNGMIKNILQPSSVNPETDM VLVNAIYFKGLWEKAFKDEGTQTVPFRITEQESKPVQMMFQIGSFKVAEITSEKIR ILELPYASGKLSLWVLLPDDISGLEQLETAITFENLKEWTSSTRMEERKIKVYLPR MKIEEKYNLTSVLTSLGITDLFSSSANLSGISSAERLKVSSAFHEVFVEIYEAGSKV EGSTGAGVDDTSVSEEFRADHPFLFLVKHNPSNSIIFFGRCYLP |
| PREDICTED: Ovalbumin-like [*Leptosomus discolor*] | 59 | MGSTGAASMEFCFALFRELKVQHVNENIFFSPVTIISALSMVYLGARENTRAQLD KVAPFDKITGFGETIGSQCSTSASSHTSLKDVFTQITKASDNYSLSFASRLYAEETY PILPEYLQCVKELYKGGLESISFQTAADQARELINSWVESQTNGMIKDILRPSSVDP QTKIILITAIYFKGMWEKAFKEEDTQAVPFRMTEQESKPVQMMYQIGSFKVAVIPS EKLKILELPYASGQLSMLVILPDDVSGLEQLETAITTEKLKEWTSPSMMKERKMK VYFPRMRIEEKYNLTSVLMALGITDLFSPSANLSGISSAESLKVSEAVHEASVDIDE AGSEVIGSTGVGTEVTSVSEEIRADHPFLFLIKHKPTNSILFFGRCFSP |

(continued)

| Name | SEQ ID | Sequence |
|---|---|---|
| Hypothetical protein H355_008077 [*Colinus virginianus*] | 60 | MEHAQLTQLVNSNMTSNTCHEADEFENIDFRMDSISVTNTKFCFDVFNEMKVHH VNENILYSPLSILTALAMVYLGARGNTESQMKKALHFDSITGAGSTTDSQCGSSE YIHNLFKEFLTEITRTNATYSLEIADKLYVDKTFTVLPEYINCARKFYTGGVEEVN FKTAAEEARQLINSWVEKETNGQIKDLLVPSSVDFGTMMVFINTIYFKGIWKTAF NTEDTREMPFSMTKQESKPVQMMCLNDTFNMATLPAEKMRILELPYASGELSML VLLPDEVSGLEQIEKAINFEKLREWTSTNAMEKKSMKVYLPRMKIEEKYNLTSTL MALGMTDLFSRSANLTGISSVENLMISDAVHGAFMEVNEEGTEAAGSTGAIGNIK HSVEFEEFRADHPFLFLIRYNPTNVILFFDNSEFTMGSIGAVSTEFCFDVFKELRVH HANENIFYSPFTVISALAMVYLGAKDSTRTQINKVVRFDKLPGFGDSIEAQCGTSA NVHSSLRDILNQITKPNDIYSFSLASRLYADETYTILPEYLQCVKELYRGGLESINF QTAADQARELINSWVESQTSGIIRNVLQPSSVDSQTAMVLVNAIYFKGLWEKGFK DEDTQAMPFRVTEQENKSVQMMYQIGTFKVASVASEKMKILELPFASGTMSMW VLLPDEVSGLEQLETTISIEKLTEWTSSSVMEERKIKVFLPRMKMEEKYNLTSVLM AMGMTDLFSSSANLSGISSTLQKKGFRSQELGDKYAKPMLESPALTPQVTAWDN SWIVAHPAAIEPDLCYQIMEQKWKPFDWPDFRLPMRVSCRFRTMEALNKANTSF ALDFFKHECQEDDDENILFSPFSISSALATVYLGAKGNTADQMAKTEIGKSGNIHA GFKALDLEINQPTKNYLLNSVNQLYGEKSLPFSKEYLQLAKKYYSAEPQSVDFLG KANEIRREINSRVEHQTEGKIKNLLPPGSIDSLTRLVLVNALYFKGNWATKFEAED TRHRPFRINMHTTKQVPMMYLRDKFNWTYVESVQTDVLELPYVNNDLSMFILLP |

(continued)

| Name | SEQ ID | Sequence |
|------|--------|----------|
| | | RDITGLQKLINELTFEKLSAWTSPELMEKMKMEVYLPRFTVEKKYDMKSTLSKM GIEDAFTKVDSCGVTNVDEITTHIVSSKCLELKHIQINKKLKCNKAVAMEQVSASI GNFTIDLFNKLNETSRDKNIFFSPWSVSSALALTSLAAKGNTAREMAEDPENEQA ENIHSGFKELMTALNKPRNTYSLKSANRIYVEKNYPLLPTYIQLSKKYYKAEPYK VNFKTAPEQSRKEINNWVEKQTERKIKNFLSSDDVKNSTKSILVNAIYFKAEWEE KFQAGNTDMQPFRMSKNKSKLVKMMYMRHTFPVLIMEKLNFKMIELPYVKREL SMFILLPDDIKDSTTGLEQLERELTYEKLSEWADSKKMSVTLVDLHLPKFSMEDR YDLKDALKSMGMASAFNSNADFSGMTGFQAVPMESLSASTNSFTLDLYKKLDET SKGQNIFFASWSIATALAMVHLGAKGDTATQVAKGPEYEETENIHSGFKELLSAI NKPRNTYLMKSANRLFGDKTYPLLPKFLELVARYYQAKPQAVNFKTDAEQARA QINSWVENETESKIQNLLPAGSIDSHTVLVLVNAIYFKGNWEKRFLEKDTSKMPF RLSKTETKPVQMMFLKDTFLIHHERTMKFKIIELPYVGNELSAFVLLPDDISDNTT GLELVERELTYEKLAEWSNSASMMKAKVELYLPKLKMEENYDLKSVLSDMGIRS AFDPAQADFTRMSEKKDLFISKVIHKAFVEVNEEDRIVQLASGRLTGRCRTLANK ELSEKNRTKNLFFSPFSISSALSMILLGSKGNTEAQIAKVLSLSKAEDAHNGYQSLL SEINNPDTKYILRTANRLYGEKTFEFLSSFIDSSQKFYHAGLEQTDFKNASEDSRKQ INGWVEEKTEGKIQKLLSEGIINSMTKLVLVNAIYFKGNWQEKFDKETTKEMPFKI NKNETKPVQMMFRKGKYNMTYIGDLETTVLEIPYVDNELSMIILLPDSIQDESTGL EKLERELTYEKLMDWINPNMMDSTEVRVSLPRFKLEENYELKPTLSTMGMPDAF DLRTADFSGISSGNELVLSEVVHKSFVEVNEEGTEAAAATAGIMLLRCAMIVANF TADHPFLFFIRHNKTNSILFCGRFCSP |
| PREDICTED: Ovalbumin isoform X2 [*Apteryx australis mantelli*] | 61 | MGSIGTASTEFCFDMFKEMKVQHANQNIIFSPLTIISALSMVYLGARDNTKAQME KVIHFDKITGFGESVESQCGTSVSIHTSLKDMLSEITKPSDNYSLSLASRLYAEETY PILPEYLQCMKELYKGGLETVSFQTAADQARELINSWVESQTNGVIKNFLQPGSV DPQTEMVLVNAIYFKGMWEKAFKDEDTQEVPFRITEQESKPVQMMYQVGSFKV ATVAAEKMKILEIPYTHRELSMFVLLPDDISGLEQLETTISFEKLTEWTSSNMMEE RKVKVYLPHMKIEEKYNLTSVLMALGMTDLFSPSANLSGISTAQTLMMSEAIHG AYVEIYEAGREMASSTGVQVEVTSVLEEVRADKPFLFFIRHNPTNSMVVFGRYMS P |

(continued)

| Name | SEQ ID | Sequence |
|---|---|---|
| Hypothetical protein ASZ78_006007 [*Callipepla squamata*] | 62 | MTSNTCHEADEFENIDFRMDSISVTNTKFCFDVFNEMKVHHVNENILYSPLSILTA LAMVYLGARGNTESQMKKALHFDSITGGGSTTDSQCGSSEYIHNLFKEFLTEITRT NATYSLEIADKLYVDKTFTVLPEYINCARKFYTGGVEEVNFKTAAEEARQLMNS WVEKETNGQIKDLLVPSSVDFGTMMVFINTIYFKGIWKTAFNTEDTREMPFSMTK QESKPVQMMCLNDTFNMVTLPAEKMRILELPYASGELSMLVLLPDEVSGLERIEK AINFEKLREWTSTNAMEKKSMKVYLPRMKIEEKYNLTSTLMALGMTDLFSRSAN LTGISSVDNLMISDAVHGAFMEVNEEGTEAAGSTGAIGNIKHSVEFEEFRADHPFL FLIRYNPTNVILFFDNSEFTMGSIGAVSTEFCFDVFKELRVHHANENIFYSPFTIISA LAMVYLGAKDSTRTQINKVVRFDKLPGFGDSIEAQCGTSANVHSSLRDILNQITKP NDIYSFSLASRLYADETYTILPEYLQCVKELYRGGLESINFQTAADQARELINSWV ESQTSGIIRNVLQPSSVDSQTAMVLVNAIYFKGLWEKGFKDEDTQAIPFRVTEQEN KSVQMMYQIGTFKVASVASEKMKILELPFASGTMSMWVLLPDEVSGLEQLETTIS IEKLTEWTSSSVMEERKIKVFLPRMKMEEKYNLTSVLMAMGMTDLFSSSANLSGI SSTLQKKGFRSQELGDKYAKPMLESPALTPQATAWDNSWIVAHPPAIEPDLYYQI MEQKWKPFDWPDFRLPMRVSCRFRTMEALNKANTSFALDFFKHECQEDDSENIL FSPFSISSALATVYLGAKGNTADQMAKVLHFNEAEGARNVTTTIRMQVYSRTDQ QRLNRRACFQKTEIGKSGNIHAGFKGLNLEINQPTKNYLLNSVNQLYGEKSLPFSK EYLQLAKKYYSAEPQSVDFVGTANEIRREINSRVEHQTEGKIKNLLPPGSIDSLTRL VLVNALYFKGNWATKFEAEDTRHRPFRINTHTTKQVPMMYLSDKFNWTYVESV QTDVLELPYVNNDLSMFILLPRDITGLQKLINELTFEKLSAWTSPELMEKMKMEV YLPRFTVEKKYDMKSTLSKMGIEDAFTKVDNCGVTNVDEITIHVVPSKCLELKHI QINKELKCNKAVAMEQVSASIGNFTIDLFNKLNETSRDKNIFFSPWSVSSALALTS LAAKGNTAREMAEDPENEQAENIHSGFNELLTALNKPRNTYSLKSANRIYVEKN YPLLPTYIQLSKKYYKAEPHKVNFKTAPEQSRKEINNWVEKQTERKIKNFLSSDD VKNSTKLILVNAIYFKAEWEEKFQAGNTDMQPFRMSKNKSKLVKMMYMRHTFP VLIMEKLNFKMIELPYVKRELSMFILLPDDIKDSTTGLEQLERELTYEKLSEWADS KKMSVTLVDLHLPKFSMEDRYDLKDALRSMGMASAFNSNADFSGMTGERDLVI SKVCHQSFVAVDEKGTEAAAATAVIAEAVPMESLSASTNSFTLDLYKKLDETSKG QNIFFASWSIATALTMVHLGAKGDTATQVAKGPEYEETENIHSGFKELLSALNKP RNTYSMKSANRLFGDKTYPLLPTKTKPVQMMFLKDTFLIHHERTMKFKIIELPYM GNELSAFVLLPDDISDNTTGLELVERELTYEKLAEWSNSASMMKVKVELYLPKL KMEENYDLKSALSDMGIRSAFDPAQADFTRMSEKKDLFISKVIHKAFVEVNEEDR IVQLASGRLTGNTEAQIAKVLSLSKAEDAHNGYQSLLSEINNPDTKYILRTANRLY GEKTFEFLSSFIDSSQKFYHAGLEQTDFKNASEDSRKQINGWVEEKTEGKIQKLLS EGIINSMTKLVLVNAIYFKGNWQEKFDKETTKEMPFKINKNETKPVQMMFRKGK YNMTYIGDLETTVLEIPYVDNELSMIILLPDSIQDESTGLEKLERELTYEKLMDWIN PNMMDSTEVRVSLPRFKLEENYELKPTLSTMGMPDAFDLRTADFSGISSSGNELVL SEVVHKSFVEVNEEGTEAAAATAGIMLLRCAMIVANFTADHPFLFFIRHNKTNSIL FCGRFCSP |

(continued)

| Name | SEQ ID | Sequence |
|---|---|---|
| PREDICTED: Ovalbumin-like [*Mesitornis unicolor*] | 63 | MASIGAASTEFCFDVFKELKTQHVKENIFYSPMAIISALSMVYIGARENTRAEIDK VVHFDKITGFGNAVESQCGPSVSVHSSLKDLITQISKRSDNYSLSYASRIYAEETYP ILPEYLQCVKEVYKGGLESISFQTAADQARENINAWVESQTNGMIKNILQPSSVNP QTEMVLVNAIYLKGMWEKAFKDEDTQTMPFRVTQQESKPVQMMYQIGSFKVAV IASEKMKILELPYTSGQLSMLVLLPDDVSGLEQVESAITAEKLMEWTSPSIMEERT MKVYLPRMKMVEKYNLTSVLMALGMTDLFTSVANLSGISSAQGLKMSQAIHEA FVEIYEAGSEAVGSTGVGMEITSVSEEFKADLSFLFLIRHNPTNSIIFFGRCISP |
| Ovalbumin, partial [*Anas platyrhynchos*] | 64 | MGSIGAASTEFCFDVFRELRVQHVNENIFYSPFSIISALAMVYLGARDNTRTQIDKI SQFQALSDEHLVLCIQQLGEFFVCTNRERREVTRYSEQTEDKTQDQNTGQIHKIV DTCMLRQDILTQITKPSDNFSLSFASRLYAEETYAILPEYLQCVKELYKGGLESISF QTAADQARELINSWVESQTNGIIKNILQPSSVDSQTTMVLVNAIYFKGMWEKAFK DEDTQAMPFRMTEQESKPVQMMYQVGSFKVAMVTSEKMKILELPFASGMMSMF VLLPDEVSGLEQLESTISFEKLTEWTSSTMMEERRMKVYLPRMKMEEKYNLTSVF MALGMTDLFSSSANMSGISSTVSLKMSEAVHAACVEIFEAGRDVVGSAEAGMDV TSVSEEFRADHPFLFFIKHNPTNSILFFGRWMSP |
| PREDICTED: Ovalbumin-like [*Chaetura pelagica*] | 65 | MGSIGAASAEFCLDIFKELKVQHVNENIIFSPMTIISALSLVYLGAKEDTRAQIEKV VPFDKIPGFGEIVESQCPKSASVHSSIQDIFNQIIKRSDNYSLSLASRLYAEESYPIRP EYLQCVKELDKEGLETISFQTAADQARQLINSWVESQTNGMIKNILQPSSVNSQTE MVLVNAIYFRGLWQKAFKDEDTQAVPFRITEQESKPVQMMQQIGSFKVAEIASE KMKILELPYASGQLSMLVLLPDDVSGLEKLESSITVEKLIEWTSSNLTEERNVKVY LPRLKIEEKYNLTSVLAALGITDLFSSSANLSGISTAESLKLSRAVHESFVEIQEAGH EVEGPKEAGIEVTSALDEFRVDRPFLFVTKHNPTNSILFLGRCLSP |
| PREDICTED: Ovalbumin-like [*Apaloderma vittatum*] | 66 | MGSISAASGEFCLDIFKELKVQHVNENIFYSPMVIVSALSLVYLGARENTRAQIDK VIPFDKITGSSEAVESQCGTPVGAHISLKDVFAQIAKRSDNYSLSFVNRLYAEETYP ILPEYLQCVKELYKGGLETISFQTAADQAREIINSWVESQTDGKIKNILQPSSVDPQ TKMVLVSAIYFKGLWEKSFKDEDTQAVPFRVTEQESKPVQMMYQIGSFKVAAIA AEKIKILELPYASEQLSMLVLLPDDVSGLEQLEKKISYEKLTEWTSSSVMEEKKIK VYLPRMKIEEKYNLTSILMSLGITDLFSSSANLSGISSTKSLKMSEAVHEASVEIYE AGSEASGITGDGMEATSVFGEFKVDHPFLFMIKHKPTNSILFFGRCISP |
| Ovalbumin-like [*Corvus cornix cornix*] | 67 | MGSIGPVSTEVCCDIFRELRSQSVQENVCYSPLLIISTLSMVYIGAKDNTKAQIEKA IHFDKIPGFGESTESQCGTSVSIHTSLKDIFTQITKPSDNYSISIARRLYAEEKYPILPE YIQCVKELYKGGLESISFQTAAEKSRELINSWVESQTNGTIKNILQPSSVSSQTDMV LVSAIYFKGLWEKAFKEEDTQTIPFRITEQESKPVQMMSQIGTFKVAEIPSEKCRIL ELPYASGRLSLWVLLPDDISGLEQLETAITFENLKEWTSSSKMEERKIRVYLPRMK IEEKYNLTSVLKSLGITDLFSSSANLSGISSAESLKVSAAFHEASVEIYEAGSKGVG SSEAGVDGTSVSEEIRADHPFLFLIKHNPSDSILFFGRCFSP |

(continued)

| Name | SEQ ID | Sequence |
|------|--------|----------|
| PREDICTED: Ovalbumin-like [*Calypte anna*] | 68 | MGSIGAASTEFCFDVFKELKVQHVNENIIISPLSIISALSMVYLGAREDTRAQIDKV VHFDKITGFGEAIESQCPTSESVHASLKETFSQLTKPSDNYSLAFASRLYAEETYPI LPEYLQCVKELYKGGLETINFQTAAEQARQVINSWVESQTDGMIKSLLQPSSVDP QTEMILVNAIYFRGLWERAFKDEDTQELPFRITEQESKPVQMMSQIGSFKVAVVA SEKVKILELPYASGQLSMLVLLPDDVSGLEQLESSITVEKLIEWISSNTKEERNIKV YLPRMKIEEKYNLTSVLVALGITDLFSSSANLSGISSAESLKISEAVHEAFVEIQEA GSEVVGSPGPEVEVTSVSEEWKADRPFLFLIKHNPTNSILFFGRYISP |
| PREDICTED: Ovalbumin [*Corvus brachyrhynchos*] | 69 | MGSIGPVSTEVCCDIFRELRSQSVQENVCYSPLLIISTLSMVYIGAKDNTKAQIEKA IHFDKIPGFGESTESQCGTSVSIHTSLKDIFTQITKPSDNYSISIARRLYAEEKYPILQ EYIQCVKELYKGGLESISFQTAAEKSRELINSWVESQTNGTIKNILQPSSVSSQTDM VLVSAIYFKGLWEKAFKEEDTQTIPFRITEQESKPVQMMSQIGTFKVAEIPSEKCRI LELPYASGRLSLWVLLPDDISGLEQLETSITFENLKEWTSSSKMEERKIRVYLPRM KIEEKYNLTSVLKSLGITDLFSSSANLSGISSAESLKVSAVFHEASVEIYEAGSKGV GSSEAGVDGTSVSEEIRADHPFLFLIKHNPSDSILFFGRCFSP |
| Hypothetical protein DUI87_08270 [*Hirundo rustica rustica*] | 70 | MLNLMHPKQFCCTMGSIGPVSTEVCCDIFRELRSQSVQENVCYSPLLIISTLSMVYI GAKDNTKAQIEKAIHFDKIPGFGESTESQCGTSVSIHTSLKDIFTQITKPSDNYSISIA SRLYAEEKYPILPEYIQCVKELYKGGLESISFQTAAEKSRELINSWVESQTNGTIKN ILQPSSVSSQTDMVLVSAIYFKGLWEKAFKEEDTQTVPFRITEQESKPVQMMSQIG TFKVAEIPSEKCRILELPYASGRLSLWVLLPDDISGLEQLETAITSENLKEWTSSSK MEERKIKVYLPRMKIEEKYNLTSVLKSLGITDLFSSSANLSGISSAESLKVSGAFHE AFVEIYEAGSKAVGSSGAGVEDTSVSEEIRADHPFLFFIKHNPSDSILFFGRCFSP |
| Ostrich OVA sequence as secreted from pichia | 71 | EAEAGSIGTASAEFCFDVFKELKVHHVNENIFYSPLSIISALSMVYLGARENTKTQ MEKVIHFDKITGLGESMESQCGTGVSIHTALKDMLSEITKPSDNYSLSLASRLYAE QTYAILPEYLQCIKELYKESLETVSFQTAADQARELINSWIESQTNGVIKNFLQPGS VDSQTELVLVNAIYFKGMWEKAFKDEDTQEVPFRITEQESRPVQMMYQAGSFKV ATVAAEKIKILELPYASGELSMLVLLPDDISGLEQLETTISFEKLTEWTSSNMMED RNMKVYLPRMKIEEKYNLTSVLIALGMTDLFSPAANLSGISAAESLKMSEAIHAA YVEIYEADSEIVSSAGVQVEVTSDSEEFRVDHPFLFLIKHNPTNSVLFFGRCISP |

(continued)

| Name | SEQ ID | Sequence |
|---|---|---|
| Ostrich construct (secretion signal + mature protein) | 72 | MRFPSIFTAVLFAASSALAAPVNTTTEDETAQIPAEAVIGYSDLEGDFDVAVLPFS NSTNNGLLFINTTIASIAAKEEGVSLEKREAEAGSIGTASAEFCFDVFKELKVHHV NENIFYSPLSIISALSMVYLGARENTKTQMEKVIHFDKITGLGESMESQCGTGVSIH TALKDMLSEITKPSDNYSLSLASRLYAEQTYAILPEYLQCIKELYKESLETVSFQTA ADQARELINSWIESQTNGVIKNFLQPGSVDSQTELVLVNAIYFKGMWEKAFKDED TQEVPFRITEQESRPVQMMYQAGSFKVATVAAEKIKILELPYASGELSMLVLLPD DISGLEQLETTISFEKLTEWTSSNMMEDRNMKVYLPRMKIEEKYNLTSVLIALGM TDLFSPAANLSGISAAESLKMSEAIHAAYVEIYEADSEIVSSAGVQVEVTSDSEEFR VDHPFLFLIKHNPTNSVLFFGRCISP |
| Duck OVA sequence as secreted from pichia | 73 | EAEAGSIGAASTEFCFDVFRELRVQHVNENIFYSPFSIISALAMVYLGARDNTRTQI DKVVHFDKLPGFGESMEAQCGTSVSVHSSLRDILTQITKPSDNFSLSFASRLYAEE TYAILPEYLQCVKELYKGGLESISFQTAADQARELINSWVESQTNGIIKNILQPSSV DSQTTMVLVNAIYFKGMWEKAFKDEDTQAMPFRMTEQESKPVQMMYQVGSFK VAMVTSEKMKILELPFASGMMSMFVLLPDEVSGLEQLESTISFEKLTEWTSSTMM EERRMKVYLPRMKMEEKYNLTSVFMALGMTDLFSSSANMSGISSTVSLKMSEAV HAACVEIFEAGRDVVGSAEAGMDVTSVSEEFRADHPFLFFIKHNPTNSILFFGRW MSP |
| Duck construct (secretion signal + mature protein) | 74 | MRFPSIFTAVLFAASSALAAPVNTTTEDETAQIPAEAVIGYSDLEGDFDVAVLPFS NSTNNGLLFINTTIASIAAKEEGVSLEKREAEAGSIGAASTEFCFDVFRELRVQHVN ENIFYSPFSIISALAMVYLGARDNTRTQIDKVVHFDKLPGFGESMEAQCGTSVSVH SSLRDILTQITKPSDNFSLSFASRLYAEETYAILPEYLQCVKELYKGGLESISFQTAA DQARELINSWVESQTNGIIKNILQPSSVDSQTTMVLVNAIYFKGMWEKAFKDEDT QAMPFRMTEQESKPVQMMYQVGSFKVAMVTSEKMKILELPFASGMMSMFVLLP DEVSGLEQLESTISFEKLTEWTSSTMMEERRMKVYLPRMKMEEKYNLTSVFMAL GMTDLFSSSANMSGISSTVSLKMSEAVHAACVEIFEAGRDVVGSAEAGMDVTSV SEEFRADHPFLFFIKHNPTNSILFFGRWMSP |

Expression of rOVA in a host cell, for instance a Pichia species, a Saccharomyces species, a Trichoderma species, a Pseudomonas species may lead to an addition of one or more amino acids to the OVA sequence as part of post-transcriptional or post-translational modifications. Such amino acids may not be part of the native OVA sequences. For instance, expressing an OVA sequence in a Pichia species, such as *Komagataella phaffii* and *Komagataella pastoris* may lead to addition of one or more amino acids at the N-terminus or C-terminus. In some cases, four amino acids EAEA (SEQ ID NO: 75) is added to the N-terminus of the OVA sequence upon expression in a host cell as shown in SEQ ID NO: 1. For example, chicken rOVA may be provided encoding SEQ ID NO: 1, and following expression and secretion, rOVA has the amino acid sequence of SEQ ID NO:2.

An rOVA can be a non-naturally occurring variant of an OVA. Such variant can comprise one or more amino acid insertions, deletions, or substitutions relative to a native OVA sequence.

Such a variant can have at least 70%, 75%, 80%, 85%, 90%, 95%, 96%, 97%, 98%, or 99% sequence identity to SEQ ID NOs: 1-74. The term "sequence identity" as used herein in the context of amino acid sequences is defined as the percentage of amino acid residues in a candidate sequence that are identical with the amino acid residues in a selected sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software, with BLAST being the preferable alignment algorithm. Those skilled in the art can determine appropriate parameters for measuring alignment, including any algorithms needed to achieve maximal alignment over the full-length of the sequences being compared.

Depending on the host organism used to express the rOVA, the rOVA can have a glycosylation, acetylation, or phosphorylation pattern different from wildtype OVA. For example, the rOVA herein may or may not be glycosylated, acetylated, or phosphorylated. An rOVA may have an avian, non-avian, microbial, non-microbial, mammalian, or non-mammalian glycosylation, acetylation, or phosphorylation pattern.

In some cases, rOVA may be deglycosylated (e.g., chemically, enzymatically, Endo-H, PNGase F, O-Glycosidase, Neuraminidase, β1-4 Galactosidase, β-N-acetylglucosaminidase), deacetylated (e.g., protein deacetylase, histone deacetylase, sirtuin), or dephosphorylated (e.g., acid phosphatase, lambda protein phosphatase, calf intestinal phosphatase, alkaline phosphatase). Deglycosylation, deacetylation or dephosphorylation may produce a protein that is more uniform or is capable of producing a composition with less variation.

An rOVA is recombinantly expressed in a host cell. As used herein, a "host" or "host cell" denotes here any protein production host selected or genetically modified to produce a desired product. Exemplary hosts include fungi, such as filamentous fungi, as well as bacteria, yeast, plant, insect, and mammalian cells. A host cell may be Arxula spp., *Arxula adeninivorans*, Kluyveromyces spp., *Kluyveromyces lactis*, *Komagataella phaffii*, Pichia spp., *Pichia angusta*, *Pichia pastoris*, Saccharomyces spp., *Saccharomyces cerevisiae*, Schizosaccharomyces spp., *Schizosaccharomyces pombe*, Yarrowia spp., *Yarrowia lipolytica*, Agaricus spp., *Agaricus bisporus*, Aspergillus spp., *Aspergillus awamori*, *Aspergillus fumigatus*, *Aspergillus nidulans*, *Aspergillus niger*, *Aspergillus oryzae*, *Bacillus subtilis*, Colletotrichum spp., *Colletotrichum gloeosporioides*, Endothia spp., *Endothia parasitica*, *Escherichia coli*, Fusarium spp., *Fusarium graminearum*, *Fusarium solani*, Mucor spp., *Mucor miehei*, *Mucor pusillus*, Myceliophthora spp., *Myceliophthora thermophila*, Neurospora spp., *Neurospora crassa*, Penicillium spp., *Penicillium camemberti*, *Penicillium canescens*, *Penicillium chrysogenum*, *Penicillium (Talaromyces) emersonii*, *Penicillium funiculo sum*, *Penicillium purpurogenum*, *Penicillium roqueforti*, Pleurotus spp., *Pleurotus ostreatus*, Rhizomucor spp., *Rhizomucor miehei*, *Rhizomucor pusillus*, Rhizopus spp., *Rhizopus arrhizus*, *Rhizopus oligosporus*, *Rhizopus oryzae*, Trichoderma spp., *Trichoderma altroviride*, *Trichoderma reesei*, or *Trichoderma vireus*. A host cell can be an organism that is approved as generally regarded as safe by the U.S. Food and Drug Administration.

An rOVA protein can be recombinantly expressed in yeast, filamentous fungi or a bacterium. In some embodiments, rOVA protein is recombinantly expressed in a Pichia species (*Komagataella phaffii* and *Komagataella pastoris*), a Saccharomyces species, a Trichoderma species, a Pseudomonas species or an *E.coli* species.

Expression of an rOVA can be provided by an expression vector, a plasmid, a nucleic acid integrated into the host genome or other means. For example, a vector for expression can include: (a) a promoter element, (b) a signal peptide, (c) an OVA sequence heterologous to the host cell, and (d) a terminator element.

Expression vectors that can be used for expression of OVA include those containing an expression cassette with elements (a), (b), (c) and (d). In some embodiments, the signal peptide (b) need not be included in the vector. In general, the expression cassette is designed to mediate the transcription of the transgene when integrated into the genome of a cognate host microorganism.

To aide in the amplification of the vector prior to transformation into the host microorganism, a replication origin (e) may be contained in the vector (such as PUC_ORIC and PUC (DNA2.0)). To aide in the selection of microorganism stably transformed with the expression vector, the vector may also include a selection marker (f) such as URA3 gene and Zeocin resistance gene (ZeoR). The expression vector may also contain a restriction enzyme site (g) that allows for linearization of the expression vector prior to transformation into the host microorganism to facilitate the expression vectors stable integration into the host genome. In some embodiments the expression vector may contain any subset of the elements (b), (e), (f), and (g), including none of elements (b), (e), (f), and (g). Other expression elements and vector element known to one of skill in the art can be used in combination or substituted for the elements described herein.

Exemplary promoter elements (a) may include, but are not limited to, a constitutive promoter, inducible promoter, and hybrid promoter. Promoters include, but are not limited to, acu-5, adh1+, alcohol dehydrogenase (ADH1, ADH2, ADH4), AHSB4m, AINV, alcA, α-amylase, alternative oxidase (AOD), alcohol oxidase I (AOX1), alcohol oxidase 2 (AOX2), AXDH, B2, CaMV, cellobiohydrolase I (cbh1), ccg-1, cDNA1, cellular filament polypeptide (cfp), cpc-2, ctr4+, CUP1, dihydroxyacetone synthase (DAS), enolase (ENO, ENO1), formaldehyde dehydrogenase (FLD1), FMD, formate dehydrogenase (FMDH), G1, G6, GAA, GAL1, GAL2, GAL3, GAL4, GAL5, GAL6, GAL7, GAL8, GAL9, GAL10, GCW14, gdhA, gla-1, α-glucoamylase (glaA), glyceraldehyde-3-phosphate dehydrogenase (gpdA, GAP, GAPDH), phosphoglycerate mutase (GPM1), glycerol kinase (GUT1), HSP82, invl+, isocitrate lyase (ICL1), acetohydroxy acid isomeroreductase (ILV5), KAR2, KEX2, β-galactosidase (lac4), LEU2, melO, MET3, methanol oxidase (MOX), nmt1, NSP, pcbC, PET9, peroxin 8 (PEX8), phosphoglycerate kinase (PGK, PGK1), pho1, PHOS, PHO89, phosphatidylinositol synthase (PIS1), PYK1, pyruvate kinase (pki1), RPS7, sorbitol dehydrogenase (SDH), 3-phosphoserine aminotransferase (SER1), SSA4, SV40, TEF, translation elongation factor 1 alpha (TEF1), THI11, homoserine kinase (THR1), tpi, TPS1, triose phosphate isomerase (TPI1), XRP2, YPT1, and any combination thereof.

A signal peptide (b), also known as a signal sequence, targeting signal, localization signal, localization sequence, signal peptide, transit peptide, leader sequence, or leader peptide, may support secretion of a protein or polynucleotide. Extracellular secretion of a recombinant or heterologously expressed protein from a host cell may facilitate protein purification. A signal peptide may be derived from a precursor (e.g., prepropeptide, preprotein) of a protein. Signal peptides can be derived from a precursor of a protein other than the signal peptides in native OVA. An example of secretion protein is a *S. cerevisiae* alpha factor pre pro sequence shown bolded and underlined in SEQ ID NO: 1.

Any nucleic acid sequence that encodes OVA can be used as (c). Preferably such sequence is codon optimized for the host cell.

Exemplary transcriptional terminator elements include, but are not limited to, acu-5, adh1+, alcohol dehydrogenase (ADH1, ADH2, ADH4), AHSB4m, AINV, alcA, $\alpha$-amylase, alternative oxidase (AOD), alcohol oxidase I (AOX1), alcohol oxidase 2 (AOX2), AXDH, B2, CaMV, cellobiohydrolase I (cbh1), ccg-1, cDNA1, cellular filament polypeptide (cfp), cpc-2, ctr4+, CUP1, dihydroxyacetone synthase (DAS), enolase (ENO, ENO1), formaldehyde dehydrogenase (FLD1), FMD, formate dehydrogenase (FMDH), G1, G6, GAA, GAL1, GAL2, GAL3, GAL4, GAL5, GAL6, GAL7, GAL8, GAL9, GAL10, GCW14, gdhA, gla-1, $\alpha$-glucoamylase (glaA), glyceraldehyde-3-phosphate dehydrogenase (gpdA, GAP, GAPDH), phosphoglycerate mutase (GPM1), glycerol kinase (GUT1), HSP82, invl+, isocitrate lyase (ICL1), acetohydroxy acid isomeroreductase (ILV5), KAR2, KEX2, $\beta$-galactosidase (lac4), LEU2, melO, MET3, methanol oxidase (MOX), nmt1, NSP, pcbC, PET9, peroxin 8 (PEX8), phosphoglycerate kinase (PGK, PGK1), pho1, PHOS, PHO89, phosphatidylinositol synthase (PIS1), PYK1, pyruvate kinase (pki1), RPS7, sorbitol dehydrogenase (SDH), 3-phosphoserine aminotransferase (SER1), SSA4, SV40, TEF, translation elongation factor 1 alpha (TEF1), THI11, homoserine kinase (THR1), tpi, TPS1, triose phosphate isomerase (TPI1), XRP2, YPT1, and any combination thereof.

Exemplary selectable markers (f) may include, but are not limited to: an antibiotic resistance gene (e.g. zeocin, ampicillin, blasticidin, kanamycin, nourseothricin, chloroamphenicol, tetracycline, triclosan, ganciclovir, and any combination thereof), an auxotrophic marker (e.g. ade1, arg4, his4, ura3, met2, and any combination thereof).

In one example, a vector for expression in Pichia sp. can include an AOX1 promoter operably linked to a signal peptide (alpha mating factor) that is fused in frame with a nucleic acid sequence encoding OVA, and a terminator element (AOX1 terminator) immediately downstream of the nucleic acid sequence encoding OVA.

In another example, a vector comprising a DAS1 promoter is operably linked to a signal peptide (alpha mating factor) that is fused in frame with a nucleic acid sequence encoding OVA and a terminator element (AOX1 terminator) immediately downstream of OVA.

A recombinant protein described herein may be secreted from the one or more host cells. In some embodiments, rOVA protein is secreted from the host cell. The secreted rOVA may be isolated and purified by methods such as centrifugation, fractionation, filtration, ion exchange chromatography, affinity purification and other methods for separating protein from cells, liquid and solid media components and other cellular products and byproducts. In some embodiments, rOVA is produced in a Pichia Sp. and secreted from the host cells into the culture media. The secreted rOVA is then separated from other media components for further use.

The present disclosure contemplates modifying glycosylation of the recombinant OVA to alter or enhance one or more functional characteristics of the protein and/or its production. In some embodiments, the change in rOVA glycosylation can be due to the host cell glycosylating the rOVA. In some embodiments, rOVA has a glycosylation pattern that is not identical to a native ovalbumin (nOVA), such as a nOVA from chicken egg. In some embodiments, rOVA is treated with a deglycosylating enzyme before it is used as an ingredient in an rOVA composition, or when rOVA is present in a composition. In some embodiments, the glycosylation of rOVA is modified or removed by expressing one or more enzymes in a host cell and exposing rOVA to the one or more enzymes. In some embodiments, rOVA and the one or more enzymes for modification or removal of glycosylation are co-expressed in the same host cell.

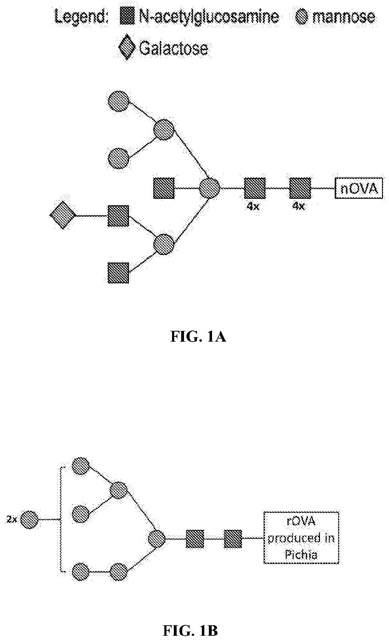

Native ovalbumin (nOVA), such as isolated from a chicken or another avian egg, has a highly complex branched form of glycosylation. The glycosylation pattern comprises N-linked glycan structures such as N-acetylglucosamine units, galactose and N-linked mannose units. See, e.g., **FIG. 1A**. In some cases, the rOVA for use in a herein disclosed consumable composition and produced using the methods described herein has a glycosylation pattern which is different from the glycosylation pattern of nOVA. For example, when rOVA is produced in a Pichia sp., the protein may be glycosylated differently from the nOVA and lack galactose units in the N-linked glycosylation. **FIG. 1B** illustrates the glycosylation patterns of rOVA produced by P. pastoris, showing a complex branched glycosylation pattern. In some embodiments of the compositions and methods disclosed herein, rOVA is treated such that the glycosylation pattern is modified from that of nOVA and also modified as compared to rOVA produced by a Pichia sp. without such treatment. In some cases, the rOVA lacks glycosylation.

The molecular weight or rOVA may be different as compared to nOVA. The molecular weight of the protein may be less than the molecular weight of nOVA or less than rOVA produced by the host cell where the glycosylation of rOVA is not modified. In embodiments, the molecular weight of an rOVA may be between 40kDa and 55kDa. In some cases, an rOVA with modified glycosylation has a different molecular weight, such as compared to a native OVA (as produced by an avian host species) or as compared to a host cell that glycosylates the rOVA, such as where the rOVA includes N-linked mannosylation. In some cases, the molecular weight of rOVA is greater than the molecular weight of the rOVA that is completely devoid of post-translational modifications. or an rOVA that lacks all forms of N-linked glycosylation.

## DEFINITIONS

The terminology used herein is for the purpose of describing particular cases only and is not intended to be limiting.

As used herein, the singular forms "a", "an" and "the" are intended to include the plural forms as well, unless the context clearly indicates otherwise.

The terms "including", "includes", "having", "has", "with", or variants thereof are used in either the detailed description and/or the claims, such terms are intended to be inclusive in a manner similar to the term "comprising".

Ranges can be expressed herein as from "about" or "approximately" one particular value, and/or to "about" or "approximately" another particular value. When such a range is expressed, another case includes from the one particular value and/or to the other particular value. Similarly, when values are expressed as approximations, by use of the antecedent "about" or "approximately", it will be understood that the particular value forms another case. It will be further understood that the endpoints of each of the ranges are significant both in relation to the other endpoint, and independently of the other endpoint. The term "about" or "approximately" as used herein refers to a range that is 15% plus or minus from a stated numerical value within the context of the particular usage. For example, about 10 would include a range from 8.5 to 11.5. The term "about" or "approximately" also accounts for typical error or imprecision in measurement of values.

Any aspect or embodiment described herein can be combined with any other aspect or embodiment as disclosed herein.

## EXAMPLES

### Example 1: Preparation of recombinant ovalbumin

A *Gallus gallus* OVA coding sequence was fused in-frame with the alpha mating factor signal sequence downstream of the promoter sequence (SEQ ID NO:1). A promoter was placed upstream of the signal sequence OVA coding sequence and a transcriptional terminator was placed downstream of the OVA sequence. The expression construct was placed into a Kpas-URA 3 vector.

The expression constructs were transformed into *Pichia pastoris.* Successful integration was confirmed by genomic sequencing.

*Fermentation:* Recombinant OVA was produced in a bioreactor at ambient conditions. A seed train for the fermentation process begins with the inoculation of shake flasks with liquid growth broth using 2ml cryovials of *Pichiapastoris* which are stored at -80°C and thawed at room temperature prior to inoculation.

The inoculated shake flasks were kept in a shaker at 30°C for 24 hours, after which the grown *Pichiapastoris* was transferred to a production scale reactor.

The culture was grown at 30°C, at a set pH and dissolved oxygen (DO). The culture was fed with a carbon source. At the end of the fermentation, the target OVA protein was harvested from the supernatant.

Cell debris was removed, protein was purified and lyophilized to a dry powder. The OVA produced was used in the examples described below.

### Example 2: Preparation of an eggless cake using recombinant ovalbumin

An eggless pound cake can be prepared with the following ingredients. A first ingredient composition made by mixing 2% to 5% recombinant ovalbumin and 0.05% to 0.5% sunflower lecithin. To prepare the pound cake, up to 4% of the dry first ingredient composition is added to 22 - 26% of unsalted butter, 20 - 25% of all-purpose flour, 18 - 26% of water, 20 - 25% sugar, 4 - 6% of sour cream, 1.2% of baking powder, 0.4% of vanilla flavor, 0.05 to 1.5% gums and starch and 0.18% of salt and all ingredients are then mixed to create a batter. For this recipe recombinant ovalbumin may be used at 2-5% and sunflower lecithin from 0.05 to 0.5%.

In one example, pound cakes with rOVA and with whole egg (as a comparison) were made as follows:

**Table 2: Cake with rOVA+ Xanthan gum**

| Ingredients | % w/w |
|---|---|
| Lecithin | 0.09 |
| All-purpose Flour | 22.61 |
| Granulated Sugar | 22.61 |
| Unsalted butter | 25.63 |
| Sour cream | 5.03 |
| Coarse salt | 0.18 |
| Baking powder | 1.21 |
| vanilla extract | 0.37 |
| rOVA | 3.41 |
| Water | 18.74 |
| Xanthan gum | 0.05 |
| Marigold yellow | 0.06 |
| Total | 100.00 |

**Table 3: Control Pound Cake with whole Egg**

| Ingredients | % w/w |
|---|---|
| Flour | 23.34 |
| Sugar | 23.34 |
| Whole egg | 23.34 |
| unsalted butter | 23.34 |
| Sour cream | 5.19 |
| baking powder | 1.25 |
| Vanilla | 0.38 |
| coarse salt | 0.21 |
| Total | 100.00 |

**Table 4: Cake with rOVA+ Potato Starch+ Xanthan gum**

| Ingredients | % w/w |
|---|---|
| Flour | 20.73 |
| Sugar | 20.73 |
| Unsalted butter | 22.08 |
| Sour cream | 4.61 |
| Coarse salt | 0.16 |
| Baking powder | 1.11 |
| Vanilla | 0.34 |
| rOVA | 3.10 |
| Potato starch | 1.48 |

(continued)

| Ingredients | % w/w |
|---|---|
| Xanthan gum | 0.09 |
| Lecithin | 0.05 |
| water | 25.45 |
| Marigold yellow | 0.05 |
| Total | 100.00 |

For each of the recipes, the batter was baked at 325°F until cooked such time that a toothpick, when inserted at the middle of the cake, came out clean.

**Table 5: Results from using rOVA in pound cake compositions**

| | rOVA + Xanthan gum | rOVA + Potato Starch + Xanthan gum | Control Egg pound cake |
|---|---|---|---|
| **Cohesiveness** | *0.6 ± 0.02 a | 0.64 ± 0.02 a | 0.62 ± 0.02 a |
| **Resilience** | 0.31 ± 0 a | 0.36 ± 0.05 a | 0.32 ± 0.01 a |
| **Hardness (g)** | 73.9 ± 2.1 a | 75.5 ± 7.5 a | 75.6 ± 12.7 a |
| **Chewiness (mJ)** | 1.48 ± 0.04 a | 1.78 ± 0.3 a | 1.63 ± 0.5 a |
| **Springiness (mm)** | 3.41 ± 0 a | 3.72 ± 0.21 a | 3.48 ± 0.32 a |
| **Cake height (cm)** | 30.08 ± 1.4 a | 30.07 ± 1.04 a | 30.64 ± 1.01 a |
| *Similar letters within each marker indicate there is no significant difference between the samples (mean ± std dev; $p > 0.05$) | | | |
| Sensory | Appearance: good yellow crumb, compact crumb, light brown crust. Good rise and volume.<br>Aroma: butter, cakey<br>Flavor: buttery, cakey<br>Texture: more moist texture than control cake with egg, not as cohesive as egg control. | Appearance: pale crust color, good yellow crumb color, open pores in crumb like the Control, good rise/volume<br>Aroma: buttery, cakey<br><br>Flavor: cakey, sweet<br>Texture: more moist texture than egg control, more cohesive than control cake with egg. | Appearance: open pores, golden crust, good yellow crumb color<br>Texture: good chewy, slightly dry<br><br>Flavor: cakey, buttery<br><br>Aroma: cakey buttery |

Texture qualities such as cohesiveness, resilience, hardness, chewiness and springiness were measured using a Brookfield CT3 Texture analyzer, 1500g load cell. No significant difference was observed between the Control Egg cakes and cakes made with rOVA in terms of textural properties and cake height. The sensory properties were comparable to the Control cake made with whole egg.

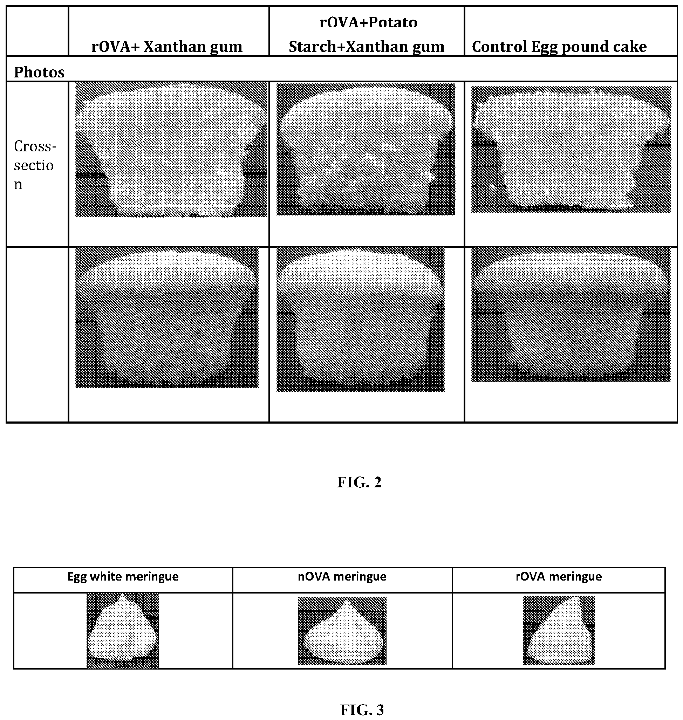

The rOVA in the pound cake demonstrated several functional features with utility in baked goods, as well as for other food products and ingredients. Results are shown in FIG. 2.

**Table 6: Functional features provided by rOVA in pound cakes**

| Functionality | Evidence |
|---|---|
| Foaming | Air cells formed, evident in the crumb structure (cross section photo) |

(continued)

| Functionality | Evidence |
|---|---|
| Whipping | Air incorporation during mixing of batter, evident from air cells in crumb structure |
| Gelling | Protein coagulation upon heating. Creates structure of cake. |
| Binding | Binds with other ingredients, giving strength and structure to cake. Evident from texture and sensory measurements. |
| Springiness | Texture measurement |
| Texturizer | Provides structure while baking, evidenced by textural characteristics: chewiness, hardness, resilience, cohesiveness |

**Example 3: rOVA applications in meringue**

This example examined the feasibility of making meringue with rOVA in the recipe without using cream of tartar.

Material: rOVA (pH: 4.12 as is), nOVA (pH: 6.06 as is), Fresh egg white (pH: 9 as is), Xanthan gum, Sodium lauryl sulfate (SLS), Cream of tartar, Granulated sugar, Flavor.

Equipment: Kitchen Aid, Classic Plus, Breville BOV800XL Smart Electric Oven.

Method: Separated egg white from the egg yolk carefully at the refrigerator temperature and then let egg whites get to room temperature before whipping. Egg white was used to make Control meringue sample. nOVA or rOVA was used to make test samples. Egg white or nOVA or rOVA solution (10% solution) was transferred to a mixer bowl and whipped for 30 seconds at medium speed (to obtain a homogeneous solution), then cream of tartar was added (for egg-whites only) and mixed at high speed until soft peaks form. While beating constantly, sugar was added gradually and beaten at high speed after each addition until sugar was dissolved completely. Continued mixing until a glossy and firm peak was formed and at the end, flavors were added. The soft meringue mix was transferred into the pan. An oven was preheated to 250°F, and meringues were baked for 50 minutes (or until an internal temperature of 160°F). After cooling, meringues were stored in an airtight container.

Exemplary meringue recipes using rOVA can include rOVA between 5-10%, sugar at about 26-32%, flavoring (e.g., 1-4%), water at about 59-64%, xanthan gum at about 0.01-0.5%, sodium lauryl sulfate at about 0.01-0.1% (all w/w). One such exemplary recipe, and comparison recipes with fresh egg white or with native OVA or with rOVA was constructed as shown below:

**Table 7: Recipes**

| | | | Meringue with rOVA and nOVA (same recipe) | |
|---|---|---|---|---|
| **Ingredients** | **% w/w** | | **Ingredients** | **% w/w** |
| Fresh egg white | 68.19 | | nOVA and/or rOVA | 8.14 |
| Cream of tartar | 1.14 | | Sugar | 28.28 |
| Sugar | 28.41 | | Flavor | 2.26 |
| Flavor | 2.26 | | Water | 61.12 |
| **Total weight** | **100** | | Xanthan gum | 0.1 |
| | | | SLS (Sodium lauryl sulfate) | 0.1 |
| | | | **Total weight** | **100** |

**Table 8: Results of meringue recipes**

| | Egg white meringue | nOVA meringue | rOVA meringue |
|---|---|---|---|
| **weight loss %** | *51 ± 1 b | 60 ± 14.6 a | **40 ± 4.5 c** |
| **volume (ml)** | 6.9 ± 1.94a | 7.82 ± 1.5a | 8.05 ±2.16a |
| **Density (g/ml)** | 0.1 ± 0.06 a | 0.07 ± 0.01ab | **0.06 ± 0.01b** |

(continued)

|  | Egg white meringue | nOVA meringue | rOVA meringue |
|---|---|---|---|
| **1/density** | 9.14 | 13.78 | 16.65 |
| **fluffiness** | 100 | 150.5 | **182.1** |
| *Samples with different letters across a row are significantly different (p<0.05; mean $\pm$ std dev). | | | |

Conclusion: Lowest weight loss was observed in meringue with rOVA. Furthermore, rOVA meringue indicated the highest fluffiness compared to the egg control and nOVA. Results are presented in FIG. 3.

The rOVA usage in meringue demonstrates several functional features of rOVA.

**Table 9: Functional features provided by rOVA in meringues**

| Functionality | Evidence |
|---|---|
| Foaming | Increased foam capacity compared to egg white |
| Whipping | Whips easily - Reduced whipping/whisking time compared to egg white |
| Aeration | Holds air bubbles, soft peak |
| Fluffing | Provides increased volume and fluffiness |
| Gelling | Protein coagulation upon heating, provides structure to the meringue sample |

**Example 4: Comparison of foam capacity and foam stability**

This example evaluated the foam capacity/stability and coagulation properties of rOVA and compared it to fresh whole egg, egg white and nOVA.

Materials: store-bought egg, nOVA (Bioceutica), rOVA.

Method: A stock solution of OVA (nOVA or rOVA) was made by mixing 0.7 g OVA in 9.3g distilled water (total volume 10 ml). Cream of tartar was used (see Table 10 below) to adjust pH. Foam was made using a Dremel at speed 3. The time of whisking was recorded. Gel was made by heating 1 ml of sample at 72°C for 10min using a heat block.

**Table 10: pH adjustments to rOVA, nOVA and egg white compositions**

| | pH adjustment | | | |
|---|---|---|---|---|
| | Initial pH | Temperature | Amount of cream of tartar added (g) | pH after adding cream of tartar |
| rOVA solution | 3.86 | 21 | 0 | 3.86 |
| nOVA solution | 5.45 | 20.7 | 0.1 | 4.01 |
| Fresh egg white | 8.57 | 20 | 2 | 4.64 |

Results of the foam capacity and stability are shown in the Table 11 below. In this set, pH was not adjusted.

$$*Foam\ capacity\% = [Initial\ liquid\ Vol.\ (ml)/Foam\ Vol.\ (ml)]*100$$

**Foam stability%= [(Initial liquid Vol. (ml)-Liquid drainage Vol. at 30 min (ml))/Initial liquid Vol. (ml)]*100

**Table 11: Results of foam capacity and stability**

| | Whole egg | Egg white | nOVA |
|---|---|---|---|
| **\*Foam capacity%** | 210 $\pm$ 14.1 a | 300 $\pm$ 0 b | **338.5 $\pm$ 2.2 c** |

(continued)

|  | Whole egg | Egg white | nOVA |
|---|---|---|---|
| **Foam Stability%** | 56 ± 2.8 b | **71 ± 1.4 a** | 59.3 ± 0.92 b |
| time of whisking | >120 | 80 | **19** |
| (second) |  |  |  |
| pH as is | 7.6 | 9.1 | 5.9 |

Conclusion: nOVA at pH 6 indicated the highest foam capacity compared to the egg white; however, its foam stability was lower than the egg white. Results are presented in FIG, 4

The experiment was repeated using cream of tartar to adjust the pH.

**Table 12: Results of foam capacity and stability after pH adjustment using cream of tartar**

|  | Egg white | nOVA | rOVA |
|---|---|---|---|
| **Foam capacity%** | 316.3 ± 5.3 b | **457.9 ± 31.2 a** | 367.9 ± 2.9 b |
| **Foam Stability%** | **83.6 ± 6.2 a** | 65.1 ± 1.3 b | 60.5 ± 0.7 b |
| **time of whisking (second)** | 64 | 19 | 32 |
| **Initial pH (as is)** | 8.57 | 5.45 | 3.86 |
| **Final pH (after adjusting with cream of tartar)** | 4.65 | 4.01 | 3.86 |

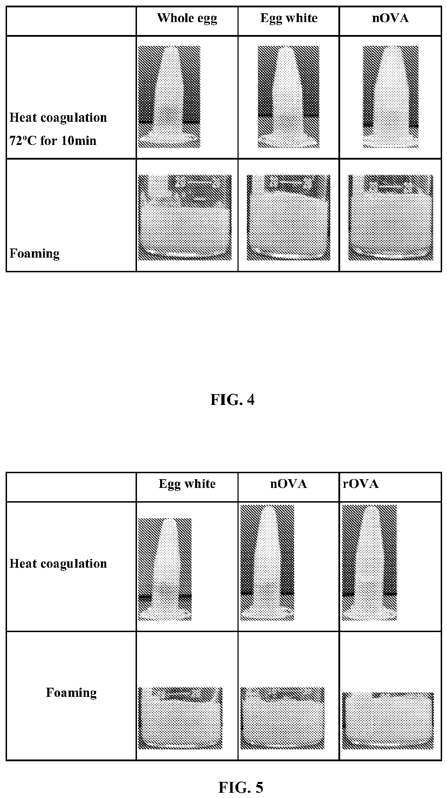

Conclusion: The foam capacity of nOVA after reducing pH was still higher than egg white. The foam capacity of rOVA was higher in value compared to that of fresh egg white. The whisking time for rOVA was half that required for fresh egg white. Results are shown in FIG. 5

**Example 5: Preparation of recombinant chicken ovalbumin expression strain**

*Expression Constructs* Seven expression cassettes were created for expression of *Gallus gallus* OVA (SEQ ID NO: 2) in *Pichia pastoris.*

**Table 13: Expression Cassettes of Interest**

| Strain | Cassette | Promoter | Terminator |
|---|---|---|---|
| Chicken OVA | GgOVA-A1 | K phaffii AOX1 promoter | K phaffii AOX1 transcriptional terminator |
| Chicken OVA | GgOVA-A2 | K phaffii AOX1 promoter | K phaffii AOX1 transcriptional terminator |
| Chicken OVA | GgOVA-A3 | K phaffii AOX1 promoter | K phaffii AOX1 transcriptional terminator |
| Chicken OVA | GgOVA-D1 | K pastoris DAS promoter | K phaffii AOX1 transcriptional terminator |
| Chicken OVA | GgOVA-F2 | K pastoris FLD1 promoter | K phaffii AOX1 transcriptional terminator |
| Chicken OVA | GgOVA-F3 | K pastoris FLD1 promoter | K phaffii AOX1 transcriptional terminator |
| Chicken OVA | HF-1 | K phaffii PEX11 promoter | K phaffii AOX1 transcriptional terminator |

The first three cassettes were made to express a chicken OVA that comprises the amino acid sequence of chicken OVA (SEQ ID NO:2) fused in-frame with a nucleic acid encoding a secretion signal sequence; the expressed fusion protein has the amino acid sequence of (SEQ ID NO: 1). In each of the three cassettes, the Alcohol oxidase 1 (AOX1) promoter was placed upstream of the secretion signal sequence and a *K phaffii* AOX1 transcriptional terminator was placed downstream of the OVA-encoding sequence. These cassettes were labeled GgOVA-A1, GgOVA-A2, and GgOVA-A3 and combined into a first plasmid.

The fourth cassette included a chicken OVA coding sequence (which encodes SEQ ID NO: 2) fused in-frame with a nucleic acid encoding a secretion signal sequence (thereby encoding SEQ ID NO: 1) but with a dihydroxyacetone synthase (DAS2) promoter placed upstream of the secretion signal sequence and a K phaffii AOX1 transcriptional terminator placed downstream of the OVA-encoding sequence. This construct was labeled GgOVA-D1.

The fifth and sixth cassettes included the chicken OVA coding sequence (which encodes SEQ ID NO: 2) fused in-frame with a nucleic acid encoding a secretion signal sequence (thereby encoding SEQ ID NO: 1) but with a formaldehyde dehydrogenase (FLD) promote placed upstream of the secretion signal sequence and a K phaffii AOX1 transcriptional terminator placed downstream of the OVA-encoding sequence. These cassettes were labeled GgOVA-F1 and GgOVA-F2 and were combined with GgOVA-D1 in a second plasmid.

The seventh cassette included the peroxisome biogenesis (PEX11) promoter placed upstream of a Helper factor protein HAC1 coding sequence and a K. phaffii AOX1 transcriptional terminator placed downstream of the Helper factor sequence. This cassette was labeled HF-1 and was transformed into a third plasmid.

The three plasmids were transformed stepwise into a background strain of *Pichiapastoris.* Genomic sequencing confirmed integration of the expression constructs and copy number of each construct is shown in Table 14 below.

**Table 14: Strain Genomic Composition**

| Strain | Cassette | Copies integrated |
|---|---|---|
| Chicken OVA | GgOVA-A1 | 1 |
| | GgOVA-A2 | 1 |
| | GgOVA-A3 | 1 |
| | GgOVA-D1 | 2 |
| | GgOVA-F2 | 2 |
| | GgOVA-F3 | 2 |
| | HF-1 | 8 |

**Example 6: Preparation of recombinant ovalbumin expression strains for Duck and Ostrich**

*Expression Constructs:* one cassette for expression of *Anas platyrhynchos* (duck) OVA and one cassette for expression of *Struthio camelus* (ostrich) OVA were created for expression in *Pichia pastoris.*

**Table 15: Expression cassettes of interest**

| Strain | Cassette | Promoter | ORF | Terminator |
|---|---|---|---|---|
| Duck OVA | ApdOVA | K phaffii AOX1 promoter | Duck OVA | K phaffii AOX1 transcriptional terminator |
| Ostrich OVA | ScOVA | K phaffii AOX1 promoter | Ostrich OVA | K phaffii AOX1 transcriptional terminator |

One expression cassette was created for the expression of ostrich OVA. A nucleic acid encoding *Struthio camelus* OVA (SEQ ID NO: 71) was fused in-frame with a nucleic acid encoding a secretion signal sequence (thereby encoding SEQ ID NO: 72). The ostrich construct included the Alcohol oxidase 1 (AOX1) promoter placed upstream of the secretion signal sequence and a K phaffii AOX1 transcriptional terminator was placed downstream of the OVA sequence. This expression cassette called ScOVA was transformed into *Pichiapastoris.* Successful integration of four copies of the ostrich OVA construct was confirmed by genomic sequencing. See Table 15.

One expression cassette was created for the expression of duck OVA. A nucleic acid encoding *Anas platyrhynchos* OVA (SEQ ID NO: 73) was fused in-frame with a nucleic acid encoding a secretion signal sequence (thereby encoding SEQ ID NO: 74). The duck cassette included the Alcohol oxidase 1 (AOX1) promoter placed upstream of the secretion signal sequence and a K phaffii AOX1 transcriptional terminator was placed downstream of the OVA sequence. This expression cassette called ApdOVA was transformed into *Pichia pastoris.* Successful integration of two copies of the duck OVA construct was confirmed by genomic sequencing. See, Table 16.

**Table 16: Strain genomic composition**

| Strain | Cassette | Copies integrated |
|---|---|---|
| Duck OVA | ApdOVA | 2 |
| Ostrich OVA | ScOVA | 4 |

### Example 7: Fermentation and production of rOVA

*Fermentation:* Strains for fermenting recombinant OVA (rOVA) were each cultured in a bioreactor at ambient conditions. A seed train for the fermentation process began with the inoculation of shake flasks with liquid growth broth. The inoculated shake flasks were kept in a shaker after which the grown *P. pastoris* was transferred to a production-scale reactor.

To expand production, a seed vial of rOVA *P. pastoris* seed strain was removed from cryo-storage and thawed to room temperature. Contents of the thawed seed vials were used to inoculate liquid seed culture media in baffled flasks which were grown at 30°C in shaking incubators. These seed flasks were then transferred and grown in a series of larger and larger seed fermenters (number to vary depending on scale) containing a basal salt media, trace metals, and glucose. Temperature in the seed reactors was controlled at 30°C, pH at 5, and dissolved oxygen (DO) at 30%. pH was maintained by feeding ammonia hydroxide, which also acted as a nitrogen source. Once sufficient cell mass was reached, the grown rOVA *P. pastoris* was inoculated into a production-scale reactor containing basal salt media, trace metals, and glucose.

Like in the seed tanks, the culture was also controlled at 30°C, pH5 and 30% DO throughout the process. pH was again maintained by feeding ammonia hydroxide. During the initial batch glucose phase, the culture was left to consume all glucose and subsequently-produced ethanol. Once the target cell density was achieved and glucose and ethanol concentrations were confirmed to be zero, the glucose fed-batch growth phase was initiated. In this phase, glucose was fed until the culture reached a target cell density. Glucose was fed at a limiting rate to prevent ethanol from building up in the presence of non-zero glucose concentrations. In the final induction phase, the culture was co-fed glucose and methanol which induced it to produce rOVA via the pAOX promoters. Glucose was fed at an amount to produce a desired growth rate, while methanol was fed to maintain the methanol concentration at 1% to ensure that expression was consistently induced. Regular samples were taken throughout the fermentation process for analyses of specific process parameters (e.g., cell density, glucose/methanol concentrations, product titer, and quality). After a designated amount of fermentation time, secreted rOVA was collected and transferred for downstream processing.

The fermentation broth containing the secreted rOVA was subjected to centrifugation at 12,000rpm. The supernatant was clarified using microfiltration. To concentrate the protein and remove excess water, ultrafiltration at room temperature was used. An appropriately sized filter was used to retain the target rOVA while the compounds, salts, and water smaller than rOVA passed through the filter. To reduce the final salt content and conductivity in preparation for chromatography, the concentrated rOVA retentate was dialyzed at pH 3.5 until the final conductivity of the material was 1.7mS/cm. The bulk of the purification was done using cation exchange chromatography at pH 3.5. Citrate buffer containing a high salt concentration of sodium chloride was used to elute the bound rOVA from the resin. To remove the excess salts, the eluant was finally dialyzed to make a final protein solution containing about 5-10% protein and 85-95% water. The final solution was sterilized by passing it through a 0.2um bioburden filter. The water was evaporated using a spray dryer/lyophilizer at appropriate temperatures to produce a final powder containing about 80% protein.

### Example 8: Preparation of Solubilized rOVA

In this example, hydrophobic recombinant chicken rOVA was solubilized and passed through a 0.2μm filter.

Recombinant rOVA was purified through ion exchange chromatography at pH 3.5 and was found to be insoluble. Sodium hydroxide was added to the solution to change the pH to 12.5 and solubilize the rOVA. The rOVA solution at pH 12.5 was passed through a 0.2μm filter. Following filtration, the pH was returned to 6.5 using hydrochloric acid and the rOVA was spray dried or lyophilized. This dried chicken rOVA was then used in the Examples below.

### Example 9: Glycosylation of *Gallus gallus* rOVA

In this example, *Pichia*-secreted rOVA was analyzed for glycosylation patterns.

Native ovalbumin (nOVA) has two potential N-linked glycosylation sites **(FIG. 1A)**. A single site of glycosylation at Asn-292 is found in the egg white. MALDI-TOF analysis has shown that the typical glycans on native OVA are organized as (Man)5(GlcNAc)5(Gal)1 **(FIG. 1A)** (Harvey et al., 2000). Analysis of glycans on rOVA showed a typical glycosylation pattern shown in (FIG. 1B).

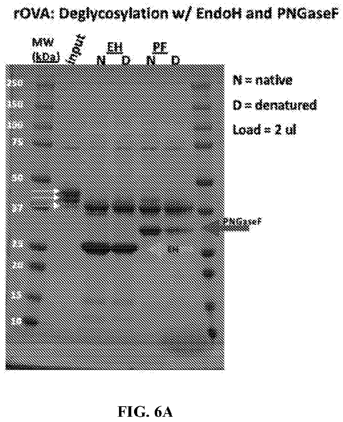

*Pichia* secreted chicken rOVA from the above Example was analyzed by gel electrophoresis migration and observed in three distinct forms (three white arrows pointing to rOVA in the "Input" lane below a) glycosylation-free, b) mono-glycosylated and c) di-glycosylated. Both the mono- and di-glycosylated glycosyl chains were cleaved from the mature rOVA protein using either of the endoglycanases EndoH or PNGaseF. Both the "denatured" or "native" deglycosylation protocols were used (as described in the NEB catalog). The green arrow indicates exogenous EndoH and the purple arrow indicates exogenous PNGaseF added to the *in vitro* reactions **(FIG. 6A)**.

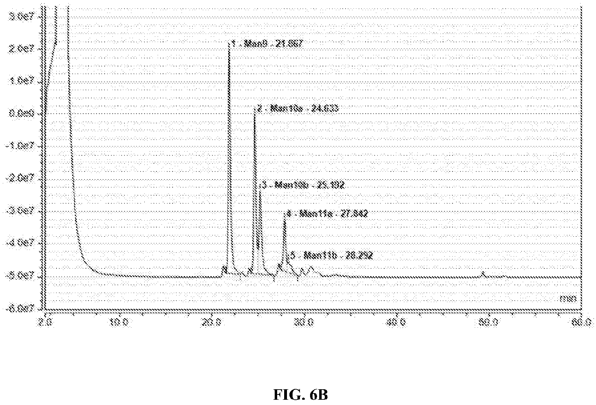

Pichia secreted chicken rOVA was subjected to standard analysis using Mass spectrometry. It was found to have five versions of N-linked Glycans (ManGlcNAc): high-mannose glycans of Man9 (~40%), Man 10 (~ 47%) or Man11 (~13%) type of N-glycan structures (**FIG. 6B**).

**Example 10: Comparison of foaming functionalities of various species rOVA**

In this example, chicken rOVA, duck rOVA and ostrich rOVA were evaluated for properties of foaming ability and foam retention.

rOVA from ostrich and duck were produced, purified and lyophilized using methods similar to those set forth in Example 5 to 7. The ostrich rOVA and duck rOVA remained close to the acidic pH used for purification. Chicken rOVA was produced as set forth in Example 5 and solubilized at pH 12 before removing bioburden and returned to pH 6 before drying as set forth in Example 7.

Lyophilized rOVA samples were blended into distilled water. Clarity and solubility of the rOVA solutions were then assessed visually. All samples were compared to chicken nOVA and chicken rOVA.

Eleven mL of solution (7% w/v of protein) was created for each ostrich rOVA, chicken rOVA, and chicken nOVA. A 6mL solution (7% w/v of protein) was created for duck rOVA due to limited availability of sample. Percent protein of the powders was used in the calculations to determine the amount necessary for a 7% solution. One mL of each solution was reserved before validation in a microtube for later use to test gelation. The samples were divided into 5mL aliquots to be tested for foam capacity and stability.

Each 5mL aliquot was pipetted into a beaker and whipped using the Dremel on speed 3. After a stiff foam was achieved, the foaming time was recorded as well as the initial volume of the foam. Foam capacity was determined by measuring the initial volume of foam following the whipping and comparing against the initial volume of 5mL. Foam Capacity (%) = (volume of foam / initial volume)* 100.

The drainage was measured in 10 minute increments for 30 minutes to gather data for foam stability. The drained volume after 30 minutes was compared to the initial liquid volume (5mL). Foam Stability (%): (Initial volume - drained volume) / initial volume* 100.

Chicken rOVA and ostrich rOVA were adjusted to pH 6 and tested again to ascertain effect of pH.

Chicken nOVA quickly formed stiff white foam. Ostrich rOVA foamed after 15 seconds. Duck rOVA foamed after 20 seconds.

**Table 17: Foaming Parameters for rOVA in various species**

| Sample | pH | Foaming Time (s) | Foam Capacity (%) | Foam Stability (%) |
|---|---|---|---|---|
| Chicken nOVA | 5.87 | 16 | 415 | 66.5 |
| Chicken rOVA | 6.49 | 101 | 257 | 61 |
| Chicken rOVA | 6.08 | 21 | 417 | 66.7 |
| Chicken rOVA | 3.5 | 28 | 472 | 100 |
| Ostrich rOVA | 3.7 | 22 | 490 | 81.5 |
| Ostrich rOVA (pH adjusted) | 5.73 | 55 | 275 | 58 |
| Duck rOVA | 4.3 | 26 | 400 | 70 |
| Egg White | 9.01 | 66.5 | 267.9 | 76.6 |

Table 17 shows the results for foaming time, foaming capacity, foam stability for chicken nOVA, at pH 5.87, chicken rOVA at pH 6.49 and pH 6.08, ostrich rOVA at pH 3.7 and pH 5.73, duck rOVA at pH 4.3 and egg white OVA at pH 9.0. Recombinant OVA from chicken, duck and ostrich generally had a similar or improved foaming capacity and foam stability as compared to egg white and these recombinant OVA proteins provided foaming capacity and foam stability between at least pH 3.5 and 6.5. Foam capacity and foam stability of rOVAs provide utility in compositions such as baked compositions.

**Example 11: Comparison of gelation of various rOVA species**

In this example, chicken, duck, and ostrich rOVA protein were evaluated for gelation properties. Gelation properties provide utility in applications such as cooked egg compositions.

One mL of each OVA solution was reserved for use to test gelation. After the Dremel procedure and foaming test in Example 10 was completed, another 1mL sample was extracted from the drained liquid (containing the OVA) and pipetted into another microtube. Both the fractions collected, before and after foaming, were placed in a water bath and heated to 72°C for 10 minutes. Samples were observed for gel formation.

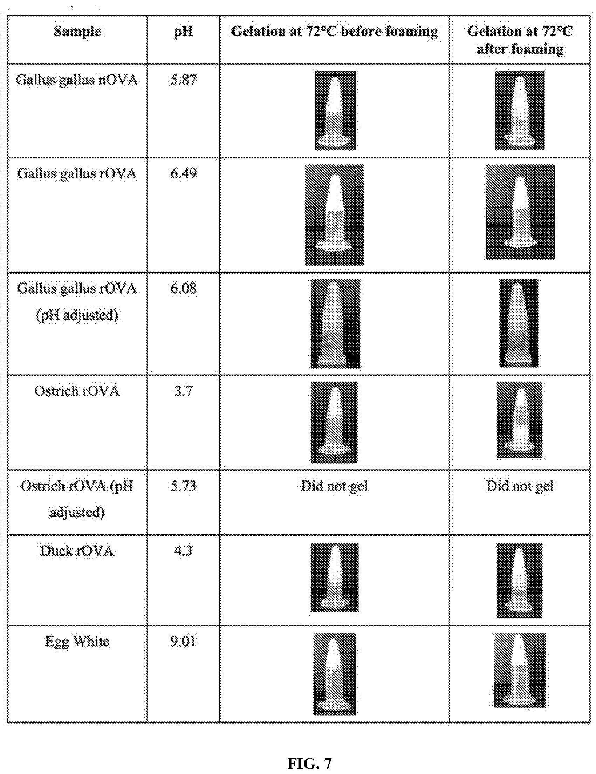

**FIG. 7** shows the results for gelation before and after foaming for chicken nOVA, at pH 5.87, chicken rOVA at pH 6.49 and pH 6.08, ostrich rOVA at PH 3.7 and pH 5.73, duck rOVA at pH 4.3 and egg white OVA at pH 9.0. Duck rOVA showed better gelation characteristics compared to chicken rOVA. Duck rOVA had gelation functionality close to that of natural egg white.

These data showed that the favorable properties disclosed above for the recombinant chicken OVA (see Example 10) are also obtainable with recombinant OVAs from other species.

**Example 12: Comparison of foaming rOVA solutions**

In this example, rOVA (chicken),solutions were compared to fresh egg white and evaluated for properties of foaming ability and foam retention.

Lyophilized samples were blended into aqueous solution (distilled water) at different concentrations and pHs. Clarity and solubility of the solutions was then assessed visually for foaming ability and foaming retention.

Protein solutions were created for each 4% rOVA, 7% rOVA, Fresh Egg White (12% protein), and 12% rOVA. Percent protein of the powders was used in the calculations to determine the amount necessary for each solution. 1 mL of each solution was reserved before validation in a microtube for later use to test gelation. The samples were divided into 5mL aliquots to be tested for foam capacity and stability.

Each 5mL aliquot was pipetted into a beaker and whipped using the Dremel on speed 3. After a stiff foam was achieved, the foaming time was recorded as well as the initial volume of the foam. Foam capacity was determined by measuring the initial volume of foam following the whipping and compare against the initial volume of 5mL. Foam Capacity (%) = (volume of foam / initial volume)* 100.

The drainage was measured in 10-minute increments for 30 minutes to gather data for foam stability. The drained volume after 30 minutes was compared to the initial liquid volume (5mL). Foam Stability (%): (Initial volume - drained volume) / initial volume* 100.

**Table 18: Foaming functionality for chicken rOVA**

| Protein Combination | pH | Foaming Capacity (%) | Foam Stability (%) | Time Spent Foaming (s) |
|---|---|---|---|---|
| Fresh Egg White (12% protein) | 9.01 | 268 | 77 | 67 |
| 4% OVA | 6.05 | 333 | 57 | 25 |
| 7% OVA | 6.03 | 333 | 66 | 19 |
| 12% OVA | 6.05 | 313 | 69 | 18 |

rOVA at 4%, 7% and 12% has greater foaming capacity, more foaming stability, and forms a foam more quickly than fresh egg white.

**Example 13: Browning and sheen properties of rOVA**

In this example, the film formation properties of browning and sheen were evaluated for functionality of rOVA in a bread application. The functionality of rOVA for film formation was evaluated regarding the visual (sensory) characteristics of bread.

Baking instructions: Yeast, sugar and warm water were mixed together in a small bowl and left to sit for five minutes. Flour was mixed into the yeast solution (30 seconds) until a firm dough was formed (mixed for 2 minutes at speed 3). Dough was kneaded on a floured board, placed into a greased bowl and left to rise for 45 minutes at 80°F. Dough was kneaded again, shaped into a 25g mini loaf, and placed in a greased pan. The mini loaf was covered and allowed to rise for 30 minutes at room temperature. A volume of 0.75g of the appropriate wash was applied to the top of the dough balls. Mini loaves were baked at 350°F for eight minutes or until golden brown. Bread loaves' locations were switched in the oven at four minutes to achieve even baking of all samples.

Lists of ingredients and their proportions used in the control bread and other samples are presented in the Table 19 below.

Table 19: Bread Ingredients

| Ingredients | % |
|---|---|
| DI Water | 41.77 |

(continued)

| Ingredients | % |
|---|---|
| Granulated Sugar | 2.94 |
| Bakers Yeast | 1 |
| All-Purpose Flour | 53.62 |
| Salt | 0.67 |
| **Total** | **100.00** |

The formulations used for protein of interest are shown in Table 20.

Table 20: Ingredients used in wash formulations:

| | Egg White Powder | rOVA |
|---|---|---|
| Ingredient | % | % |
| DI water | 90.67 | 91.30 |
| Film forming agent | 9.33 | 8.7 |

Colorimetric assay: Individual sample pictures were analyzed for color data in the RGB spectrum using the Colorgrab application (Loomatix). Sample values were generated using a 2x2 cm cross-section taken from the center of the bread surface. RGB data was then converted to a CIELAB system using the online software www.colormine.org. CIELAB model is a color space system that expresses color in 3 values: L* for the lightness from black (0) to white (100), a* from green (-) to red (+), b* from blue (-) to yellow (+).

Table 21: CIELAB results for bread post baking:

| | L* | a* | b* |
|---|---|---|---|
| **Negative Control** | 63.669 | 1.10972 | 25.4527 |
| **Whole egg** | 62.255 | 8.39894 | 45.57611 |
| **Commercial egg wash substitute** | 68.349 | 0.04763 | 34.7033 |
| **8% Egg white protein** | 76.831 | 2.58977 | 31.1123 |
| **8% rOVA** | 80.135 | 3.24212 | 31.53948 |

rOVA and egg white protein samples had a higher L* value suggesting higher brightness or luminance. Control (no egg wash), commercial egg wash substitute and egg white protein samples had a low a* value suggesting lower redness or brownness as compared to whole egg, and rOVA samples. 8% egg white protein and rOVA samples also had similar b* values, suggesting similar yellow hues as compared to the other samples.

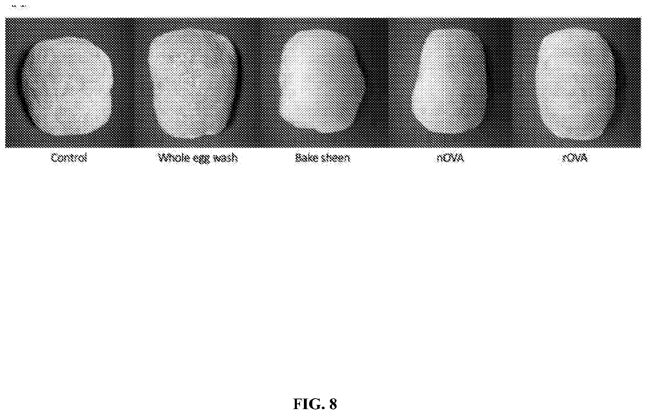

Visual Inspection: The control sample looked pale, wrinkly and had no shine. The sample with whole egg had good browning, great sheen and a smooth surface. The commercial egg wash substitute sample had a smooth surface, slight noticeable sheen but lacked on browning. nOVA samples had good brown, smooth skin but lacked shine/sheen. Similarly, for rOVA samples, it had good browning, smooth skin but lacked shine/sheen. Photographs of the samples are shown in **FIG. 8.** In conclusion, rOVA was able to form a film comparable to a commercial egg wash substitute and nOVA.

**Example 14: Adhesive properties of rOVA**

In this example, rOVA was evaluated for the film formation property of adhesiveness functionality in a bread application creating a uniform film to aid addition of toppings (e.g., sesame seeds).

Retention of sesame seeds: Retention of any topping on cake, bread, bagels or other baked goods is an intended consequence of an egg wash. Sesame seeds were used to evaluate the toping retention function of each film forming agent after baking.

Dough balls and protein of interest were prepared as Example 13. Ten sesame seeds were applied to each dough ball after the application of wash and before baking. Retention of these sesame seeds was calculated based on the amount of seeds stuck to the bread after baking.

The following results were obtained: The control sample with no egg wash had no binding capacity for the sesame seeds and zero sesame seeds were retained on the surface after baking. All other film-forming agents retained all 10 seeds post baking suggesting a 100% retention rate for toppings.

**Table 22: Retention levels of sesame seeds**

| Samples | Negative Control | Commercial egg wash | Whole egg | Egg white protein (EWP) | rOVA 8% |
|---|---|---|---|---|---|
| Retention level | 0% | 100% | 100% | 100% | 100% |

**Example 15: Combined proteins rOVA emulsions**

In this example, the emulsification functionality of recombinant proteins individually and in combination was observed in a salad dressing application.

Lists of ingredients and their proportions used in the control dressing and other samples are presented in the Table 23 below.

**Table 23: List of Ingredients**

| Ingredients for Salad dressing |
|---|
| Canola oil |
| DI water |
| Vinegar |
| Proteins of interest to be tested:<br>        nOVA - 90% Protein content<br>        rOVA - 92% Protein Content<br>        Egg white protein powder - 85.71% Protein content |

Water, vinegar and protein of interest were combined in a mixer for 30 seconds. Oil was gradually added for 30 seconds and mixed for an additional 2.5 minutes. Samples were prepared without vinegar to test the emulsification capabilities of the proteins at neutral pH. pH of the solutions was adjusted using 1N sodium hydroxide. The emulsion was homogenized with a L5MA homogenizer (Silverson) Square Hole shear head mixer for 9 minutes at 4000rpm at ambient temperature.

All emulsion samples were transferred into glass tubes, sealed with a plastic cap, and stored at 4°C or ambient temperature for 3 days. The stability of the samples was evaluated by visually monitoring the height of the visible serum separation at the bottom phase with storage time. Physical stability was monitored for 3 days at both ambient and refrigerated conditions. The stability of the emulsion was expressed as: Creaming Index (CI) = (Ht/H0)*100. Where (H0) represents the initial emulsion height and the height of visible serum separation layer (Ht).

List of ingredients and their proportions used in the control and other salad dressing samples with specific protein of interest are presented in Table 25.

**Table 24: List of Ingredients**

| | Acidic pH | | | | Neutral pH | | |
|---|---|---|---|---|---|---|---|
| | Egg white protein (EWP) 8% | nOVA 8% | rOVA 8% | Negative control | Egg white protein 8% | rOVA 8% | Negative control |
| **Ingredient** | % | % | % | % | % | % | % |
| Canola oil | 30 | 30 | 30 | 30 | 30 | 30 | 30 |
| Water | 54.67 | 55.11 | 55.30 | 64 | 60.67 | 61.30 | 70 |
| Vinegar | 6 | 6 | 6 | 6 | 0 | 0 | 0 |
| Emulsifier | 9.33 | 8.89 | 8.70 | 0 | 9.33 | 8.70 | 0 |
| Total | 100 | 100 | 100 | 100 | 100 | 100 | 100 |

# EP 4 017 287 B1

**Table 25: Creaming Index**

| | Acidic pH | | | | Neutral pH | | |
|---|---|---|---|---|---|---|---|
| | 8% EWP | 8% nOVA | 8% rOVA | Negative control | 8% EWP | 8% rOVA | Negative control |
| Day 0 | 0 | 0 | 0 | 40 | 0 | 0 | 40 |
| Day 1 Ambient | 40 | 50 | 5 | 60 | - | - | - |
| Day 1 Refrigerated | 40 | 50 | 5 | 90 | - | - | - |
| Day 2 Ambient | 40 | 50 | 10 | 70 | - | - | - |
| Day 2 Refrigerated | 40 | 50 | 10 | 90 | - | - | - |
| Day 3 Ambient | 40 | 50 | 15 | 70 | 38 | 41 | 39 |
| Day 3 Refrigerated | 40 | 50 | 15 | 90 | 38 | 40 | 43 |

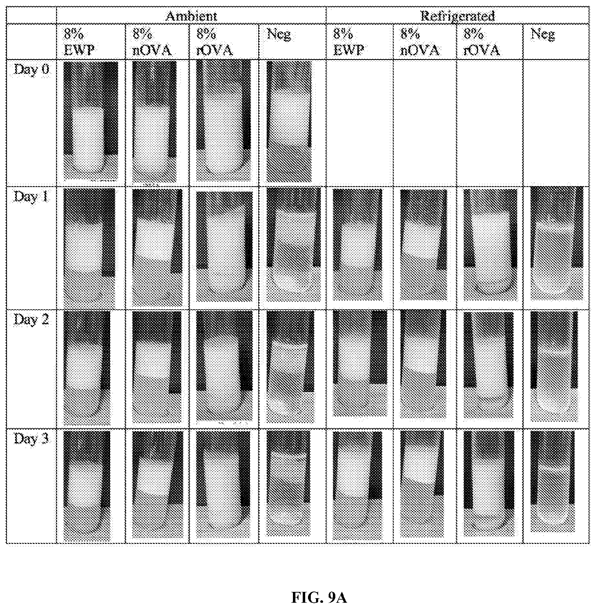

Acidic pH results: On day 0, all samples except the negative control showed good emulsification properties. Thereafter, the samples were stored in ambient temperature or refrigerated temperatures to monitor stability. Samples with egg white protein (EWP) had a slight yellow appearance and separated on day 1 for both conditions of storage. Control samples separated immediately on day 1 for both conditions of storage. Eight percent nOVA also exhibited emulsion breakage on day 1, however, recombinant OVA exhibited good emulsion properties with only minimally noticeable separation. The emulsion remained equally stable until day 3 without any further separation observed. Overall, 8% rOVA performed significantly better than 8% nOVA. rOVA also exhibited better emulsion stability than EWP. Photographs of the samples are shown in **FIG. 9A.**

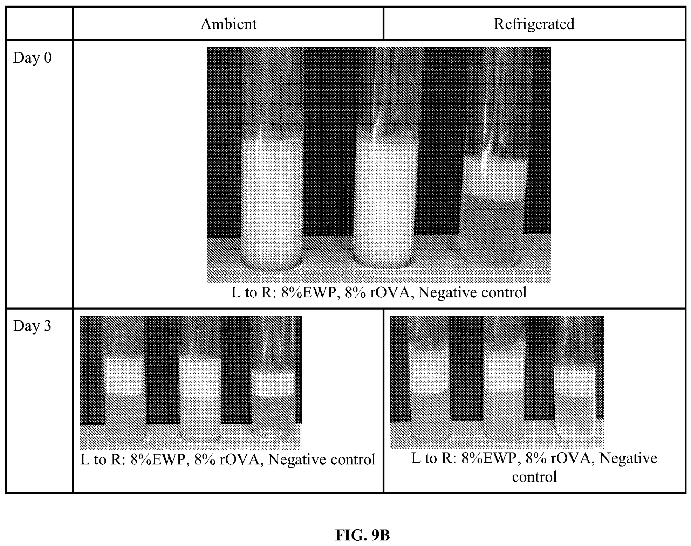

Neutral pH results: Emulsion stability of rOVA was comparable to egg white proteins on day 0 and 3. Neither rOVA, nor egg white proteins were able to maintain emulsion stability over three days in refrigerated form or at ambient temperature. Photographs of the samples are shown in **FIG. 9B.**

## Example 16: Foaming functionality

In this example, the foaming functionality of rOVA was observed in an alcohol-based drink (e.g., such as a Whiskey Sour which includes a foaming agent).

Bourbon whisky, fresh lemon juice, simple syrup, and protein of interest were combined in a cocktail shaker and shaken for 15 seconds. Ice was added to the cocktail shaker and the mixture shaken for another 15 seconds. Shaken mixture was poured into a glass and observed.

Formulations: Control formulation included natural egg white. The negative formulation was prepared without any egg white.

**Table 26: List of ingredients and the formulations**

| Ingredient | Ounces | mL |
|---|---|---|
| Bourbon Whiskey | 2 | 59 |
| Fresh Lemon Juice | 0.75 | 22.125 |
| Simple syrup | 0.5 | 14.75 |
| Egg white | 0.5 | 14.75 |
| Total | 3.75 | 110.625 |

The proteins of interest were used to substitute the natural egg white protein and the following formulations were used:

**Table 27: Protein formulation**

| Ingredients | 7% rOVA | 12% rOVA |
|---|---|---|
| rOVA | 8.40 | 14.41 |
| Water | 91.60 | 85.59 |

(continued)

| Ingredients | 7% rOVA | 12% rOVA |
|---|---|---|
| Total | 100 | 100 |

The pH of the rOVA solutions was adjusted to pH 6 (with 1M NaOH) to provide optimal foaming performance.

Original recipe used 0.5 oz egg white and the same proportion was used for recombinant protein testing. rOVA at 7% and 12% foamed well but no significant difference was observed between the two levels.

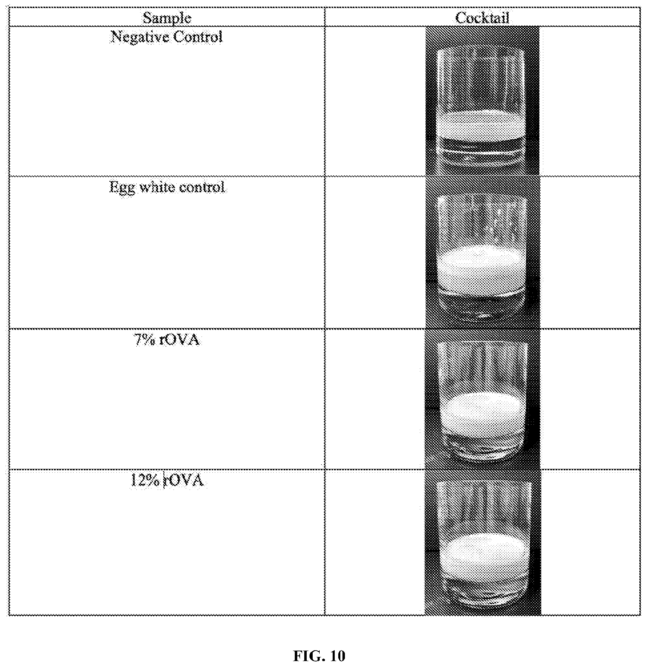

Photographs of craft cocktails prepared with the samples are shown in **FIG. 10.**

**Example 17: Burger Binding**

In this example, texture analysis was used to observe hardness attributes along with cohesiveness, springiness and chewiness of both raw and cooked vegan burgers made with rOVA and other binding agents.

The objective of this example was to evaluate the binding functionality of rOVA. Parameters such as appearance (how well the burger held together), textural aspects such as cohesiveness, springiness, chewiness and hardness were evaluated and compared against egg white, nOVA and commercially used non-protein binder.

*Materials:* Dry base ingredients: Extruded soy protein 1 (Arcon T U172 (158172)), Extruded soy protein 2 (Arcon T Caramel Crumble 240 (158225)), Extruded soy protein 3 (Arcon T U-118 (158118)), Binding agent/Protein of interest. Wet ingredients: Canola oil, coconut oil, Water. Binding agents of interest to be tested: Natural egg white protein ("NEW"), Methylcellulose ("MC"), nOVA 90% Protein content, rOVA (chicken) 92% Protein Content.

*Mixing:* Extruded soy protein 1 was mixed with 1/3rd amount of water for 2.5 min. The remaining extrudated samples and water were combined with the previous mix for another 7.5 min. The blend was chilled in the freezer for 10 minutes. The binding agent was added and mixed in for 30 seconds. Canola and coconut oil blend was added and mixed for 30 seconds. The mixture was chilled in the freezer for 5 minutes, then molded into 5g burger forms and frozen.

*Cooking:* The frozen burger samples were thawed in the refrigerator to a 4°C internal temperature. The samples were cooked on a griddle set at 350°F for 5-6 min until an internal temperature of 165°F was reached.

*Formulations:* List of ingredients and their proportions used in the control and other experimental burger samples, with specific protein of interest, are presented below in Table 28.

**Table 28: List of Ingredients.**

| | Control - Methylcellulose | Natural Egg White | nOVA | rOVA |
|---|---|---|---|---|
| **Ingredients** | % | % | % | % |
| Extruded soy protein 1 | 5 | 5 | 5 | 5 |
| Extruded soy protein 2 | 13 | 13 | 13 | 13 |
| Extruded soy protein 3 | 8 | 8 | 8 | 8 |
| Binding agent | 0.7 | 25 | 5 | 5 |
| Canola oil | 12 | 12 | 12 | 12 |
| Coconut oil | 6.5 | 6.5 | 6.5 | 6.5 |
| Water | 54.8 | 30.5 | 50.5 | 50.5 |
| **Total** | **100.00** | **100.00** | **100.00** | **100.00** |

*Texture Analysis:* Texture analysis was performed to analyze the attributes of vegan burgers against the control. Texture analysis was used to quantify hardness attributes along with cohesiveness, springiness and chewiness.

The textural properties of vegan burgers were measured using a CT3 Brookfield Texture Analyzer (1500 g load cell). The test parameters were used are presented in Table 29.

**Table 29: Test parameters used for three-point bend test to measure hardness of vegan burgers using a CT3 Brookfield Texture Analyzer**

| Test type | Texture Profile Analysis (TPA) |
|---|---|
| **Probe** | TA52 (Mohrs shear blade) |

# EP 4 017 287 B1

(continued)

| Base Fixture | TA-Base Fixture |
|---|---|
| Target type | Distance |
| Target value | 5 mm |
| Trigger load | 15 g |
| Test speed | 0.5 mm/s |
| Post test speed | 4.5 mm/s |
| Textural properties | Hardness 1 (g), Hardness 2 (g), Cohesiveness, Springiness, Chewiness |
| Average Sample dimensions (Diameter*Height) | 25 mm *12.5 mm |

The frozen samples were thawed in the refrigerator to a 4°C internal temperature and tested for raw binding. The thawed samples were also cooked and used to measure the cooked binding values.

*Findings for raw binding:* In terms of hardness, rOVA was significantly higher than methylcellulose and natural egg white and no difference was observed between nOVA and rOVA. All the samples were similar in terms of cohesiveness and springiness. rOVA exhibited significantly more chewiness than methylcellulose and natural egg white. Results are presented in Table 30.

Table 30: Texture (TPA) results for raw binding in terms of hardness, cohesiveness, springiness and chewiness. Data that does not share the same letter within a specific attribute is significantly different from each other (p<0.05). The results were averaged over n=3.

**Table 30: Texture (TPA) results for raw binding in vegan burgers**

| Sample | Hardness 1 (g) | Hardness 2 (g) | Cohesiveness | Springiness | Chewiness |
|---|---|---|---|---|---|
| methylcellu lose 0.7% | 58.27 ± 10.17 (a) | 40.53 ± 9.59 (a) | 0.12 ± 0.07 (a) | 0.42 ± 0.05 (a) | 3.0 ± 1.56 (a) |
| natural egg white 25% | 45.27 ± 9.45 (a) | 33.20 ± 5.02 (a) | 0.21 ± 0.03 (a) | 0.34 ± 0.1 (a) | 3.33 ± 1.26 (ab) |
| nOVA 5% | 81 ± 4.39 (ab) | 44.27 ± 6.45 (a) | 0.18 ± 0.01 (a) | 0.46 ± 0.04 (a) | 6.93 ± 1.12 (bc) |
| rOVA 5% | 145.07 ± 52.85 (b) | 62.80 ± 21.70 (a) | 0.13 ± 0.01 (a) | 0.47 ± 0.04 (a) | 8.23 ± 1.86 (c) |

*Findings for cooked binding:* rOVA exhibited significantly higher hardness values than methylcellulose and natural egg white. All the samples were similar to each other in terms of cohesiveness. For springiness, methylcellulose samples exhibited significantly lower values than natural egg white, nOVA and rOVA. Both nOVA and rOVA samples exhibited higher values chewiness values than methylcellulose. Results are presented in Table 31.

Table 31: Texture (TPA) results for cooked binding in terms of hardness, cohesiveness, springiness and chewiness. Data that does not share the same letter within a specific attribute is significantly different from each other (p<0.05). The results are averaged over n=3.

**Table 31: Texture (TPA) results for cooked binding in vegan burgers**

| Sample | Hardness 1 (g) | Hardness 2 (g) | Cohesiveness | Springiness | Chewiness |
|---|---|---|---|---|---|
| Methylcellulose 0.7% | 281.73 ± 154.7 (a) | 215.80 ± 161.84 (a) | 0.37 ± 0.07 (a) | 0.69 ± 0.05 (a) | 76.03 ± 55.15 (a) |
| Natural egg white 25% | 390.33 ± 158.15 (a) | 304.27 ± 55.83 (a) | 0.57 ± 0.11 (a) | 0.80 ± 0.03 (b) | 178.07 ± 65.85 (ab) |
| nOVA 5% | 617.07 ± 197.49 (ab) | 464.07 ± 135.33 (ab) | 0.56 ± 0.08 (a) | 0.81 ± 0.05 (b) | 285.5 ± 104.72 (bc) |
| rOVA 5% | 922.0 ± 96.71 (b) | 712.33 ± 78.23 (b) | 0.51 ± 0.08 (a) | 0.86 ± 0.02 (b) | 398.13 ± 44.37 (c) |

# EP 4 017 287 B1

**Example 18: Egg white patty**

In this example, the suitability of inclusion of native and recombinant protein OVA in an egg white patty application as an example of cooked egg systems was evaluated. Parameters such as nutritional value of fresh egg white when substituted by OVA and effects on texture (in terms of functionality) and appearance were evaluated.

**Table 32: List of ingredients used to prepare egg white patties**

| Ingredients |
|---|
| Dry base ingredients: Gellan gum (LT100 - Modernist pantry), baking powder (Trader Joe's), salt (The spice club), Sodium Alginate (CP Kelco), Psyllium (CFF) |
| Wet ingredients: Coconut oil, canola oil (Crisco), tapioca syrup (Ciranda) , pineapple yellow AET color (Sensient), water |
| Proteins of interest to be tested: <br>     - Natural egg white <br>     - nOVA (Neova Technologies) - 90% Protein content <br>     - rOVA (Clara Foods: 008USU CW) - 86.1% Protein Content |

*Mixing:* The dry ingredients from Table 32, except sodium alginate were mixed together. The tapioca syrup, sodium alginate and lemon-yellow color were blended separately in water. All ingredients were mixed with oil and vortexed till all ingredients are dissolved. The mixture was allowed to equilibrate by allowing to stand for 10 minutes.

*Cooking:* A griddle was used to cook the samples. The griddle was set to 250 °F and ½ inch diameter ring molds were used to cook samples. The molds were sprayed with oil and the mixture was poured into the molds. 1/2 ice cubes were added to the molds to generate steam. The patties were allowed to cook and another ice cube was added. The patties were cooked for 5 minutes and the lid was opened. The ring molds with the cooked samples to serving plates.

The textural properties of egg white patties were measured using a CT3 Brookfield Texture Analyzer (1500 g load cell). A TPA compression test was used to compress and measure the hardness of egg white patties. Four samples from each set were analyzed to compare. The following test parameters were used:

**Table 33: Test parameters used for TPA test to measure textural properties of patty:**

| Test type | TPA |
|---|---|
| Test parameters | 50% deformation |
| Probe | TA4 (38mm diameter cylinder) |
| Base Fixture | Base fixture |
| Trigger load | 5 g |
| Test speed | 2 mm/s |
| Textural properties | Hardness (g), Adhesiveness, Cohesiveness, |
| | Chewiness, Gumminess |
| Sample dimension (Height) * (Diameter) | ~12mm * 12mm |

**Results:**

**Table 34: Texture Analyzer results**

| Sample/ Attribute | Hardness 1 (g) | Hardness 2 (g) | Adhesiveness | Fracturability | Cohesiveness | Gumminess | Chewiness |
|---|---|---|---|---|---|---|---|
| Natural egg white | 726.3 ± 6.65 a | 652 ± 15.56 a | 0.375 ± 0.11 a | 726.7 ± 7.21 a | 0.765 ± 0.05 a | 555.1 ± 44.55 a | 33.75 ± 0.05 ab |
| nOVA | 817.6 ± 174.51 a | 761.3 ± 171.54 a | 0.315 ± 0.02 a | 817.6 ± 174.51 a | 0.71 ± 0.01 a | 583.55 ± 133.86 a | 50.95 ± 9.40 a |

(continued)

| Sample/ Attribute | Hardness 1 (g) | Hardness 2 (g) | Adhesiveness | Fracturability | Cohesiveness | Gumminess | Chewiness |
|---|---|---|---|---|---|---|---|
| rOVA | 869.9 ± 58.12 a | 747.1 ± 50.49 a | 0.185 ± 0.16 a | 869.9 ± 58.12 a | 0.55 ± 0.04 a | 484.65 ± 3.46 a | 25.53 ± 3.82 b |

Data that does not share the same letter for a given attribute is significantly different from each other (p<0.05)

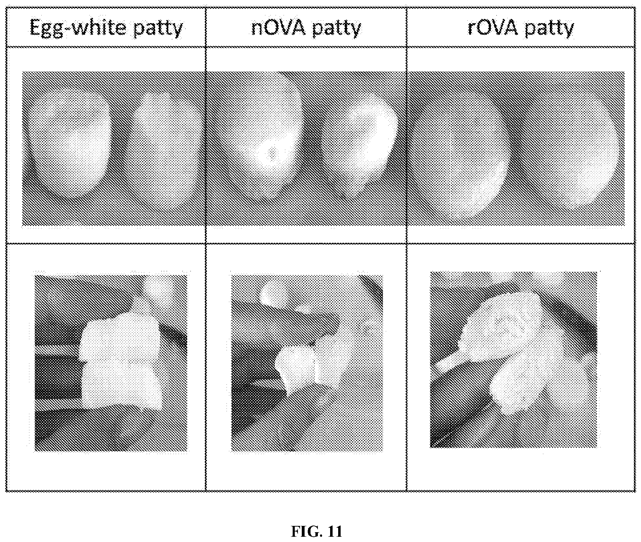

*Findings:* All the samples, natural egg white, nOVA and rOVA were statistically similar in terms of hardness, adhesiveness, fracturability, cohesiveness and gumminess. For chewiness, natural egg white patty was similar to nOVA and rOVA individually, however, nOVA and rOVA were statistically different from each other. nOVA had higher chewiness values as compared to other samples. Overall, OVA protein, in both native and recombinant form, provides a good substitute to natural egg white in a non-animal patty (cooked egg application). rOVA liquid formulation was thicker in viscosity than nOVA sample and egg white sample. Results are shown in **FIG. 11.**

**Example 19: Meringue**

The functionality of rOVA in a meringue food system compared to fresh egg white was evaluated in this example.

Material:

- rOVA (Lyo 008; pH: 6.7 as is)
- Fresh egg white (pH: 9 as is)
- Sugar (C&H Sugar, Pure Cane, Granulated white)
- Xanthan - pre hydrated Ticaxan - Tic Gums
- TEC **(Triethyl Citrate)**
- SLS (Sodium lauryl sulfate)
- Kitchen Aid, Classic Plus
- Breville BOV800XL Smart Electric Oven

Method: Egg white was separated from the egg yolk carefully at the refrigerator temperature and let egg whites get to the room temperature before whipping. rOVA powder, SLS, Xanthan gum and TEC were reconstituted in DI water at the room temperature. The mixture was whipped for 30 seconds at speed 5 (to obtain a homogeneous solution), then mixed at speed 8 until soft peaks formed. While beating constantly, sugar was added gradually and beat at high speed after each addition until sugar was dissolved before adding the next. Mixing was continued until a glossy and firm peak was formed. Oven (Breville BOV800XL Smart Electric Oven) was heated to 200°F; meringues were baked for 70 minutes (or until light and crisp but not brown. After cooling, meringues were stored in an airtight container. Whipping time to produced firm foam for each protein solution was recorded.

**Table 35: Formulations**

| Fresh egg white | | | rOVA8.3% + SLS + Xanthan gum | | | rOVA8.3% + TEC + Xanthan gum | |
|---|---|---|---|---|---|---|---|
| Ingredients | Percentage % | | Ingredients | Percentage % | | Ingredients | Percentage % |
| Fresh egg white | 70.6 | | rOVA | 9.5 | | rOVA | 9.5 |
| Sugar | 29.4 | | Sugar | 29 | | Sugar | 29 |
| - | - | | Water | 61.3 | | Water | 61.3 |
| - | - | | Xanthan gum | 0.1 | | Xanthan gum | 0.1 |
| - | - | | SLS | 0.1 | | TEC | 0.048 |
| Total weight | 100 | | Total weight | 100 | | Total weight | 100 |

**Table 36: Physical parameters of meringues**

| Parameter | Fresh egg white | rOVA 8.3% + SLS + Xanthan gum | rOVA 8.3% + TEC + Xanthan gum |
|---|---|---|---|
| weight loss % | * 60 ± 2 | 60 ± 1.1 | 58 ± 2.5 |
| volume (ml) | 7 ± 1.5 | 7.3 ± 1.5 | 7.9 ± 2 |
| foam density (g/ml) | 0.19 | 0.2 | 0.22 |
| Meringue density (g/ml) | 0.056 ± 0.014 | 0.074 ± 0.02 | 0.064 ± 0.018 |
| *Average ± standard deviation (n=6) | | | |

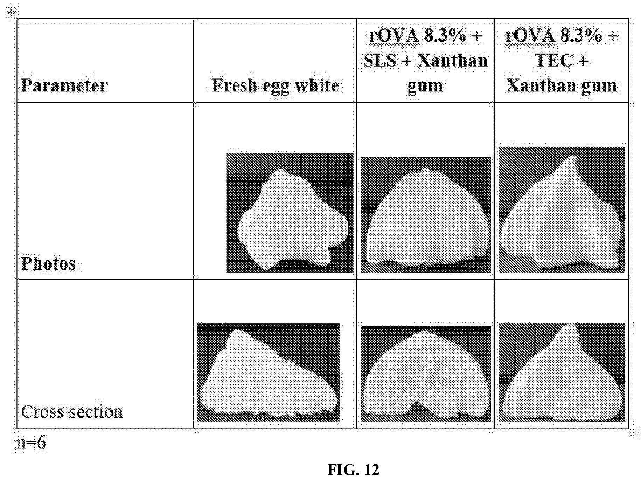

*Findings:* rOVA produces meringue that is comparable to fresh egg white sample in terms of physical parameters. The appearance of rOVA meringues were visually better than fresh egg white controls. The ridges were more well defined in rOVA meringue and the samples were whiter compared to the fresh egg white control. Results are shown in **FIG. 12.**

**Example 20: Effect of pH on gelation characteristics**

The effects of different pH conditions on the gelation characteristics of rOVA compositions in comparison to fresh egg white was evaluated in this example.

**Table 37: Materials:**

| Ingredients |
|---|
| DI water, 1N Hydrochloric acid, 1N Sodium hydroxide, 3N Sodium hydroxide |
| Proteins of interests<br>      - rOVA (008USU_CW - 86.1% protein content)<br>      - Egg white protein (Modernist pantry - 85.71% protein content) |

Method:

1. 7% protein solution was prepared for both rOVA and egg white protein
2. Based on the native pH, the pH of the solution was adjusted to pH 3,4,5,6 with 1N HCl
3. pH was also adjusted to the alkaline spectrum of pH 7, 8, 9, 10, 11 and 12 with microliter amounts of 1N and 3N sodium hydroxide
4. All solutions were gelled at 85°C for 5min and then cooled at room temperature
5. All the gels/solutions were taken out and evaluated visually for gel characteristics

**Table 38: Results: pH was recorded as follows before any pH adjustments:**

| Sample | pH |
|---|---|
| 7% EWP | 6.98 |
| 7% rOVA | 6.82 |

*Findings:* Egg white protein exhibited gelling properties at all pH's while forming firm gels at pH 4-10. The solutions for both EWP and rOVA at pH 11 and pH 12 were clear liquids, however, only EWP gelled into clear gels, while rOVA remained in solution at pH 11 and 12. rOVA 7% solutions gelled at pH 6, 7, 8 and 9. Dramatic increase in viscosity was observed for rOVA solutions at pH 5 and lower. All EWP gels had a strong egg-like smell, while for rOVA, only solutions/gels for pH 9-12 had an egg-like smell. pH 3.5- 8 for rOVA did not have any characteristic smell properties. EWP and rOVA both gelled at pH 6-9; however, EWP gels were stronger and firmer than rOVA gels. Overall, although EWP exhibited better gelling properties than rOVA over a broader pH spectrum, it came with the presence of a strong egg-like smell. rOVA provided gelling properties in the pH 6-8 range and provided sensory neutrality (e.g., no smell). At pH 8 and 9, rOVA provided clear firm gel which can have unique value proposition in embodiments requiring transparent visual appearance.

**Example 21: Protein Bars**

rOVA was used as a protein source in a protein bar application and compared to eff white proteins and nOVA.

Preparation instructions:

In a small mixer, dates, nuts were chopped/blended. Dates, nuts, cocoa and the protein of interest were added in a mixing bowl till a homogenous mixture was formed. The mixture was split into two equal parts and one part was tested as the unbaked version. The other half was baked in an oven at 350F for 10 minutes.

**Table 39: List of Ingredients and their proportions used in control formulation:**

| Ingredients | Amount (%) |
|---|---|
| Dates | 78.53 |
| Nuts | 17.47 |
| Cocoa | 4 |
| Total | 100 |

For formulations with inclusion of protein powders, the dates and nuts inclusion was reduced, however keeping the dates: nuts ratio constant at a 4.5 level.

**Table 40:** List of Ingredients and their proportions used in egg white protein formulations:

| Ingredients | 4% protein | 8% protein | 12% protein | 16% protein | 23% protein |
|---|---|---|---|---|---|
| Dates | 74.73 | 70.93 | 67.13 | 63.27 | 56.62 |
| Nuts | 16.60 | 15.73 | 14.87 | 14.07 | 12.54 |
| Cocoa | 4 | 4 | 4 | 4 | 4 |
| Protein powder | 4.67 | 9.33 | 14 | 18.67 | 26.84 |
| Total | 100 | 100 | 100 | 100 | 100 |

**Table 41: List of Ingredients and their proportions used in nOVA formulations:**

| Ingredients | 4% protein | 8% protein | 12% protein | 16% protein | 23% protein |
|---|---|---|---|---|---|
| Dates | 75.02 | 71.25 | 67.67 | 64.01 | 57.72 |
| Nuts | 16.54 | 15.86 | 13.33 | 14.20 | 12 |
| Cocoa | 4 | 4 | 4 | 4 | 4 |
| Protein powder | 4.45 | 8.89 | 15 | 17.78 | 28.75 |
| Total | 100 | 100 | 100 | 100 | 100 |

**Table 42: List of Ingredients and their proportions used in rOVA formulations:**

| Ingredients | 4% protein | 8% protein | 12% protein | 16% protein | 23% protein |
|---|---|---|---|---|---|
| Dates | 74.60 | 70.66 | 66.76 | 62.86 | 55.92 |
| Nuts | 16.6 | 15.73 | 14.83 | 13.93 | 12.52 |
| Cocoa | 4 | 4 | 4 | 4 | 4 |

(continued)

| Ingredients | 4% protein | 8% protein | 12% protein | 16% protein | 23% protein |
|---|---|---|---|---|---|
| Protein powder | 4.8 | 9.60 | 14.40 | 19.21 | 27.57 |
| Total | 100 | 100 | 100 | 100 | 100 |

Texture analysis: The textural properties of the protein bar (baked and unbaked) were measured using a CT3 Brookfield Texture Analyzer (1500 g load cell). A three point bend test was used to snap, bend and measure the hardness of the protein bar. One sample for each protein inclusion level was analyzed. The following test parameters were used:

**Table 43: Test parameters used for three-point bend test to measure hardness of crackers using a CT3 Brookfield Texture Analyzer**

| Test type | Rupture test |
|---|---|
| **Probe** | TA7 blade |
| **Base Fixture** | TA-TPB |
| **Trigger load** | 5 g |
| **Correction load** | 30 g |
| **Test speed** | 3 mm/s |
| **Sample rate** | 30 points/sec |
| **Distance between support arms** | 2.5 cm |
| **Textural properties** | Hardness (g) |

**Table 44: Texture analysis test results for unbaked protein bar samples: (n=1)**

| Sample | Hardness (g) for protein inclusion levels | | | | | |
|---|---|---|---|---|---|---|
| | Control (0%) | 4% | 8% | 12% | 16% | 23% |
| Egg white protein | 113.9 | 168.8 | 319.2 | 422.8 | 475 | 597.8 |
| nOVA | | 204.8 | 231 | 408 | 420.05 | 443.8 |
| rOVA | | 182 | 222.6 | 314.4 | 418 | 689.8 |

**Table 45: Texture analysis test results for baked protein bar samples: (n=1)**

| Sample | Hardness (g) for protein inclusion levels | | | | | |
|---|---|---|---|---|---|---|
| | Control (0%) | 4% | 8% | 12% | 16% | 23% |
| Egg white protein | 902.7 | 1499.6 | 1484 | 1561 | 1553.4 | 1609.4 |
| nOVA | | 1505.4 | 1523.8 | 1542.2 | 1585 | 1662.8 |
| rOVA | | 1485.2 | 1530 | 1561 | 1522.4 | 1552.8 |

For the unbaked samples, the control sample with no protein had the lowest hardness values. For all the proteins of interest, EWP, nOVA and rOVA, hardness values increased with increasing protein content. Egg white protein samples had higher hardness values than nOVA and rOVA samples at 8, 12, 16 and 23%. nOVA samples had minimal increase in hardness from 12-23% protein inclusion. nOVA and rOVA sample hardness was comparable at 4, 8, 12 and 16%. However, rOVA had a much higher hardness value for 23% protein inclusion.

Overall, the hardness of the baked samples was much higher than the unbaked samples. The control sample had the lowest hardness. All the samples with protein inclusions were much harder even at lower protein inclusion rates.

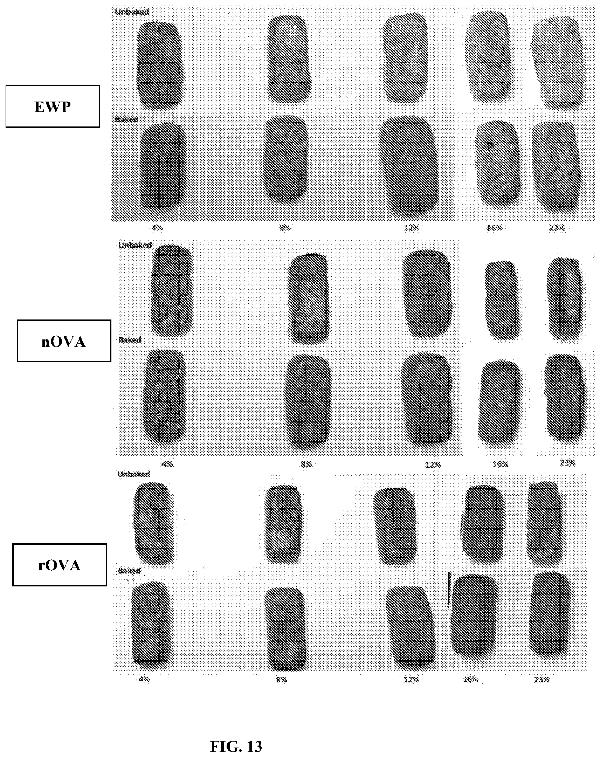

The upper threshold limit (load cell) for the TA unit is 1500 g. All the baked protein samples reached the threshold value making it difficult to identify subtle differences between the samples. nOVA and rOVA sample hardness was comparable at 4, 8, 12 and 16% for both, unbaked and baked protein bar. Photos are shown in **FIG. 13.**

**Claims**

1. A food item comprising an ingredient composition, the ingredient composition comprising a recombinant ovalbumin (rOVA), wherein the rOVA is present in the food item in an amount between 2% and 15% (w/w), and wherein the food item does not comprise any egg-white proteins except rOVA.

2. The food item of claim 1, wherein the food item is an egg-less food item.

3. The food item of any one of claims 1 or 2, wherein the ingredient composition is a powder ingredient composition comprising at least 75% rOVA (w/w of total protein or w/w of total composition), optionally wherein the powder ingredient composition is a concentrate.

4. The food item of any one of claims 1-3, wherein

   (a) the rOVA comprises a polypeptide represented by an amino acid sequence selected from the group consisting of SEQ ID NO: 1-2 or an amino acid sequence with at least 97% identity with one of SEQ ID NO 1-2; or
   (b) the rOVA comprises an amino acid sequence of a duck OVA, an ostrich OVA, or a chicken OVA.

5. The food item of any one of the previous claims, wherein the amino acid sequence of the rOVA lacks an N-terminal methionine.

6. The food item of any one of the previous claims, wherein

   (a) the rOVA provides to the food item a foam capacity of at least 20%, 30%, 40%, or 50% greater than native egg white;
   (b) the rOVA provides to the food item a hardness that is greater than native egg white; or
   (c) the rOVA provides to the egg-less food item a chewiness that is greater than native egg white.

7. The food item of any one of the previous claims, wherein the rOVA is expressed by a yeast host cell or a fungal host cell.

8. The food item of any one of the previous claims, wherein the rOVA is expressed by a *Pichia* sp. host cell and a glycosylation pattern of the rOVA is devoid of N-linked galactose units.

9. The food item of any one of claims 1-8, wherein rOVA is present in the egg-less food item in an amount of less than 8% w/w.

10. The food item of any one of claims 1-9, further comprising at least one fat or oil and at least one grain starch.

11. The food item of any one of claims 2-10 wherein

    (a) the egg-less food item is a baked product; or
    (b) the egg-less food item is a cake, a bread, a roll, a pastry, a cracker, a muffin, a scone, a bagel, a biscuit, or a cookie.

12. The food item of claim 11, wherein the egg-less food item has a crumb structure comparable to or better than a similar food item made with a natural egg white or a natural whole egg.

13. The food item of claim 2, wherein the egg-less food item is an emulsified product further comprising at least one fat or oil and water.

**Patentansprüche**

1. Lebensmittelartikel, umfassend eine Zutatenzusammensetzung, die Zutatenzusammensetzung umfassend ein rekombinantes Ovalbumin (rOVA), wobei das rOVA in dem Lebensmittelartikel in einer Menge von zwischen 2 Gew.-% und 15 Gew.-% vorliegt und wobei der Lebensmittelartikel neben rOVA keine Eiklarproteine umfasst.

2. Lebensmittelartikel nach Anspruch 1, wobei der Lebensmittelartikel ein eifreier Lebensmittelartikel ist.

3. Lebensmittelartikel nach einem der Ansprüche 1 und 2, wobei die Zutatenzusammensetzung eine Pulverzutatenzusammensetzung ist, umfassend mindestens 75 Gew.-% rOVA (bezogen auf das Gewicht des Gesamtproteins oder auf das Gewicht der Gesamtzusammensetzung), optional wobei die Pulverzutatenzusammensetzung ein Konzentrat ist.

4. Lebensmittelartikel nach einem der Ansprüche 1-3, wobei

   (a) das rOVA ein Polypeptid umfasst, dargestellt durch eine Aminosäuresequenz, ausgewählt aus der Gruppe bestehend aus SEQ ID NO: 1-2 oder eine Aminosäuresequenz mit einer Identität von mindestens 97 % zu einer der SEQ ID NO: 1-2; oder
   (b) das rOVA eine Aminosäuresequenz eines Enten-OVA, eines Strauß-OVA oder eines Hühner-OVA umfasst.

5. Lebensmittelartikel nach einem der vorhergehenden Ansprüche, wobei die Aminosäuresequenz des rOVA kein N-terminales Methionin aufweist.

6. Lebensmittelartikel nach einem der vorhergehenden Ansprüche, wobei

   (a) das rOVA dem Lebensmittelprodukt eine Schaumkapazität von 20 %, 30 %, 40 % oder 50 % über der von nativem Eiklar verleiht;
   (b) das rOVA dem Lebensmittelprodukt eine Härte größer als die von nativem Eiklar verleiht; oder
   (c) das rOVA dem eifreien Lebensmittelprodukt eine Zähigkeit größer als die von nativem Eiklar verleiht.

7. Lebensmittelartikel nach einem der vorhergehenden Ansprüche, wobei das rOVA durch eine Hefewirtszelle oder eine Pilzwirtszelle exprimiert wird.

8. Lebensmittelartikel nach einem der vorhergehenden Ansprüche, wobei das rOVA durch eine *Pichia-sp.*-Wirtszelle exprimiert wird und wobei ein Glykosylierungsmuster des rOVA frei von N-verknüpften Galaktoseeinheiten ist.

9. Lebensmittelartikel nach einem der Ansprüche 1-8, wobei rOVA in dem eifreien Lebensmittelartikel in einer Menge von weniger als 8 Gew.-% vorliegt.

10. Lebensmittelartikel nach einem der Ansprüche 1-9, ferner umfassend mindestens ein Fett oder Öl und mindestens eine Getreidestärke.

11. Lebensmittelartikel nach einem der Ansprüche 2-10, wobei

    (a) das eifreie Lebensmittelprodukt ein gebackenes Produkt ist; oder
    (b) das eifreie Lebensmittelprodukt ein Kuchen, ein Brot, ein Brötchen, ein Gebäck, ein Cracker, ein Muffin, ein Scone, ein Bagel, ein Keks oder ein Cookie ist.

12. Lebensmittelprodukt nach Anspruch 11, wobei das eifreie Lebensmittelprodukt eine Krumenstruktur aufweist, die mindestens vergleichbar ist mit einem ähnlichen Lebensmittelprodukt, das mit einem natürlichen Eiklar oder mit einem natürlichen Vollei erzeugt worden ist.

13. Lebensmittelartikel nach Anspruch 2, wobei der eifreie Lebensmittelartikel ein emulgiertes Produkt ist, ferner umfassend mindestens ein Fett oder Öl und Wasser.

**Revendications**

1. Produit alimentaire comprenant une composition d'ingrédients, la composition d'ingrédients comprenant une ovalbumine recombinante (rOVA), dans lequel la rOVA est présente dans le produit alimentaire en une quantité comprise entre 2 % et 15 % (p/p), et dans lequel le produit alimentaire ne comprend aucune protéine de blanc d'oeuf à l'exception de rOVA.

2. Produit alimentaire selon la revendication 1, dans lequel le produit alimentaire est un produit alimentaire sans oeuf.

3. Produit alimentaire selon l'une quelconque des revendications 1 ou 2, dans lequel la composition d'ingrédients est une composition d'ingrédients en poudre comprenant au moins 75 % de rOVA (p/p de protéines totales ou p/p de composition totale), éventuellement dans lequel la composition d'ingrédients en poudre est un concentré.

4. Produit alimentaire selon l'une quelconque des revendications 1 à 3, dans lequel

   (a) la rOVA comprend un polypeptide représenté par une séquence d'acides aminés sélectionnée dans le groupe constitué par SEQ ID NO: 1-2 ou une séquence d'acides aminés présentant au moins 97 % d'identité avec l'une des séquences SEQ ID NO 1-2 ; ou
   (b) la rOVA comprend une séquence d'acides aminés d'un OVA de canard, d'un OVA d'autruche ou d'un OVA de poulet.

5. Produit alimentaire selon l'une quelconque des revendications précédentes, dans lequel la séquence d'acides aminés de la rOVA est dépourvue de méthionine N-terminale.

6. Produit alimentaire selon l'une quelconque des revendications précédentes, dans lequel

   (a) la rOVA confère au produit alimentaire une capacité de mousse d'au moins 20 %, 30 %, 40 % ou 50 % supérieure à celle du blanc d'oeuf natif ;
   (b) la rOVA confère au produit alimentaire une dureté qui est supérieure à celle du blanc d'oeuf natif ; ou
   (c) la rOVA fournit au produit alimentaire sans oeuf une masticabilité supérieure à celle du blanc d'oeuf natif.

7. Produit alimentaire selon l'une quelconque des revendications précédentes, dans lequel la rOVA est exprimée par une cellule hôte de levure ou une cellule hôte fongique.

8. Produit alimentaire selon l'une quelconque des revendications précédentes, dans lequel la rOVA est exprimée par une cellule hôte de *Pichia* sp. et le profil de glycosylation de la rOVA est dépourvu d'unités de galactose N-liées.

9. Produit alimentaire selon l'une quelconque des revendications 1 à 8, dans lequel la rOVA est présente dans le produit alimentaire sans oeuf en une quantité inférieure à 8 % p/p.

10. Produit alimentaire selon l'une quelconque des revendications 1 à 9, comprenant en outre au moins une graisse ou huile et au moins un amidon de grain.

11. Produit alimentaire selon l'une quelconque des revendications 2-10 dans lequel

   a) le produit alimentaire sans oeuf est un produit cuit ; ou
   b) le produit alimentaire sans oeuf est un gâteau, un pain, un rouleau, une pâtisserie, un cracker, un muffin, un scone, un bagel, un biscuit ou un cookie.

12. Produit alimentaire selon la revendication 11, dans lequel le produit alimentaire sans oeuf a une structure de miettes comparable à ou meilleure qu'un produit alimentaire similaire fabriqué avec un blanc d'oeuf naturel ou un oeuf entier naturel.

13. Produit alimentaire selon la revendication 2, dans lequel le produit alimentaire sans oeuf est un produit émulsifié comprenant en outre au moins une graisse ou huile et de l'eau.

**FIG. 1A**

**FIG. 1B**

| | rOVA+ Xanthan gum | rOVA+Potato Starch+Xanthan gum | Control Egg pound cake |
|---|---|---|---|
| **Photos** | | | |
| Cross-section | | | |
| | | | |

FIG. 2

| Egg white meringue | nOVA meringue | rOVA meringue |
|---|---|---|
| | | |

FIG. 3

|  | Whole egg | Egg white | nOVA |
|---|---|---|---|
| Heat coagulation 72°C for 10min | | | |
| Foaming | | | |

FIG. 4

|  | Egg white | nOVA | rOVA |
|---|---|---|---|
| Heat coagulation | | | |
| Foaming | | | |

FIG. 5

FIG. 6A

**FIG. 6B**

| Sample | pH | Gelation at 72°C before foaming | Gelation at 72°C after foaming |
|---|---|---|---|
| Gallus gallus nOVA | 5.87 | | |
| Gallus gallus rOVA | 6.49 | | |
| Gallus gallus rOVA (pH adjusted) | 6.08 | | |
| Ostrich rOVA | 3.7 | | |
| Ostrich rOVA (pH adjusted) | 5.73 | Did not gel | Did not gel |
| Duck rOVA | 4.3 | | |
| Egg White | 9.01 | | |

FIG. 7

EP 4 017 287 B1

Control     Whole egg wash     Bake sheen     nOVA     nOVA

**FIG. 8**

**FIG. 9A**

| | Ambient | Refrigerated |
|---|---|---|
| Day 0 |  L to R: 8%EWP, 8% rOVA, Negative control | |
| Day 3 |  L to R: 8%EWP, 8% rOVA, Negative control |  L to R: 8%EWP, 8% rOVA, Negative control |

**FIG. 9B**

| Sample | Cocktail |
|---|---|
| Negative Control | |
| Egg white control | |
| 7% rOVA | |
| 12% rOVA | |

**FIG. 10**

| Egg-white patty | nOVA patty | rOVA patty |
|---|---|---|
| | | |
| | | |

FIG. 11

| Parameter | Fresh egg white | rOVA 8.3% + SLS + Xanthan gum | rOVA 8.3% + TEC + Xanthan gum |
|---|---|---|---|
| Photos | | | |
| Cross section | | | |

n=6

**FIG. 12**

**FIG. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2018355020 A

**Non-patent literature cited in the description**

• **RAEKER M.O. et al.** *Cake-Baking (High-Ratio White Layer) Properties of Egg White, Bovine Blood Plasma, and Their Protein Fractions,* 1995